# EUROPEAN PATENT APPLICATION

(11) **EP 2 397 555 A1**
(43) Date of publication of application: **21.12.2011**
(21) Application number: 10182700.4
(22) Date of filing: 07.04.1999
(51) Int. Cl.: C12N 15/31, C07K 14/35, C07K 19/00, C12P 21/02, A61K 39/04

(54) **Fusion proteins of mycobacterium tuberculosis antigens and their uses**

(30) Priority: 07.04.1998 US 56556; 30.12.1998 US 223040
(62) Divisional of application: 99916513.7
(71) Applicant: CORIXA CORPORATION, Wilmington, DE 19808 (US)
(72) Inventor: Skeiky, Yasir A W, Maryland, WA 20904 (US); Alderson, Mark, Bainbridge Island, WA 98110 (US); Campos-Neto, Antionio, Boston, MA 02115-3799 (US)
(74) Representative: Goodall, Scott

(57) **Abstract**

The present invention relates to fusion proteins containing *Mycobacterium tuberculosis* antigens and methods for their use in the diagnosis, treatment and prevention of tuberculosis infection.

SEQUENCE LISTING

<110> Corixa Corporation

<120> Fusion Protiens of Mycobacterium tuberculosis Antigens
      and Their Uses

<130> CRX-P457EPD1

<140> EP10182700.4
<141> 1999-04-07

<150> US 09/056,556
<151> 1998-04-07

<150> US 09/223,040
<151> 1998-12-30

<160> 41

<170> PatentIn Ver. 2.1

<210> 1
<211> 2287
<212> DNA
<213> Artificial Sequence

<220>
<223> Description of Artificial Sequence:tri-fusion
      protein Ra12-TbH9-Ra35 (designated Mtb32A)

<220>
<221> modified_base
<222> (30)
<223> n = g, a, c or t

<220>
<221> modified_base
<222> (33)
<223> n = g, a, c or t

<220>
<221> CDS
<222> (42)..(2231)

<220>
<221> modified_base
<222> (2270)
<223> n = g, a, c or t

<400> 1
tctagaaata attttgttta ctttaagaan ganatataca t atg cat cac cat cac 56

cat cac acg gcc gcg tcc gat aac ttc cag ctg tcc cag ggt ggg cag   104

gga ttc gcc att ccg atc ggg cag gcg atg gcg atc gcg ggc cag atc   152

cga tcg ggt ggg ggg tca ccc acc gtt cat atc ggg cct acc gcc ttc 200

ctc ggc ttg ggt gtt gtc gac aac aac ggc aac ggc gca cga gtc caa 248

cgc gtg gtc ggg agc gct ccg gcg gca agt ctc ggc atc tcc acc ggc 296

gac gtg atc acc gcg gtc gac ggc gct ccg atc aac tcg gcc acc gcg 344

atg gcg gac gcg ctt aac ggg cat cat ccc ggt gac gtc atc tcg gtg 392

acc tgg caa acc aag tcg ggc ggc acg cgt aca ggg aac gtg aca ttg 440

gcc gag gga ccc ccg gcc gaa ttc atg gtg gat ttc ggg gcg tta cca 488

ccg gag atc aac tcc gcg agg atg tac gcc ggc ccg ggt tcg gcc tcg 536

ctg gtg gcc gcg gct cag atg tgg gac agc gtg gcg agt gac ctg ttt 584

tcg gcc gcg tcg gcg ttt cag tcg gtg gtc tgg ggt ctg acg gtg ggg 632

tcg tgg ata ggt tcg tcg gcg ggt ctg atg gtg gcg gcg gcc tcg ccg 680

tat gtg gcg tgg atg agc gtc acc gcg ggg cag gcc gag ctg acc gcc 728

gcc cag gtc cgg gtt gct gcg gcg gcc tac gag acg gcg tat ggg ctg 776

acg gtg ccc ccg ccg gtg atc gcc gag aac cgt gct gaa ctg atg att 824

ctg ata gcg acc aac ctc ttg ggg caa aac acc ccg gcg atc gcg gtc 872

aac gag gcc gaa tac ggc gag atg tgg gcc caa gac gcc gcc gcg atg 920

ttt ggc tac gcc gcg gcg acg gcg acg gcg acg gcg acg ttg ctg ccg 968

ttc gag gag gcg ccg gag atg acc agc gcg ggt ggg ctc ctc gag cag 1016

gcc gcc gcg gtc gag gag gcc tcc gac acc gcc gcg gcg aac cag ttg 1064

atg aac aat gtg ccc cag gcg ctg caa cag ctg gcc cag ccc acg cag 1112

ggc acc acg cct tct tcc aag ctg ggt ggc ctg tgg aag acg gtc tcg 1160

ccg cat cgg tcg ccg atc agc aac atg gtg tcg atg gcc aac aac cac 1208

atg tcg atg acc aac tcg ggt gtg tcg atg acc aac acc ttg agc tcg 1256

atg ttg aag ggc ttt gct ccg gcg gcg gcc gcc cag gcc gtg caa acc 1304

gcg gcg caa aac ggg gtc cgg gcg atg agc tcg ctg ggc agc tcg ctg 1352

ggt tct tcg ggt ctg ggc ggt ggg gtg gcc gcc aac ttg ggt cgg gcg 1400

gcc tcg gtc ggt tcg ttg tcg gtg ccg cag gcc tgg gcc gcg gcc aac 1448

cag gca gtc acc ccg gcg gcg cgg gcg ctg ccg ctg acc agc ctg acc 1496

agc gcc gcg gaa aga ggg ccc ggg cag atg ctg ggc ggg ctg ccg gtg 1544

ggg cag atg ggc gcc agg gcc ggt ggt ggg ctc agt ggt gtg ctg cgt 1592

gtt ccg ccg cga ccc tat gtg atg ccg cat tct ccg gca gcc ggc gat 1640

atc gcc ccg ccg gcc ttg tcg cag gac cgg ttc gcc gac ttc ccc gcg 1688

ctg ccc ctc gac ccg tcc gcg atg gtc gcc caa gtg ggg cca cag gtg 1736

gtc aac atc aac acc aaa ctg ggc tac aac aac gcc gtg ggc gcc ggg 1784

acc ggc atc gtc atc gat ccc aac ggt gtc gtg ctg acc aac aac cac 1832

gtg atc gcg ggc gcc acc gac atc aat gcg ttc agc gtc ggc tcc ggc 1880

caa acc tac ggc gtc gat gtg gtc ggg tat gac cgc acc cag gat gtc 1928

gcg gtg ctg cag ctg cgc ggt gcc ggt ggc cta cca tcg gcg gcg atc 1976

ggt ggc ggc gtc gcg gtt ggt gag ccc ttc gtc gcg atg ggc aac agc 2024

ggt ggg cag ggc gga acg ccc cgt gcg gtg cct ggc agg gtg gtc gcg 2072

ctc ggc caa acc gtg cag gcg tcg gat tcg ctg acc ggt gcc gaa gag 2120

aca ttg aac ggg ttg atc cag ttc gat gcc gcg atc cag ccc ggt gat 2168

tcg ggc ggg ccc gtc gtc aac ggc cta gga cag gtg gtc ggt atg aac 2216

acg gcc gcg tcc taggatatcc atcacactgg cggccgctcg agcagatccg 2268

gntgtaacaa agcccgaaa 2287

<210> 2
<211> 729
<212> PRT
<213> Artificial Sequence
<223> Description of Artificial Sequence:tri-fusion
      protein Ra12-TbH9-Ra35 (designated Mtb32A)

<400> 2
Met His His His His His His Thr Ala Ala Ser Asp Asn Phe Gln Leu
1               5                   10                  15

Ser Gln Gly Gly Gln Gly Phe Ala Ile Pro Ile Gly Gln Ala Met Ala
            20                  25                  30

Ile Ala Gly Gln Ile Arg Ser Gly Gly Gly Ser Pro Thr Val His Ile
        35                  40                  45

Gly Pro Thr Ala Phe Leu Gly Leu Gly Val Val Asp Asn Asn Gly Asn
    50                  55                  60

Gly Ala Arg Val Gln Arg Val Val Gly Ser Ala Pro Ala Ala Ser Leu
65                  70                  75                  80

Gly Ile Ser Thr Gly Asp Val Ile Thr Ala Val Asp Gly Ala Pro Ile
                85                  90                  95

Asn Ser Ala Thr Ala Met Ala Asp Ala Leu Asn Gly His His Pro Gly
            100                 105                 110

Asp Val Ile Ser Val Thr Trp Gln Thr Lys Ser Gly Gly Thr Arg Thr
        115                 120                 125

Gly Asn Val Thr Leu Ala Glu Gly Pro Pro Ala Glu Phe Met Val Asp
    130                 135                 140

Phe Gly Ala Leu Pro Pro Glu Ile Asn Ser Ala Arg Met Tyr Ala Gly
145                 150                 155                 160

Pro Gly Ser Ala Ser Leu Val Ala Ala Ala Gln Met Trp Asp Ser Val
                165                 170                 175

Ala Ser Asp Leu Phe Ser Ala Ala Ser Ala Phe Gln Ser Val Val Trp
            180                 185                 190

Gly Leu Thr Val Gly Ser Trp Ile Gly Ser Ser Ala Gly Leu Met Val
        195                 200                 205

Ala Ala Ala Ser Pro Tyr Val Ala Trp Met Ser Val Thr Ala Gly Gln
    210                 215                 220

Ala Glu Leu Thr Ala Ala Gln Val Arg Val Ala Ala Ala Ala Tyr Glu
225                 230                 235                 240

Thr Ala Tyr Gly Leu Thr Val Pro Pro Pro Val Ile Ala Glu Asn Arg
                245                 250                 255

Ala Glu Leu Met Ile Leu Ile Ala Thr Asn Leu Leu Gly Gln Asn Thr
            260                 265                 270

Pro Ala Ile Ala Val Asn Glu Ala Glu Tyr Gly Glu Met Trp Ala Gln
        275                 280                 285

Asp Ala Ala Ala Met Phe Gly Tyr Ala Ala Ala Thr Ala Thr Ala Thr
    290                 295                 300

Ala Thr Leu Leu Pro Phe Glu Glu Ala Pro Glu Met Thr Ser Ala Gly
305                 310                 315                 320

Gly Leu Leu Glu Gln Ala Ala Ala Val Glu Glu Ala Ser Asp Thr Ala
                325                 330                 335

Ala Ala Asn Gln Leu Met Asn Asn Val Pro Gln Ala Leu Gln Gln Leu
            340                 345                 350

Ala Gln Pro Thr Gln Gly Thr Thr Pro Ser Ser Lys Leu Gly Gly Leu
        355                 360                 365

Trp Lys Thr Val Ser Pro His Arg Ser Pro Ile Ser Asn Met Val Ser
    370                 375                 380

Met Ala Asn Asn His Met Ser Met Thr Asn Ser Gly Val Ser Met Thr
385                 390                 395                 400

Asn Thr Leu Ser Ser Met Leu Lys Gly Phe Ala Pro Ala Ala Ala Ala
                405                 410                 415

Gln Ala Val Gln Thr Ala Ala Gln Asn Gly Val Arg Ala Met Ser Ser
            420                 425                 430

Leu Gly Ser Ser Leu Gly Ser Ser Gly Leu Gly Gly Gly Val Ala Ala
        435                 440                 445

Asn Leu Gly Arg Ala Ala Ser Val Gly Ser Leu Ser Val Pro Gln Ala
    450                 455                 460

Trp Ala Ala Ala Asn Gln Ala Val Thr Pro Ala Ala Arg Ala Leu Pro
465                 470                 475                 480

Leu Thr Ser Leu Thr Ser Ala Ala Glu Arg Gly Pro Gly Gln Met Leu
                485                 490                 495

Gly Gly Leu Pro Val Gly Gln Met Gly Ala Arg Ala Gly Gly Gly Leu
            500                 505                 510

Ser Gly Val Leu Arg Val Pro Pro Arg Pro Tyr Val Met Pro His Ser
        515                 520                 525

Pro Ala Ala Gly Asp Ile Ala Pro Pro Ala Leu Ser Gln Asp Arg Phe
    530                 535                 540

Ala Asp Phe Pro Ala Leu Pro Leu Asp Pro Ser Ala Met Val Ala Gln
545                 550                 555                 560

Val Gly Pro Gln Val Val Asn Ile Asn Thr Lys Leu Gly Tyr Asn Asn
                565                 570                 575

Ala Val Gly Ala Gly Thr Gly Ile Val Ile Asp Pro Asn Gly Val Val
            580                 585                 590

Leu Thr Asn Asn His Val Ile Ala Gly Ala Thr Asp Ile Asn Ala Phe
        595                 600                 605

Ser Val Gly Ser Gly Gln Thr Tyr Gly Val Asp Val Val Gly Tyr Asp
    610                 615                 620

Arg Thr Gln Asp Val Ala Val Leu Gln Leu Arg Gly Ala Gly Gly Leu
625                 630                 635                 640

Pro Ser Ala Ala Ile Gly Gly Gly Val Ala Val Gly Glu Pro Phe Val
                645                 650                 655

Ala Met Gly Asn Ser Gly Gly Gln Gly Gly Thr Pro Arg Ala Val Pro
            660                 665                 670

Gly Arg Val Val Ala Leu Gly Gln Thr Val Gln Ala Ser Asp Ser Leu
        675                 680                 685

Thr Gly Ala Glu Glu Thr Leu Asn Gly Leu Ile Gln Phe Asp Ala Ala
    690                 695                 700

Ile Gln Pro Gly Asp Ser Gly Gly Pro Val Val Asn Gly Leu Gly Gln
705                 710                 715                 720

Val Val Gly Met Asn Thr Ala Ala Ser
                725

<210> 3
<211> 1081
<212> DNA
<213> Artificial Sequence

<220>
<223> Description of Artificial Sequence:tri-fusion
      protein Erd14-DPV-MTI (designated Mtb39A)

<220>
<221> CDS
<222> (10)..(1002)

<400> 3
gatatacat atg cat cac cat cac cat cac atg gcc acc acc ctt ccc gtt 51

cag cgc cac ccg cgg tcc ctc ttc ccc gag ttt tct gag ctg ttc gcg   99

gcc ttc ccg tca ttc gcc gga ctc cgg ccc acc ttc gac acc cgg ttg   147

atg cgg ctg gaa gac gag atg aaa gag ggg cgc tac gag gta cgc gcg   195

gag ctt ccc ggg gtc gac ccc gac aag gac gtc gac att atg gtc cgc   243

gat ggt cag ctg acc atc aag gcc gag cgc acc gag cag aag gac ttc   291

gac ggt cgc tcg gaa ttc gcg tac ggt tcc ttc gtt cgc acg gtg tcg   339

ctg ccg gta ggt gct gac gag gac gac att aag gcc acc tac gac aag   387

ggc att ctt act gtg tcg gtg gcg gtt tcg gaa ggg aag cca acc gaa   435

aag cac att cag atc cgg tcc acc aac aag ctt gat ccc gtg gac gcg   483

gtc att aac acc acc tgc aat tac ggg cag gta gta gct gcg ctc aac   531

gcg acg gat ccg ggg gct gcc gca cag ttc aac gcc tca ccg gtg gcg   579

cag tcc tat ttg cgc aat ttc ctc gcc gca ccg cca cct cag cgc gct   627

gcc atg gcc gcg caa ttg caa gct gtg ccg ggg gcg gca cag tac atc   675

ggc ctt gtc gag tcg gtt gcc ggc tcc tgc aac aac tat gag ctc atg   723

acg att aat tac cag ttc ggg gac gtc gac gct cat ggc gcc atg atc   771

cgc gct cag gcg gcg tcg ctt gag gcg gag cat cag gcc atc gtt cgt   819

gat gtg ttg gcc gcg ggt gac ttt tgg ggc ggc gcc ggt tcg gtg gct   867

tgc cag gag ttc att acc cag ttg ggc cgt aac ttc cag gtg atc tac   915

gag cag gcc aac gcc cac ggg cag aag gtg cag gct gcc ggc aac aac   963

atg gcg caa acc gac agc gcc gtc ggc tcc agc tgg gcc actagtaacg   1012

gccgccagtg tgctggaatt ctgcagatat ccatcacact ggcggccgct cgagcagatc 1072

cggctgcta                                                         1081

<210> 4
<211> 331
<212> PRT
<213> Artificial Sequence
<223> Description of Artificial Sequence:tri-fusion
      protein Erd14-DPV-MTI (designated Mtb39A)

<400> 4
Met His His His His His His Met Ala Thr Thr Leu Pro Val Gln Arg
  1               5                  10                  15

His Pro Arg Ser Leu Phe Pro Glu Phe Ser Glu Leu Phe Ala Ala Phe
             20                  25                  30

Pro Ser Phe Ala Gly Leu Arg Pro Thr Phe Asp Thr Arg Leu Met Arg
         35                  40                  45

Leu Glu Asp Glu Met Lys Glu Gly Arg Tyr Glu Val Arg Ala Glu Leu
     50                  55                  60

Pro Gly Val Asp Pro Asp Lys Asp Val Asp Ile Met Val Arg Asp Gly
 65                  70                  75                  80

Gln Leu Thr Ile Lys Ala Glu Arg Thr Glu Gln Lys Asp Phe Asp Gly
                 85                  90                  95

Arg Ser Glu Phe Ala Tyr Gly Ser Phe Val Arg Thr Val Ser Leu Pro
            100                 105                 110

Val Gly Ala Asp Glu Asp Asp Ile Lys Ala Thr Tyr Asp Lys Gly Ile
        115                 120                 125

Leu Thr Val Ser Val Ala Val Ser Glu Gly Lys Pro Thr Glu Lys His
    130                 135                 140

Ile Gln Ile Arg Ser Thr Asn Lys Leu Asp Pro Val Asp Ala Val Ile
145                 150                 155                 160

Asn Thr Thr Cys Asn Tyr Gly Gln Val Val Ala Ala Leu Asn Ala Thr
                165                 170                 175

Asp Pro Gly Ala Ala Ala Gln Phe Asn Ala Ser Pro Val Ala Gln Ser
            180                 185                 190

Tyr Leu Arg Asn Phe Leu Ala Ala Pro Pro Pro Gln Arg Ala Ala Met
        195                 200                 205

Ala Ala Gln Leu Gln Ala Val Pro Gly Ala Ala Gln Tyr Ile Gly Leu
    210                 215                 220

Val Glu Ser Val Ala Gly Ser Cys Asn Asn Tyr Glu Leu Met Thr Ile
225                 230                 235                 240

Asn Tyr Gln Phe Gly Asp Val Asp Ala His Gly Ala Met Ile Arg Ala
                245                 250                 255

Gln Ala Ala Ser Leu Glu Ala Glu His Gln Ala Ile Val Arg Asp Val
            260                 265                 270

Leu Ala Ala Gly Asp Phe Trp Gly Gly Ala Gly Ser Val Ala Cys Gln
        275                 280                 285

Glu Phe Ile Thr Gln Leu Gly Arg Asn Phe Gln Val Ile Tyr Glu Gln
    290                 295                 300

Ala Asn Ala His Gly Gln Lys Val Gln Ala Ala Gly Asn Asn Met Ala
305                 310                 315                 320

Gln Thr Asp Ser Ala Val Gly Ser Ser Trp Ala
                325                 330

<210> 5
<211> 1993
<212> DNA
<213> Artificial Sequence

<220>
<223> Description of Artificial Sequence:tri-fusion
      protein TbRa3-38kD-Tb38-1

<220>
<221> CDS
<222> (152)..(1276)

<400> 5
tgttcttcga cggcaggctg gtggaggaag ggcccaccga acagctgttc tcctcgccga 60

agcatgcgga aaccgcccga tacgtcgccg gactgtcggg ggacgtcaag gacgccaagc 120

gcggaaattg aagagcacag aaaggtatgg c gtg aaa att cgt ttg cat acg   172

ctg ttg gcc gtg ttg acc gct gcg ccg ctg ctg cta gca gcg gcg ggc   220

tgt ggc tcg aaa cca ccg agc ggt tcg cct gaa acg ggc gcc ggc gcc   268

ggt act gtc gcg act acc ccc gcg tcg tcg ccg gtg acg ttg gcg gag   316

acc ggt agc acg ctg ctc tac ccg ctg ttc aac ctg tgg ggt ccg gcc   364

ttt cac gag agg tat ccg aac gtc acg atc acc gct cag ggc acc ggt   412

tct ggt gcc ggg atc gcg cag gcc gcc gcc ggg acg gtc aac att ggg   460

gcc tcc gac gcc tat ctg tcg gaa ggt gat atg gcc gcg cac aag ggg   508

ctg atg aac atc gcg cta gcc atc tcc gct cag cag gtc aac tac aac   556

ctg ccc gga gtg agc gag cac ctc aag ctg aac gga aaa gtc ctg gcg   604

gcc atg tac cag ggc acc atc aaa acc tgg gac gac ccg cag atc gct   652

gcg ctc aac ccc ggc gtg aac ctg ccc ggc acc gcg gta gtt ccg ctg   700

cac cgc tcc gac ggg tcc ggt gac acc ttc ttg ttc acc cag tac ctg   748

tcc aag caa gat ccc gag ggc tgg ggc aag tcg ccc ggc ttc ggc acc   796

acc gtc gac ttc ccg gcg gtg ccg ggt gcg ctg ggt gag aac ggc aac 844

ggc ggc atg gtg acc ggt tgc gcc gag aca ccg ggc tgc gtg gcc tat 892

atc ggc atc agc ttc ctc gac cag gcc agt caa cgg gga ctc ggc gag 940

gcc caa cta ggc aat agc tct ggc aat ttc ttg ttg ccc gac gcg caa 988

agc att cag gcc gcg gcg gct ggc ttc gca tcg aaa acc ccg gcg aac 1036

cag gcg att tcg atg atc gac ggg ccc gcc ccg gac ggc tac ccg atc 1084

atc aac tac gag tac gcc atc gtc aac aac cgg caa aag gac gcc gcc 1132

acc gcg cag acc ttg cag gca ttt ctg cac tgg gcg atc acc gac ggc 1180

aac aag gcc tcg ttc ctc gac cag gtt cat ttc cag ccg ctg ccg ccc 1228

gcg gtg gtg aag ttg tct gac gcg ttg atc gcg acg att tcc agc 1273

tagcctcgtt gaccaccacg cgacagcaac ctccgtcggg ccatcgggct gctttgcgga 1333

gcatgctggc ccgtgccggt gaagtcggcc gcgctggccc ggccatccgg tggttgggtg 1393

ggataggtgc ggtgatcccg ctgcttgcgc tggtcttggt gctggtggtg ctggtcatcg 1453

aggcgatggg tgcgatcagg ctcaacgggt tgcatttctt caccgccacc gaatggaatc 1513

caggcaacac ctacggcgaa accgttgtca ccgacgcgtc gcccatccgg tcggcgccta 1573

ctacggggcg ttgccgctga tcgtcgggac gctggcgacc tcggcaatcg ccctgatcat 1633

cgcggtgccg gtctctgtag gagcggcgct ggtgatcgtg gaacggctgc cgaaacggtt 1693

ggccgaggct gtgggaatag tcctggaatt gctcgccgga atccccagcg tggtcgtcgg 1753

tttgtggggg gcaatgacgt tcgggccgtt catcgctcat cacatcgctc cggtgatcgc 1813

tcacaacgct cccgatgtgc cggtgctgaa ctacttgcgc ggcgacccgg gcaacgggga 1873

gggcatgttg gtgtccggtc tggtgttggc ggtgatggtc gttcccatta tcgccaccac 1933

cactcatgac ctgttccggc aggtgccggt gttgccccgg gagggcgcga tcgggaattc 1993

<210> 6
<211> 374
<212> PRT
<213> Artificial Sequence
<223> Description of Artificial Sequence:tri-fusion
      protein TbRa3-38kD-Tb38-1

<400> 6
Val Lys Ile Arg Leu His Thr Leu Leu Ala Val Leu Thr Ala Ala Pro
  1               5                  10                  15

Leu Leu Leu Ala Ala Ala Gly Cys Gly Ser Lys Pro Pro Ser Gly Ser
             20                  25                  30

Pro Glu Thr Gly Ala Gly Ala Gly Thr Val Ala Thr Thr Pro Ala Ser
            35                  40                  45

Ser Pro Val Thr Leu Ala Glu Thr Gly Ser Thr Leu Leu Tyr Pro Leu
    50                  55                  60

Phe Asn Leu Trp Gly Pro Ala Phe His Glu Arg Tyr Pro Asn Val Thr
65                  70                  75                  80

Ile Thr Ala Gln Gly Thr Gly Ser Gly Ala Gly Ile Ala Gln Ala Ala
                85                  90                  95

Ala Gly Thr Val Asn Ile Gly Ala Ser Asp Ala Tyr Leu Ser Glu Gly
            100                 105                 110

Asp Met Ala Ala His Lys Gly Leu Met Asn Ile Ala Leu Ala Ile Ser
        115                 120                 125

Ala Gln Gln Val Asn Tyr Asn Leu Pro Gly Val Ser Glu His Leu Lys
    130                 135                 140

Leu Asn Gly Lys Val Leu Ala Ala Met Tyr Gln Gly Thr Ile Lys Thr
145                 150                 155                 160

Trp Asp Asp Pro Gln Ile Ala Ala Leu Asn Pro Gly Val Asn Leu Pro
                165                 170                 175

Gly Thr Ala Val Val Pro Leu His Arg Ser Asp Gly Ser Gly Asp Thr
            180                 185                 190

Phe Leu Phe Thr Gln Tyr Leu Ser Lys Gln Asp Pro Glu Gly Trp Gly
        195                 200                 205

Lys Ser Pro Gly Phe Gly Thr Thr Val Asp Phe Pro Ala Val Pro Gly
    210                 215                 220

Ala Leu Gly Glu Asn Gly Asn Gly Gly Met Val Thr Gly Cys Ala Glu
225                 230                 235                 240

Thr Pro Gly Cys Val Ala Tyr Ile Gly Ile Ser Phe Leu Asp Gln Ala
                245                 250                 255

Ser Gln Arg Gly Leu Gly Glu Ala Gln Leu Gly Asn Ser Ser Gly Asn
            260                 265                 270

Phe Leu Leu Pro Asp Ala Gln Ser Ile Gln Ala Ala Ala Ala Gly Phe
        275                 280                 285

Ala Ser Lys Thr Pro Ala Asn Gln Ala Ile Ser Met Ile Asp Gly Pro
    290                 295                 300

Ala Pro Asp Gly Tyr Pro Ile Ile Asn Tyr Glu Tyr Ala Ile Val Asn
305                 310                 315                 320

Asn Arg Gln Lys Asp Ala Ala Thr Ala Gln Thr Leu Gln Ala Phe Leu
                325                 330                 335

His Trp Ala Ile Thr Asp Gly Asn Lys Ala Ser Phe Leu Asp Gln Val
            340                 345                 350

His Phe Gln Pro Leu Pro Pro Ala Val Val Lys Leu Ser Asp Ala Leu
        355                 360                 365

Ile Ala Thr Ile Ser Ser
    370

<210> 7
<211> 1777
<212> DNA
<213> Artificial Sequence

<220>
<223> Description of Artificial Sequence:bi-fusion
      protein TbH9-Tb38-1

<400> 7
ggtcttgacc accacctggg tgtcgaagtc ggtgcccgga ttgaagtcca ggtactcgtg 60
ggtggggcgg gcgaaacaat agcgacaagc atgcgagcag ccgcggtagc cgttgacggt 120
gtagcgaaac ggcaacgcgg ccgcgttggg caccttgttc agcgctgatt tgcacaacac 180
ctcgtggaag gtgatgccgt cgaattgtgg cgcgcgaacg ctgcggacca ggccgatccg 240
ctgcaacccg gcagcgcccg tcgtcaacgg gcatcccgtt caccgcgacg gcttgccggg 300
cccaacgcat accattattc gaacaaccgt tctatacttt gtcaacgctg gccgctaccg 360
agcgccgcac aggatgtgat atgccatctc tgcccgcaca gacaggagcc aggccttatg 420
acagcattcg gcgtcgagcc ctacgggcag ccgaagtacc tagaaatcgc cgggaagcgc 480
atggcgtata tcgacgaagg caagggtgac gccatcgtct ttcagcacgg caaccccacg 540
tcgtcttact tgtggcgcaa catcatgccg cacttggaag ggctgggccg gctggtggcc 600
tgcgatctga tcgggatggg cgcgtcggac aagctcagcc catcgggacc cgaccgctat 660
agctatggcg agcaacgaga ctttttgttc gcgctctggg atgcgctcga cctcggcgac 720
cacgtggtac tggtgctgca cgactggggc tcggcgctcg gcttcgactg ggctaaccag 780
catcgcgacc gagtgcaggg gatcgcgttc atggaagcga tcgtcacccc gatgacgtgg 840
gcggactggc cgccggccgt gcggggtgtg ttccagggtt tccgatcgcc tcaaggcgag 900
ccaatggcgt tggagcacaa catctttgtc gaacgggtgc tgcccggggc gatcctgcga 960
cagctcagcg acgaggaaat gaaccactat cggcggccat tcgtgaacgg cggcgaggac 1020
cgtcgcccca cgttgtcgtg gccacgaaac cttccaatcg acggtgagcc cgccgaggtc 1080
gtcgcgttgg tcaacgagta ccggagctgg ctcgaggaaa ccgacatgcc gaaactgttc 1140
atcaacgccg agcccggcgc gatcatcacc ggccgcatcc gtgactatgt caggagctgg 1200
cccaaccaga ccgaaatcac agtgcccggc gtgcatttcg ttcaggagga cagcgatggc 1260
gtcgtatcgt gggcgggcgc tcggcagcat cggcgacctg ggagcgctct catttcacga 1320
gaccaagaat gtgatttccg gcgaaggcgg cgccctgctt gtcaactcat aagacttcct 1380
gctccgggca gagattctca gggaaaaggg caccaatcgc agccgcttcc ttcgcaacga 1440
ggtcgacaaa tatacgtggc aggacaaagg tcttcctatt tgcccagcga attagtcgct 1500
gcctttctat gggctcagtt cgaggaagcc gagcggatca cgcgtatccg attggaccta 1560
tggaaccggt atcatgaaag cttcgaatca ttggaacagc gggggctcct gcgccgtccg 1620
atcatcccac agggctgctc tcacaacgcc cacatgtact acgtgttact agcgcccagc 1680
gccgatcggg aggaggtgct ggcgcgtctg acgagcgaag gtataggcgc ggtctttcat 1740
tacgtgccgc ttcacgattc gccggccggg cgtcgct                          1777

<210> 8
<211> 358
<212> PRT
<213> Artificial Sequence

<220>
<223> Description of Artificial Sequence:bi-fusion
      protein TbH9-Tb38-1

<220>
<221> MOD_RES
<222> (254)
<223> Xaa = any amino acid

<400> 8
Val Ala Trp Met Ser Val Thr Ala Gly Gln Ala Glu Leu Thr Ala Ala
1               5                   10                  15

Gln Val Arg Val Ala Ala Ala Ala Tyr Glu Thr Ala Tyr Gly Leu Thr
            20                  25                  30

Val Pro Pro Pro Val Ile Ala Glu Asn Arg Ala Glu Leu Met Ile Leu
        35                  40                  45

Ile Ala Thr Asn Leu Leu Gly Gln Asn Thr Pro Ala Ile Ala Val Asn
    50                  55                  60

Glu Ala Glu Tyr Gly Glu Met Trp Ala Gln Asp Ala Ala Ala Met Phe
65                  70                  75                  80

Gly Tyr Ala Ala Ala Thr Ala Thr Ala Thr Ala Thr Leu Leu Pro Phe
                85                  90                  95

Glu Glu Ala Pro Glu Met Thr Ser Ala Gly Gly Leu Leu Glu Gln Ala
            100                 105                 110

Ala Ala Val Glu Glu Ala Ser Asp Thr Ala Ala Ala Asn Gln Leu Met
        115                 120                 125

Asn Asn Val Pro Gln Ala Leu Lys Gln Leu Ala Gln Pro Thr Gln Gly
    130                 135                 140

Thr Thr Pro Ser Ser Lys Leu Gly Gly Leu Trp Lys Thr Val Ser Pro
145                 150                 155                 160

His Arg Ser Pro Ile Ser Asn Met Val Ser Met Ala Asn Asn His Met
                165                 170                 175

Ser Met Thr Asn Ser Gly Val Ser Met Thr Asn Thr Leu Ser Ser Met
            180                 185                 190

Leu Lys Gly Phe Ala Pro Ala Ala Ala Ala Gln Ala Val Gln Thr Ala
        195                 200                 205

Ala Gln Asn Gly Val Arg Ala Met Ser Ser Leu Gly Ser Ser Leu Gly
    210                 215                 220

Ser Ser Gly Leu Gly Gly Gly Val Ala Ala Asn Leu Gly Arg Ala Ala
225                 230                 235                 240

Ser Val Arg Tyr Gly His Arg Asp Gly Gly Lys Tyr Ala Xaa Ser Gly
                245                 250                 255

Arg Arg Asn Gly Gly Pro Ala Thr Asp Ala Ala Thr Leu Ala Gln Glu
            260                 265                 270

Ala Gly Asn Phe Glu Arg Ile Ser Gly Asp Leu Lys Thr Gln Ile Asp
        275                 280                 285

Gln Val Glu Ser Thr Ala Gly Ser Leu Gln Gly Gln Trp Arg Gly Ala
    290                 295                 300

Ala Gly Thr Ala Ala Gln Ala Ala Val Val Arg Phe Gln Glu Ala Ala
305                 310                 315                 320

Asn Lys Gln Lys Gln Glu Leu Asp Glu Ile Ser Thr Asn Ile Arg Gln
                325                 330                 335

Ala Gly Val Gln Tyr Ser Arg Ala Asp Glu Glu Gln Gln Gln Ala Leu
            340                 345                 350

Ser Ser Gln Met Gly Phe
        355

<210> 9
<211> 7676
<212> DNA
<213> Artificial Sequence

<220>
<223> Description of Artificial Sequence:tetra-fusion
      protein TbRa3-38kD-Tb38-1-DPEP (designated TbF-2)

<220>
<221> CDS
<222> (5072)..(7480)

<400> 9
tggcgaatgg gacgcgccct gtagcggcgc attaagcgcg gcgggtgtgg tggttacgcg 60

cagcgtgacc gctacacttg ccagcgccct agcgcccgct cctttcgctt tcttcccttc 120

ctttctcgcc acgttcgccg gctttccccg tcaagctcta aatcgggggc tccctttagg 180

gttccgattt agtgctttac ggcacctcga ccccaaaaaa cttgattagg gtgatggttc 240

acgtagtggg ccatcgccct gatagacggt ttttcgccct ttgacgttgg agtccacgtt 300

ctttaatagt ggactcttgt tccaaactgg aacaacactc aaccctatct cggtctattc 360

ttttgattta taagggattt tgccgatttc ggcctattgg ttaaaaaatg agctgattta 420

acaaaaattt aacgcgaatt ttaacaaaat attaacgttt acaatttcag gtggcacttt 480

tcggggaaat gtgcgcggaa cccctatttg tttatttttc taaatacatt caaatatgta 540

tccgctcatg aattaattct tagaaaaact catcgagcat caaatgaaac tgcaatttat 600

tcatatcagg attatcaata ccatattttt gaaaaagccg tttctgtaat gaaggagaaa 660

actcaccgag gcagttccat aggatggcaa gatcctggta tcggtctgcg attccgactc 720

gtccaacatc aatacaacct attaatttcc cctcgtcaaa aataaggtta tcaagtgaga 780

aatcaccatg agtgacgact gaatccggtg agaatggcaa aagtttatgc atttctttcc 840

agacttgttc aacaggccag ccattacgct cgtcatcaaa atcactcgca tcaaccaaac 900

cgttattcat tcgtgattgc gcctgagcga gacgaaatac gcgatcgctg ttaaaaggac 960

aattacaaac aggaatcgaa tgcaaccggc gcaggaacac tgccagcgca tcaacaatat 1020

tttcacctga atcaggatat tcttctaata cctggaatgc tgttttcccg gggatcgcag 1080

tggtgagtaa ccatgcatca tcaggagtac ggataaaatg cttgatggtc ggaagaggca 1140

taaattccgt cagccagttt agtctgacca tctcatctgt aacatcattg gcaacgctac 1200

ctttgccatg tttcagaaac aactctggcg catcgggctt cccatacaat cgatagattg 1260

tcgcacctga ttgcccgaca ttatcgcgag cccatttata cccatataaa tcagcatcca 1320

tgttggaatt taatcgcggc ctagagcaag acgtttcccg ttgaatatgg ctcataacac 1380

cccttgtatt actgtttatg taagcagaca gttttattgt tcatgaccaa aatcccttaa 1440

cgtgagtttt cgttccactg agcgtcagac cccgtagaaa agatcaaagg atcttcttga 1500

gatccttttt ttctgcgcgt aatctgctgc ttgcaaacaa aaaaaccacc gctaccagcg 1560

gtggtttgtt tgccggatca agagctacca actctttttc cgaaggtaac tggcttcagc 1620

agagcgcaga taccaaatac tgtccttcta gtgtagccgt agttaggcca ccacttcaag 1680

aactctgtag caccgcctac atacctcgct ctgctaatcc tgttaccagt ggctgctgcc 1740

agtggcgata agtcgtgtct taccgggttg gactcaagac gatagttacc ggataaggcg 1800

cagcggtcgg gctgaacggg gggttcgtgc acacagccca gcttggagcg aacgacctac 1860

accgaactga gatacctaca gcgtgagcta tgagaaagcg ccacgcttcc cgaagggaga 1920

aaggcggaca ggtatccggt aagcggcagg gtcggaacag gagagcgcac gagggagctt 1980

ccagggggaa acgcctggta tctttatagt cctgtcgggt ttcgccacct ctgacttgag 2040

cgtcgatttt tgtgatgctc gtcagggggg cggagcctat ggaaaaacgc cagcaacgcg 2100

gcctttttac ggttcctggc cttttgctgg ccttttgctc acatgttctt tcctgcgtta 2160

tcccctgatt ctgtggataa ccgtattacc gcctttgagt gagctgatac cgctcgccgc 2220

agccgaacga ccgagcgcag cgagtcagtg agcgaggaag cggaagagcg cctgatgcgg 2280

tattttctcc ttacgcatct gtgcggtatt tcacaccgca tatatggtgc actctcagta 2340

caatctgctc tgatgccgca tagttaagcc agtatacact ccgctatcgc tacgtgactg 2400

ggtcatggct gcgccccgac acccgccaac acccgctgac gcgccctgac gggcttgtct 2460

gctcccggca tccgcttaca gacaagctgt gaccgtctcc gggagctgca tgtgtcagag 2520

gttttcaccg tcatcaccga aacgcgcgag gcagctgcgg taaagctcat cagcgtggtc 2580

gtgaagcgat tcacagatgt ctgcctgttc atccgcgtcc agctcgttga gtttctccag 2640

aagcgttaat gtctggcttc tgataaagcg ggccatgtta agggcggttt tttcctgttt 2700

ggtcactgat gcctccgtgt aagggggatt tctgttcatg ggggtaatga taccgatgaa 2760

acgagagagg atgctcacga tacgggttac tgatgatgaa catgcccggt tactggaacg 2820

ttgtgagggt aaacaactgg cggtatggat gcggcgggac cagagaaaaa tcactcaggg 2880

tcaatgccag cgcttcgtta atacagatgt aggtgttcca cagggtagcc agcagcatcc 2940

tgcgatgcag atccggaaca taatggtgca gggcgctgac ttccgcgttt ccagacttta 3000

cgaaacacgg aaaccgaaga ccattcatgt tgttgctcag gtcgcagacg ttttgcagca 3060

gcagtcgctt cacgttcgct cgcgtatcgg tgattcattc tgctaaccag taaggcaacc 3120

ccgccagcct agccgggtcc tcaacgacag gagcacgatc atgcgcaccc gtggggccgc 3180

catgccggcg ataatggcct gcttctcgcc gaaacgtttg gtggcgggac cagtgacgaa 3240

ggcttgagcg agggcgtgca agattccgaa taccgcaagc gacaggccga tcatcgtcgc 3300

gctccagcga aagcggtcct cgccgaaaat gacccagagc gctgccggca cctgtcctac 3360

gagttgcatg ataaagaaga cagtcataag tgcggcgacg atagtcatgc cccgcgccca 3420

ccggaaggag ctgactgggt tgaaggctct caagggcatc ggtcgagatc ccggtgccta 3480

atgagtgagc taacttacat taattgcgtt gcgctcactg cccgctttcc agtcgggaaa 3540

cctgtcgtgc cagctgcatt aatgaatcgg ccaacgcgcg gggagaggcg gtttgcgtat 3600

tgggcgccag ggtggttttt cttttcacca gtgagacggg caacagctga ttgcccttca 3660

ccgcctggcc ctgagagagt tgcagcaagc ggtccacgct ggtttgcccc agcaggcgaa 3720

aatcctgttt gatggtggtt aacggcggga tataacatga gctgtcttcg gtatcgtcgt 3780

atcccactac cgagatatcc gcaccaacgc gcagcccgga ctcggtaatg gcgcgcattg 3840

cgcccagcgc catctgatcg ttggcaacca gcatcgcagt gggaacgatg ccctcattca 3900

gcatttgcat ggtttgttga aaaccggaca tggcactcca gtcgccttcc cgttccgcta 3960

tcggctgaat ttgattgcga gtgagatatt tatgccagcc agccagacgc agacgcgccg 4020

agacagaact taatgggccc gctaacagcg cgatttgctg gtgacccaat gcgaccagat 4080

gctccacgcc cagtcgcgta ccgtcttcat gggagaaaat aatactgttg atgggtgtct 4140

ggtcagagac atcaagaaat aacgccggaa cattagtgca ggcagcttcc acagcaatgg 4200

catcctggtc atccagcgga tagttaatga tcagcccact gacgcgttgc gcgagaagat 4260

tgtgcaccgc cgctttacag gcttcgacgc cgcttcgttc taccatcgac accaccacgc 4320

tggcacccag ttgatcggcg cgagatttaa tcgccgcgac aatttgcgac ggcgcgtgca 4380

gggccagact ggaggtggca acgccaatca gcaacgactg tttgcccgcc agttgttgtg 4440

ccacgcggtt gggaatgtaa ttcagctccg ccatcgccgc ttccactttt tcccgcgttt 4500

tcgcagaaac gtggctggcc tggttcacca cgcgggaaac ggtctgataa gagacaccgg 4560

catactctgc gacatcgtat aacgttactg gtttcacatt caccaccctg aattgactct 4620

cttccgggcg ctatcatgcc ataccgcgaa aggttttgcg ccattcgatg gtgtccggga 4680

tctcgacgct ctcccttatg cgactcctgc attaggaagc agcccagtag taggttgagg 4740

ccgttgagca ccgccgccgc aaggaatggt gcatgcaagg agatggcgcc caacagtccc 4800

ccggccacgg ggcctgccac catacccacg ccgaaacaag cgctcatgag cccgaagtgg 4860

cgagcccgat cttccccatc ggtgatgtcg gcgatatagg cgccagcaac cgcacctgtg 4920

gcgccggtga tgccggccac gatgcgtccg gcgtagagga tcgagatctc gatcccgcga 4980

aattaatacg actcactata ggggaattgt gagcggataa caattcccct ctagaaataa 5040

ttttgtttaa ctttaagaag gagatataca t atg ggc cat cat cat cat cat 5092

cac gtg atc gac atc atc ggg acc agc ccc aca tcc tgg gaa cag gcg 5140

gcg gcg gag gcg gtc cag cgg gcg cgg gat agc gtc gat gac atc cgc 5188

gtc gct cgg gtc att gag cag gac atg gcc gtg gac agc gcc ggc aag 5236

atc acc tac cgc atc aag ctc gaa gtg tcg ttc aag atg agg ccg gcg 5284

caa ccg agg ggc tcg aaa cca ccg agc ggt tcg cct gaa acg ggc gcc 5332

ggc gcc ggt act gtc gcg act acc ccc gcg tcg tcg ccg gtg acg ttg 5380

gcg gag acc ggt agc acg ctg ctc tac ccg ctg ttc aac ctg tgg ggt 5428

ccg gcc ttt cac gag agg tat ccg aac gtc acg atc acc gct cag ggc 5476

acc ggt tct ggt gcc ggg atc gcg cag gcc gcc gcc ggg acg gtc aac 5524

att ggg gcc tcc gac gcc tat ctg tcg gaa ggt gat atg gcc gcg cac 5572

aag ggg ctg atg aac atc gcg cta gcc atc tcc gct cag cag gtc aac 5620

tac aac ctg ccc gga gtg agc gag cac ctc aag ctg aac gga aaa gtc 5668

ctg gcg gcc atg tac cag ggc acc atc aaa acc tgg gac gac ccg cag 5716

atc gct gcg ctc aac ccc ggc gtg aac ctg ccc ggc acc gcg gta gtt 5764

ccg ctg cac cgc tcc gac ggg tcc ggt gac acc ttc ttg ttc acc cag 5812

tac ctg tcc aag caa gat ccc gag ggc tgg ggc aag tcg ccc ggc ttc 5860

ggc acc acc gtc gac ttc ccg gcg gtg ccg ggt gcg ctg ggt gag aac 5908

ggc aac ggc ggc atg gtg acc ggt tgc gcc gag aca ccg ggc tgc gtg 5956

gcc tat atc ggc atc agc ttc ctc gac cag gcc agt caa cgg gga ctc 6004

ggc gag gcc caa cta ggc aat agc tct ggc aat ttc ttg ttg ccc gac 6052

gcg caa agc att cag gcc gcg gcg gct ggc ttc gca tcg aaa acc ccg 6100

gcg aac cag gcg att tcg atg atc gac ggg ccc gcc ccg gac ggc tac 6148

ccg atc atc aac tac gag tac gcc atc gtc aac aac cgg caa aag gac 6196

gcc gcc acc gcg cag acc ttg cag gca ttt ctg cac tgg gcg atc acc 6244

gac ggc aac aag gcc tcg ttc ctc gac cag gtt cat ttc cag ccg ctg 6292

ccg ccc gcg gtg gtg aag ttg tct gac gcg ttg atc gcg acg att tcc 6340

agc gct gag atg aag acc gat gcc gct acc ctc gcg cag gag gca ggt 6388

aat ttc gag cgg atc tcc ggc gac ctg aaa acc cag atc gac cag gtg 6436

gag tcg acg gca ggt tcg ttg cag ggc cag tgg cgc ggc gcg gcg ggg 6484

acg gcc gcc cag gcc gcg gtg gtg cgc ttc caa gaa gca gcc aat aag 6532

cag aag cag gaa ctc gac gag atc tcg acg aat att cgt cag gcc ggc 6580

gtc caa tac tcg agg gcc gac gag gag cag cag cag gcg ctg tcc tcg 6628

caa atg ggc ttt gtg ccc aca acg gcc gcc tcg ccg ccg tcg acc gct 6676

gca gcg cca ccc gca ccg gcg aca cct gtt gcc ccc cca cca ccg gcc 6724

gcc gcc aac acg ccg aat gcc cag ccg ggc gat ccc aac gca gca cct 6772

ccg ccg gcc gac ccg aac gca ccg ccg cca cct gtc att gcc cca aac 6820

gca ccc caa cct gtc cgg atc gac aac ccg gtt gga gga ttc agc ttc 6868

gcg ctg cct gct ggc tgg gtg gag tct gac gcc gcc cac ttc gac tac 6916

ggt tca gca ctc ctc agc aaa acc acc ggg gac ccg cca ttt ccc gga 6964

cag ccg ccg ccg gtg gcc aat gac acc cgt atc gtg ctc ggc cgg cta 7012

gac caa aag ctt tac gcc agc gcc gaa gcc acc gac tcc aag gcc gcg 7060

gcc cgg ttg ggc tcg gac atg ggt gag ttc tat atg ccc tac ccg ggc 7108

acc cgg atc aac cag gaa acc gtc tcg ctt gac gcc aac ggg gtg tct 7156

gga agc gcg tcg tat tac gaa gtc aag ttc agc gat ccg agt aag ccg 7204

aac ggc cag atc tgg acg ggc gta atc ggc tcg ccc gcg gcg aac gca 7252

ccg gac gcc ggg ccc cct cag cgc tgg ttt gtg gta tgg ctc ggg acc 7300

gcc aac aac ccg gtg gac aag ggc gcg gcc aag gcg ctg gcc gaa tcg 7348

atc cgg cct ttg gtc gcc ccg ccg ccg gcg ccg gca ccg gct cct gca 7396

gag ccc gct ccg gcg ccg gcg ccg gcc ggg gaa gtc gct cct acc ccg 7444

acg aca ccg aca ccg cag cgg acc tta ccg gcc tgagaattct gcagatatcc 7497

atcacactgg cggccgctcg agcaccacca ccaccaccac tgagatccgg ctgctaacaa 7557

agcccgaaag gaagctgagt tggctgctgc caccgctgag caataactag cataacccct 7617

tggggcctct aaacgggtct tgaggggttt tttgctgaaa ggaggaacta tatccggat 7676

<210> 10
<211> 802
<212> PRT
<213> Artificial Sequence
<223> Description of Artificial Sequence:tetra-fusion
      protein TbRa3-38kD-Tb38-1-DPEP (designated TbF-2)

<400> 10
Met Gly His His His His His His Val Ile Asp Ile Ile Gly Thr Ser
  1               5                  10                  15

Pro Thr Ser Trp Glu Gln Ala Ala Ala Glu Ala Val Gln Arg Ala Arg
             20                  25                  30

Asp Ser Val Asp Asp Ile Arg Val Ala Arg Val Ile Glu Gln Asp Met
         35                  40                  45

Ala Val Asp Ser Ala Gly Lys Ile Thr Tyr Arg Ile Lys Leu Glu Val
     50                  55                  60

Ser Phe Lys Met Arg Pro Ala Gln Pro Arg Gly Ser Lys Pro Pro Ser
 65                  70                  75                  80

Gly Ser Pro Glu Thr Gly Ala Gly Ala Gly Thr Val Ala Thr Thr Pro
                 85                  90                  95

Ala Ser Ser Pro Val Thr Leu Ala Glu Thr Gly Ser Thr Leu Leu Tyr
            100                 105                 110

Pro Leu Phe Asn Leu Trp Gly Pro Ala Phe His Glu Arg Tyr Pro Asn
        115                 120                 125

Val Thr Ile Thr Ala Gln Gly Thr Gly Ser Gly Ala Gly Ile Ala Gln
    130                 135                 140

Ala Ala Ala Gly Thr Val Asn Ile Gly Ala Ser Asp Ala Tyr Leu Ser
145                 150                 155                 160

Glu Gly Asp Met Ala Ala His Lys Gly Leu Met Asn Ile Ala Leu Ala
                165                 170                 175

Ile Ser Ala Gln Gln Val Asn Tyr Asn Leu Pro Gly Val Ser Glu His
            180                 185                 190

Leu Lys Leu Asn Gly Lys Val Leu Ala Ala Met Tyr Gln Gly Thr Ile
        195                 200                 205

Lys Thr Trp Asp Asp Pro Gln Ile Ala Ala Leu Asn Pro Gly Val Asn
    210                 215                 220

Leu Pro Gly Thr Ala Val Val Pro Leu His Arg Ser Asp Gly Ser Gly
225                 230                 235                 240

Asp Thr Phe Leu Phe Thr Gln Tyr Leu Ser Lys Gln Asp Pro Glu Gly
                245                 250                 255

Trp Gly Lys Ser Pro Gly Phe Gly Thr Thr Val Asp Phe Pro Ala Val
            260                 265                 270

Pro Gly Ala Leu Gly Glu Asn Gly Asn Gly Gly Met Val Thr Gly Cys
        275                 280                 285

Ala Glu Thr Pro Gly Cys Val Ala Tyr Ile Gly Ile Ser Phe Leu Asp
    290                 295                 300

Gln Ala Ser Gln Arg Gly Leu Gly Glu Ala Gln Leu Gly Asn Ser Ser
305                 310                 315                 320

Gly Asn Phe Leu Leu Pro Asp Ala Gln Ser Ile Gln Ala Ala Ala Ala
                325                 330                 335

Gly Phe Ala Ser Lys Thr Pro Ala Asn Gln Ala Ile Ser Met Ile Asp
            340                 345                 350

Gly Pro Ala Pro Asp Gly Tyr Pro Ile Ile Asn Tyr Glu Tyr Ala Ile
        355                 360                 365

Val Asn Asn Arg Gln Lys Asp Ala Ala Thr Ala Gln Thr Leu Gln Ala
    370                 375                 380

Phe Leu His Trp Ala Ile Thr Asp Gly Asn Lys Ala Ser Phe Leu Asp
385                 390                 395                 400

Gln Val His Phe Gln Pro Leu Pro Pro Ala Val Val Lys Leu Ser Asp
                405                 410                 415

Ala Leu Ile Ala Thr Ile Ser Ser Ala Glu Met Lys Thr Asp Ala Ala
            420                 425                 430

Thr Leu Ala Gln Glu Ala Gly Asn Phe Glu Arg Ile Ser Gly Asp Leu
        435                 440                 445

Lys Thr Gln Ile Asp Gln Val Glu Ser Thr Ala Gly Ser Leu Gln Gly
    450                 455                 460

Gln Trp Arg Gly Ala Ala Gly Thr Ala Ala Gln Ala Ala Val Val Arg
465                 470                 475                 480

Phe Gln Glu Ala Ala Asn Lys Gln Lys Gln Glu Leu Asp Glu Ile Ser
                485                 490                 495

Thr Asn Ile Arg Gln Ala Gly Val Gln Tyr Ser Arg Ala Asp Glu Glu
            500                 505                 510

Gln Gln Gln Ala Leu Ser Ser Gln Met Gly Phe Val Pro Thr Thr Ala
        515                 520                 525

Ala Ser Pro Pro Ser Thr Ala Ala Ala Pro Pro Ala Pro Ala Thr Pro
    530                 535                 540

Val Ala Pro Pro Pro Pro Ala Ala Ala Asn Thr Pro Asn Ala Gln Pro
545                 550                 555                 560

Gly Asp Pro Asn Ala Ala Pro Pro Pro Ala Asp Pro Asn Ala Pro Pro
                565                 570                 575

Pro Pro Val Ile Ala Pro Asn Ala Pro Gln Pro Val Arg Ile Asp Asn
            580                 585                 590

Pro Val Gly Gly Phe Ser Phe Ala Leu Pro Ala Gly Trp Val Glu Ser
        595                 600                 605

Asp Ala Ala His Phe Asp Tyr Gly Ser Ala Leu Leu Ser Lys Thr Thr
    610                 615                 620

Gly Asp Pro Pro Phe Pro Gly Gln Pro Pro Pro Val Ala Asn Asp Thr
625                 630                 635                 640

Arg Ile Val Leu Gly Arg Leu Asp Gln Lys Leu Tyr Ala Ser Ala Glu
                645                 650                 655

Ala Thr Asp Ser Lys Ala Ala Ala Arg Leu Gly Ser Asp Met Gly Glu
            660                 665                 670

Phe Tyr Met Pro Tyr Pro Gly Thr Arg Ile Asn Gln Glu Thr Val Ser
        675                 680                 685

Leu Asp Ala Asn Gly Val Ser Gly Ser Ala Ser Tyr Tyr Glu Val Lys
    690                 695                 700

Phe Ser Asp Pro Ser Lys Pro Asn Gly Gln Ile Trp Thr Gly Val Ile
705                 710                 715                 720

Gly Ser Pro Ala Ala Asn Ala Pro Asp Ala Gly Pro Pro Gln Arg Trp
                725                 730                 735

Phe Val Val Trp Leu Gly Thr Ala Asn Asn Pro Val Asp Lys Gly Ala
            740                 745                 750

Ala Lys Ala Leu Ala Glu Ser Ile Arg Pro Leu Val Ala Pro Pro Pro
        755                 760                 765

Ala Pro Ala Pro Ala Pro Ala Glu Pro Ala Pro Ala Pro Ala Pro Ala
    770                 775                 780

Gly Glu Val Ala Pro Thr Pro Thr Thr Pro Thr Pro Gln Arg Thr Leu
785                 790                 795                 800

Pro Ala

<210> 11
<211> 2577
<212> DNA
<213> Artificial Sequence

<220>
<223> Description of Artificial Sequence:penta-fusion

protein Erd14-DPV-MTI-MSL-MTCC2 (designated
      Mtb88f)

<220>
<221> CDS
<222> (1)..(2571)

<400> 11
cat atg cat cac cat cac cat cac atg gcc acc acc ctt ccc gtt cag    48

cgc cac ccg cgg tcc ctc ttc ccc gag ttt tct gag ctg ttc gcg gcc    96

ttc ccg tca ttc gcc gga ctc cgg ccc acc ttc gac acc cgg ttg atg   144

cgg ctg gaa gac gag atg aaa gag ggg cgc tac gag gta cgc gcg gag   192

ctt ccc ggg gtc gac ccc gac aag gac gtc gac att atg gtc cgc gat   240

ggt cag ctg acc atc aag gcc gag cgc acc gag cag aag gac ttc gac   288

ggt cgc tcg gaa ttc gcg tac ggt tcc ttc gtt cgc acg gtg tcg ctg   336

ccg gta ggt gct gac gag gac gac att aag gcc acc tac gac aag ggc   384

att ctt act gtg tcg gtg gcg gtt tcg gaa ggg aag cca acc gaa aag   432

cac att cag atc cgg tcc acc aac aag ctt gat ccc gtg gac gcg gtc   480

att aac acc acc tgc aat tac ggg cag gta gta gct gcg ctc aac gcg   528

acg gat ccg ggg gct gcc gca cag ttc aac gcc tca ccg gtg gcg cag   576

tcc tat ttg cgc aat ttc ctc gcc gca ccg cca cct cag cgc gct gcc   624

atg gcc gcg caa ttg caa gct gtg ccg ggg gcg gca cag tac atc ggc   672

ctt gtc gag tcg gtt gcc ggc tcc tgc aac aac tat gag ctc atg acg   720

att aat tac cag ttc ggg gac gtc gac gct cat ggc gcc atg atc cgc   768

gct cag gcg gcg tcg ctt gag gcg gag cat cag gcc atc gtt cgt gat   816

gtg ttg gcc gcg ggt gac ttt tgg ggc ggc gcc ggt tcg gtg gct tgc   864

cag gag ttc att acc cag ttg ggc cgt aac ttc cag gtg atc tac gag   912

cag gcc aac gcc cac ggg cag aag gtg cag gct gcc ggc aac aac atg   960

gcg caa acc gac agc gcc gtc ggc tcc agc tgg gcc act agt atg agc  1008

ctt ttg gat gct cat atc cca cag ttg gtg gcc tcc cag tcg gcg ttt  1056

gcc gcc aag gcg ggg ctg atg cgg cac acg atc ggt cag gcc gag cag  1104

gcg gcg atg tcg gct cag gcg ttt cac cag ggg gag tcg tcg gcg gcg  1152

ttt cag gcc gcc cat gcc cgg ttt gtg gcg gcg gcc gcc aaa gtc aac  1200

acc ttg ttg gat gtc gcg cag gcg aat ctg ggt gag gcc gcc ggt acc  1248

tat gtg gcc gcc gat gct gcg gcc gcg tcg acc tat acc ggg ttc gat 1296

atc atg gat ttc ggg ctt tta cct ccg gaa gtg aat tca agc cga atg 1344

tat tcc ggt ccg ggg ccg gag tcg atg cta gcc gcc gcg gcc gcc tgg 1392

gac ggt gtg gcc gcg gag ttg act tcc gcc gcg gtc tcg tat gga tcg 1440

gtg gtg tcg acg ctg atc gtt gag ccg tgg atg ggg ccg gcg gcg gcc 1488

gcg atg gcg gcc gcg gca acg ccg tat gtg ggg tgg ctg gcc gcc acg 1536

gcg gcg ctg gcg aag gag acg gcc aca cag gcg agg gca gcg gcg gaa 1584

gcg ttt ggg acg gcg ttc gcg atg acg gtg cca cca tcc ctc gtc gcg 1632

gcc aac cgc agc cgg ttg atg tcg ctg gtc gcg gcg aac att ctg ggg 1680

caa aac agt gcg gcg atc gcg gct acc cag gcc gag tat gcc gaa atg 1728

tgg gcc caa gac gct gcc gtg atg tac agc tat gag ggg gca tct gcg 1776

gcc gcg tcg gcg ttg ccg ccg ttc act cca ccc gtg caa ggc acc ggc 1824

ccg gcc ggg ccc gcg gcc gca gcc gcg gcg acc caa gcc gcc ggt gcg 1872

ggc gcc gtt gcg gat gca cag gcg aca ctg gcc cag ctg ccc ccg ggg 1920

atc ctg agc gac att ctg tcc gca ttg gcc gcc aac gct gat ccg ctg 1968

aca tcg gga ctg ttg ggg atc gcg tcg acc ctc aac ccg caa gtc gga 2016

tcc gct cag ccg ata gtg atc ccc acc ccg ata ggg gaa ttg gac gtg 2064

atc gcg ctc tac att gca tcc atc gcg acc ggc agc att gcg ctc gcg 2112

atc acg aac acg gcc aga ccc tgg cac atc ggc cta tac ggg aac gcc 2160

ggc ggg ctg gga ccg acg cag ggc cat cca ctg agt tcg gcg acc gac 2208

gag ccg gag ccg cac tgg ggc ccc ttc ggg ggc gcg gcg ccg gtg tcc 2256

gcg ggc gtc ggc cac gca gca tta gtc gga gcg ttg tcg gtg ccg cac 2304

agc tgg acc acg gcc gcc ccg gag atc cag ctc gcc gtt cag gca aca 2352

ccc acc ttc agc tcc agc gcc ggc gcc gac ccg acg gcc cta aac ggg 2400

atg ccg gca ggc ctg ctc agc ggg atg gct ttg gcg agc ctg gcc gca 2448

cgc ggc acg acg ggc ggt ggc ggc acc cgt agc ggc acc agc act gac 2496

ggc caa gag gac ggc cgc aaa ccc ccg gta gtt gtg att aga gag cag 2544

ccg ccg ccc gga aac ccc ccg cgg taagatatc 2577

<210> 12
<211> 856

<212> PRT
<213> Artificial Sequence
<223> Description of Artificial Sequence:penta-fusion
      protein Erd14-DPV-MTI-MSL-MTCC2 (designated
      Mtb88f)

<400> 12
His Met His His His His His His Met Ala Thr Thr Leu Pro Val Gln
 1               5                  10                  15

Arg His Pro Arg Ser Leu Phe Pro Glu Phe Ser Glu Leu Phe Ala Ala
            20                  25                  30

Phe Pro Ser Phe Ala Gly Leu Arg Pro Thr Phe Asp Thr Arg Leu Met
        35                  40                  45

Arg Leu Glu Asp Glu Met Lys Glu Gly Arg Tyr Glu Val Arg Ala Glu
    50                  55                  60

Leu Pro Gly Val Asp Pro Asp Lys Asp Val Asp Ile Met Val Arg Asp
 65                  70                  75                  80

Gly Gln Leu Thr Ile Lys Ala Glu Arg Thr Glu Gln Lys Asp Phe Asp
                85                  90                  95

Gly Arg Ser Glu Phe Ala Tyr Gly Ser Phe Val Arg Thr Val Ser Leu
            100                 105                 110

Pro Val Gly Ala Asp Glu Asp Asp Ile Lys Ala Thr Tyr Asp Lys Gly
        115                 120                 125

Ile Leu Thr Val Ser Val Ala Val Ser Glu Gly Lys Pro Thr Glu Lys
    130                 135                 140

His Ile Gln Ile Arg Ser Thr Asn Lys Leu Asp Pro Val Asp Ala Val
145                 150                 155                 160

Ile Asn Thr Thr Cys Asn Tyr Gly Gln Val Val Ala Ala Leu Asn Ala
                165                 170                 175

Thr Asp Pro Gly Ala Ala Ala Gln Phe Asn Ala Ser Pro Val Ala Gln
            180                 185                 190

Ser Tyr Leu Arg Asn Phe Leu Ala Ala Pro Pro Pro Gln Arg Ala Ala
        195                 200                 205

Met Ala Ala Gln Leu Gln Ala Val Pro Gly Ala Ala Gln Tyr Ile Gly
    210                 215                 220

Leu Val Glu Ser Val Ala Gly Ser Cys Asn Asn Tyr Glu Leu Met Thr
225                 230                 235                 240

Ile Asn Tyr Gln Phe Gly Asp Val Asp Ala His Gly Ala Met Ile Arg
                245                 250                 255

Ala Gln Ala Ala Ser Leu Glu Ala Glu His Gln Ala Ile Val Arg Asp
            260                 265                 270

Val Leu Ala Ala Gly Asp Phe Trp Gly Gly Ala Gly Ser Val Ala Cys
        275                 280                 285

Gln Glu Phe Ile Thr Gln Leu Gly Arg Asn Phe Gln Val Ile Tyr Glu
    290                 295                 300

Gln Ala Asn Ala His Gly Gln Lys Val Gln Ala Ala Gly Asn Asn Met
305                 310                 315                 320

Ala Gln Thr Asp Ser Ala Val Gly Ser Ser Trp Ala Thr Ser Met Ser
                325                 330                 335

Leu Leu Asp Ala His Ile Pro Gln Leu Val Ala Ser Gln Ser Ala Phe
            340                 345                 350

Ala Ala Lys Ala Gly Leu Met Arg His Thr Ile Gly Gln Ala Glu Gln
        355                 360                 365

Ala Ala Met Ser Ala Gln Ala Phe His Gln Gly Glu Ser Ser Ala Ala
    370                 375                 380

Phe Gln Ala Ala His Ala Arg Phe Val Ala Ala Ala Ala Lys Val Asn
385                 390                 395                 400

Thr Leu Leu Asp Val Ala Gln Ala Asn Leu Gly Glu Ala Ala Gly Thr
                405                 410                 415

Tyr Val Ala Ala Asp Ala Ala Ala Ala Ser Thr Tyr Thr Gly Phe Asp
            420                 425                 430

Ile Met Asp Phe Gly Leu Leu Pro Pro Glu Val Asn Ser Ser Arg Met
        435                 440                 445

Tyr Ser Gly Pro Gly Pro Glu Ser Met Leu Ala Ala Ala Ala Ala Trp
    450                 455                 460

Asp Gly Val Ala Ala Glu Leu Thr Ser Ala Ala Val Ser Tyr Gly Ser
465                 470                 475                 480

Val Val Ser Thr Leu Ile Val Glu Pro Trp Met Gly Pro Ala Ala Ala
                485                 490                 495

Ala Met Ala Ala Ala Ala Thr Pro Tyr Val Gly Trp Leu Ala Ala Thr
            500                 505                 510

Ala Ala Leu Ala Lys Glu Thr Ala Thr Gln Ala Arg Ala Ala Ala Glu
        515                 520                 525

Ala Phe Gly Thr Ala Phe Ala Met Thr Val Pro Pro Ser Leu Val Ala
    530                 535                 540

Ala Asn Arg Ser Arg Leu Met Ser Leu Val Ala Ala Asn Ile Leu Gly
545                 550                 555                 560

Gln Asn Ser Ala Ala Ile Ala Ala Thr Gln Ala Glu Tyr Ala Glu Met
                565                 570                 575

Trp Ala Gln Asp Ala Ala Val Met Tyr Ser Tyr Glu Gly Ala Ser Ala
            580                 585                 590

Ala Ala Ser Ala Leu Pro Pro Phe Thr Pro Pro Val Gln Gly Thr Gly
        595                 600                 605

Pro Ala Gly Pro Ala Ala Ala Ala Ala Ala Thr Gln Ala Ala Gly Ala
    610                 615                 620

Gly Ala Val Ala Asp Ala Gln Ala Thr Leu Ala Gln Leu Pro Pro Gly
625                 630                 635                 640

Ile Leu Ser Asp Ile Leu Ser Ala Leu Ala Ala Asn Ala Asp Pro Leu
                645                 650                 655

Thr Ser Gly Leu Leu Gly Ile Ala Ser Thr Leu Asn Pro Gln Val Gly
            660                 665                 670

Ser Ala Gln Pro Ile Val Ile Pro Thr Pro Ile Gly Glu Leu Asp Val
        675                 680                 685

Ile Ala Leu Tyr Ile Ala Ser Ile Ala Thr Gly Ser Ile Ala Leu Ala
    690                 695                 700

Ile Thr Asn Thr Ala Arg Pro Trp His Ile Gly Leu Tyr Gly Asn Ala
705                 710                 715                 720

Gly Gly Leu Gly Pro Thr Gln Gly His Pro Leu Ser Ser Ala Thr Asp
                725                 730                 735

Glu Pro Glu Pro His Trp Gly Pro Phe Gly Gly Ala Ala Pro Val Ser
            740                 745                 750

Ala Gly Val Gly His Ala Ala Leu Val Gly Ala Leu Ser Val Pro His
        755                 760                 765

Ser Trp Thr Thr Ala Ala Pro Glu Ile Gln Leu Ala Val Gln Ala Thr
    770                 775                 780

Pro Thr Phe Ser Ser Ser Ala Gly Ala Asp Pro Thr Ala Leu Asn Gly
785                 790                 795                 800

Met Pro Ala Gly Leu Leu Ser Gly Met Ala Leu Ala Ser Leu Ala Ala
                805                 810                 815

Arg Gly Thr Thr Gly Gly Gly Gly Thr Arg Ser Gly Thr Ser Thr Asp
            820                 825                 830

Gly Gln Glu Asp Gly Arg Lys Pro Pro Val Val Val Ile Arg Glu Gln
        835                 840                 845

Pro Pro Pro Gly Asn Pro Pro Arg
    850                 855

<210> 13
<211> 1299
<212> DNA
<213> Artificial Sequence

<220>
<223> Description of Artificial Sequence:tetra-fusion
      protein Erd14-DPV-MTI-MSL (designated Mtb46f)

<220>
<221> CDS
<222> (1)..(1299)

<400> 13
cat atg cat cac cat cac cat cac atg gcc acc acc ctt ccc gtt cag     48

cgc cac ccg cgg tcc ctc ttc ccc gag ttt tct gag ctg ttc gcg gcc     96

ttc ccg tca ttc gcc gga ctc cgg ccc acc ttc gac acc cgg ttg atg    144

cgg ctg gaa gac gag atg aaa gag ggg cgc tac gag gta cgc gcg gag    192

ctt ccc ggg gtc gac ccc gac aag gac gtc gac att atg gtc cgc gat    240

ggt cag ctg acc atc aag gcc gag cgc acc gag cag aag gac ttc gac    288

ggt cgc tcg gaa ttc gcg tac ggt tcc ttc gtt cgc acg gtg tcg ctg    336

ccg gta ggt gct gac gag gac gac att aag gcc acc tac gac aag ggc    384

att ctt act gtg tcg gtg gcg gtt tcg gaa ggg aag cca acc gaa aag    432

cac att cag atc cgg tcc acc aac aag ctt gat ccc gtg gac gcg gtc    480

att aac acc acc tgc aat tac ggg cag gta gta gct gcg ctc aac gcg    528

acg gat ccg ggg gct gcc gca cag ttc aac gcc tca ccg gtg gcg cag    576

tcc tat ttg cgc aat ttc ctc gcc gca ccg cca cct cag cgc gct gcc    624

atg gcc gcg caa ttg caa gct gtg ccg ggg gcg gca cag tac atc ggc    672

ctt gtc gag tcg gtt gcc ggc tcc tgc aac aac tat gag ctc atg acg    720

att aat tac cag ttc ggg gac gtc gac gct cat ggc gcc atg atc cgc    768

gct cag gcg gcg tcg ctt gag gcg gag cat cag gcc atc gtt cgt gat    816

gtg ttg gcc gcg ggt gac ttt tgg ggc ggc gcc ggt tcg gtg gct tgc    864

cag gag ttc att acc cag ttg ggc cgt aac ttc cag gtg atc tac gag    912

cag gcc aac gcc cac ggg cag aag gtg cag gct gcc ggc aac aac atg    960

gcg caa acc gac agc gcc gtc ggc tcc agc tgg gcc act agt atg agc   1008

ctt ttg gat gct cat atc cca cag ttg gtg gcc tcc cag tcg gcg ttt   1056

gcc gcc aag gcg ggg ctg atg cgg cac acg atc ggt cag gcc gag cag   1104

gcg gcg atg tcg gct cag gcg ttt cac cag ggg gag tcg tcg gcg gcg   1152

ttt cag gcc gcc cat gcc cgg ttt gtg gcg gcg gcc gcc aaa gtc aac   1200

acc ttg ttg gat gtc gcg cag gcg aat ctg ggt gag gcc gcc ggt acc   1248

tat gtg gcc gcc gat gct gcg gcc gcg tcg acc tat acc ggg ttc gat   1296

atc                                                               1299

<210> 14
<211> 433
<212> PRT
<213> Artificial Sequence
<223> Description of Artificial Sequence:tetra-fusion
      protein Erd14-DPV-MTI-MSL (designated Mtb46f)

<400> 14
His Met His His His His His His Met Ala Thr Thr Leu Pro Val Gln
  1               5                  10                  15

Arg His Pro Arg Ser Leu Phe Pro Glu Phe Ser Glu Leu Phe Ala Ala
             20                  25                  30

Phe Pro Ser Phe Ala Gly Leu Arg Pro Thr Phe Asp Thr Arg Leu Met
         35                  40                  45

Arg Leu Glu Asp Glu Met Lys Glu Gly Arg Tyr Glu Val Arg Ala Glu
     50                  55                  60

Leu Pro Gly Val Asp Pro Asp Lys Asp Val Asp Ile Met Val Arg Asp
 65                  70                  75                  80

Gly Gln Leu Thr Ile Lys Ala Glu Arg Thr Glu Gln Lys Asp Phe Asp
                 85                  90                  95

Gly Arg Ser Glu Phe Ala Tyr Gly Ser Phe Val Arg Thr Val Ser Leu
            100                 105                 110

Pro Val Gly Ala Asp Glu Asp Asp Ile Lys Ala Thr Tyr Asp Lys Gly
        115                 120                 125

Ile Leu Thr Val Ser Val Ala Val Ser Glu Gly Lys Pro Thr Glu Lys
    130                 135                 140

His Ile Gln Ile Arg Ser Thr Asn Lys Leu Asp Pro Val Asp Ala Val
145                 150                 155                 160

Ile Asn Thr Thr Cys Asn Tyr Gly Gln Val Val Ala Ala Leu Asn Ala
                165                 170                 175

Thr Asp Pro Gly Ala Ala Ala Gln Phe Asn Ala Ser Pro Val Ala Gln
            180                 185                 190

Ser Tyr Leu Arg Asn Phe Leu Ala Ala Pro Pro Pro Gln Arg Ala Ala
        195                 200                 205

Met Ala Ala Gln Leu Gln Ala Val Pro Gly Ala Ala Gln Tyr Ile Gly
    210                 215                 220

Leu Val Glu Ser Val Ala Gly Ser Cys Asn Asn Tyr Glu Leu Met Thr
225                 230                 235                 240

Ile Asn Tyr Gln Phe Gly Asp Val Asp Ala His Gly Ala Met Ile Arg
                245                 250                 255

Ala Gln Ala Ala Ser Leu Glu Ala Glu His Gln Ala Ile Val Arg Asp

260                 265                 270

Val Leu Ala Ala Gly Asp Phe Trp Gly Gly Ala Gly Ser Val Ala Cys
        275                 280                 285

Gln Glu Phe Ile Thr Gln Leu Gly Arg Asn Phe Gln Val Ile Tyr Glu
    290                 295                 300

Gln Ala Asn Ala His Gly Gln Lys Val Gln Ala Ala Gly Asn Asn Met
305                 310                 315                 320

Ala Gln Thr Asp Ser Ala Val Gly Ser Ser Trp Ala Thr Ser Met Ser
                325                 330                 335

Leu Leu Asp Ala His Ile Pro Gln Leu Val Ala Ser Gln Ser Ala Phe
            340                 345                 350

Ala Ala Lys Ala Gly Leu Met Arg His Thr Ile Gly Gln Ala Glu Gln
        355                 360                 365

Ala Ala Met Ser Ala Gln Ala Phe His Gln Gly Glu Ser Ser Ala Ala
    370                 375                 380

Phe Gln Ala Ala His Ala Arg Phe Val Ala Ala Ala Ala Lys Val Asn
385                 390                 395                 400

Thr Leu Leu Asp Val Ala Gln Ala Asn Leu Gly Glu Ala Ala Gly Thr
                405                 410                 415

Tyr Val Ala Ala Asp Ala Ala Ala Ala Ser Thr Tyr Thr Gly Phe Asp
            420                 425                 430

Ile

<210> 15
<211> 2168
<212> DNA
<213> Artificial Sequence

<220>
<223> Description of Artificial Sequence:tetra-fusion
      protein DPV-MTI-MSL-MTCC2 (designated Mtb71f)

<220>
<221> CDS
<222> (1)..(2133)

<400> 15
cat atg cat cac cat cac cat cac gat ccc gtg gac gcg gtc att aac      48

acc acc tgc aat tac ggg cag gta gta gct gcg ctc aac gcg acg gat      96

ccg ggg gct gcc gca cag ttc aac gcc tca ccg gtg gcg cag tcc tat     144

ttg cgc aat ttc ctc gcc gca ccg cca cct cag cgc gct gcc atg gcc     192

gcg caa ttg caa gct gtg ccg ggg gcg gca cag tac atc ggc ctt gtc     240

gag tcg gtt gcc ggc tcc tgc aac aac tat gag ctc atg acg att aat 288

tac cag ttc ggg gac gtc gac gct cat ggc gcc atg atc cgc gct cag 336

gcg gcg tcg ctt gag gcg gag cat cag gcc atc gtt cgt gat gtg ttg 384

gcc gcg ggt gac ttt tgg ggc ggc gcc ggt tcg gtg gct tgc cag gag 432

ttc att acc cag ttg ggc cgt aac ttc cag gtg atc tac gag cag gcc 480

aac gcc cac ggg cag aag gtg cag gct gcc ggc aac aac atg gcg caa 528

acc gac agc gcc gtc ggc tcc agc tgg gcc act agt atg agc ctt ttg 576

gat gct cat atc cca cag ttg gtg gcc tcc cag tcg gcg ttt gcc gcc 624

aag gcg ggg ctg atg cgg cac acg atc ggt cag gcc gag cag gcg gcg 672

atg tcg gct cag gcg ttt cac cag ggg gag tcg tcg gcg gcg ttt cag 720

gcc gcc cat gcc cgg ttt gtg gcg gcg gcc gcc aaa gtc aac acc ttg 768

ttg gat gtc gcg cag gcg aat ctg ggt gag gcc gcc ggt acc tat gtg 816

gcc gcc gat gct gcg gcc gcg tcg acc tat acc ggg ttc gat atc atg 864

gat ttc ggg ctt tta cct ccg gaa gtg aat tca agc cga atg tat tcc 912

ggt ccg ggg ccg gag tcg atg cta gcc gcc gcg gcc gcc tgg gac ggt 960

gtg gcc gcg gag ttg act tcc gcc gcg gtc tcg tat gga tcg gtg gtg 1008

tcg acg ctg atc gtt gag ccg tgg atg ggg ccg gcg gcg gcc gcg atg 1056

gcg gcc gcg gca acg ccg tat gtg ggg tgg ctg gcc gcc acg gcg gcg 1104

ctg gcg aag gag acg gcc aca cag gcg agg gca gcg gcg gaa gcg ttt 1152

ggg acg gcg ttc gcg atg acg gtg cca cca tcc ctc gtc gcg gcc aac 1200

cgc agc cgg ttg atg tcg ctg gtc gcg gcg aac att ctg ggg caa aac 1248

agt gcg gcg atc gcg gct acc cag gcc gag tat gcc gaa atg tgg gcc 1296

caa gac gct gcc gtg atg tac agc tat gag ggg gca tct gcg gcc gcg 1344

tcg gcg ttg ccg ccg ttc act cca ccc gtg caa ggc acc ggc ccg gcc 1392

ggg ccc gcg gcc gca gcc gcg gcg acc caa gcc gcc ggt gcg ggc gcc 1440

gtt gcg gat gca cag gcg aca ctg gcc cag ctg ccc ccg ggg atc ctg 1488

agc gac att ctg tcc gca ttg gcc gcc aac gct gat ccg ctg aca tcg 1536

gga ctg ttg ggg atc gcg tcg acc ctc aac ccg caa gtc gga tcc gct 1584

cag ccg ata gtg atc ccc acc ccg ata ggg gaa ttg gac gtg atc gcg 1632

ctc tac att gca tcc atc gcg acc ggc agc att gcg ctc gcg atc acg 1680

aac acg gcc aga ccc tgg cac atc ggc cta tac ggg aac gcc ggc ggg 1728

ctg gga ccg acg cag ggc cat cca ctg agt tcg gcg acc gac gag ccg 1776

gag ccg cac tgg ggc ccc ttc ggg ggc gcg gcg ccg gtg tcc gcg ggc 1824

gtc ggc cac gca gca tta gtc gga gcg ttg tcg gtg ccg cac agc tgg 1872

acc acg gcc gcc ccg gag atc cag ctc gcc gtt cag gca aca ccc acc 1920

ttc agc tcc agc gcc ggc gcc gac ccg acg gcc cta aac ggg atg ccg 1968

gca ggc ctg ctc agc ggg atg gct ttg gcg agc ctg gcc gca cgc ggc 2016

acg acg ggc ggt ggc ggc acc cgt agc ggc acc agc act gac ggc caa 2064

gag gac ggc cgc aaa ccc ccg gta gtt gtg att aga gag cag ccg ccg 2112

ccc gga aac ccc ccg cgg taagatttct aaatccatca cactggcggc cgctcgag 2168

<210> 16
<211> 710
<212> PRT
<213> Artificial Sequence
<223> Description of Artificial Sequence:tetra-fusion
      protein DPV-MTI-MSL-MTCC2 (designated Mtb71f)

<400> 16
His Met His His His His His His Asp Pro Val Asp Ala Val Ile Asn
  1               5                  10                  15

Thr Thr Cys Asn Tyr Gly Gln Val Val Ala Ala Leu Asn Ala Thr Asp
             20                  25                  30

Pro Gly Ala Ala Ala Gln Phe Asn Ala Ser Pro Val Ala Gln Ser Tyr
         35                  40                  45
Leu Arg Asn Phe Leu Ala Ala Pro Pro Pro Gln Arg Ala Ala Met Ala
     50                  55                  60

Ala Gln Leu Gln Ala Val Pro Gly Ala Ala Gln Tyr Ile Gly Leu Val
 65                  70                  75                  80

Glu Ser Val Ala Gly Ser Cys Asn Asn Tyr Glu Leu Met Thr Ile Asn
                 85                  90                  95

Tyr Gln Phe Gly Asp Val Asp Ala His Gly Ala Met Ile Arg Ala Gln
            100                 105                 110

Ala Ala Ser Leu Glu Ala Glu His Gln Ala Ile Val Arg Asp Val Leu
        115                 120                 125

Ala Ala Gly Asp Phe Trp Gly Gly Ala Gly Ser Val Ala Cys Gln Glu
    130                 135                 140

Phe Ile Thr Gln Leu Gly Arg Asn Phe Gln Val Ile Tyr Glu Gln Ala
145                 150                 155                 160

Asn Ala His Gly Gln Lys Val Gln Ala Ala Gly Asn Asn Met Ala Gln

                165                 170                 175

Thr Asp Ser Ala Val Gly Ser Ser Trp Ala Thr Ser Met Ser Leu Leu
            180                 185                 190

Asp Ala His Ile Pro Gln Leu Val Ala Ser Gln Ser Ala Phe Ala Ala
        195                 200                 205

Lys Ala Gly Leu Met Arg His Thr Ile Gly Gln Ala Glu Gln Ala Ala
    210                 215                 220

Met Ser Ala Gln Ala Phe His Gln Gly Glu Ser Ser Ala Ala Phe Gln
225                 230                 235                 240

Ala Ala His Ala Arg Phe Val Ala Ala Ala Ala Lys Val Asn Thr Leu
                245                 250                 255

Leu Asp Val Ala Gln Ala Asn Leu Gly Glu Ala Ala Gly Thr Tyr Val
            260                 265                 270

Ala Ala Asp Ala Ala Ala Ala Ser Thr Tyr Thr Gly Phe Asp Ile Met
        275                 280                 285

Asp Phe Gly Leu Leu Pro Pro Glu Val Asn Ser Ser Arg Met Tyr Ser
    290                 295                 300

Gly Pro Gly Pro Glu Ser Met Leu Ala Ala Ala Ala Ala Trp Asp Gly
305                 310                 315                 320

Val Ala Ala Glu Leu Thr Ser Ala Ala Val Ser Tyr Gly Ser Val Val
                325                 330                 335

Ser Thr Leu Ile Val Glu Pro Trp Met Gly Pro Ala Ala Ala Ala Met
            340                 345                 350

Ala Ala Ala Ala Thr Pro Tyr Val Gly Trp Leu Ala Ala Thr Ala Ala
        355                 360                 365

Leu Ala Lys Glu Thr Ala Thr Gln Ala Arg Ala Ala Ala Glu Ala Phe
    370                 375                 380

Gly Thr Ala Phe Ala Met Thr Val Pro Pro Ser Leu Val Ala Ala Asn
385                 390                 395                 400

Arg Ser Arg Leu Met Ser Leu Val Ala Ala Asn Ile Leu Gly Gln Asn
                405                 410                 415

Ser Ala Ala Ile Ala Ala Thr Gln Ala Glu Tyr Ala Glu Met Trp Ala
            420                 425                 430

Gln Asp Ala Ala Val Met Tyr Ser Tyr Glu Gly Ala Ser Ala Ala Ala
        435                 440                 445

Ser Ala Leu Pro Pro Phe Thr Pro Pro Val Gln Gly Thr Gly Pro Ala
    450                 455                 460

Gly Pro Ala Ala Ala Ala Ala Ala Thr Gln Ala Ala Gly Ala Gly Ala
465                 470                 475                 480

Val Ala Asp Ala Gln Ala Thr Leu Ala Gln Leu Pro Pro Gly Ile Leu

485                 490                 495

Ser Asp Ile Leu Ser Ala Leu Ala Ala Asn Ala Asp Pro Leu Thr Ser
            500                 505                 510

Gly Leu Leu Gly Ile Ala Ser Thr Leu Asn Pro Gln Val Gly Ser Ala
        515                 520                 525

Gln Pro Ile Val Ile Pro Thr Pro Ile Gly Glu Leu Asp Val Ile Ala
    530                 535                 540

Leu Tyr Ile Ala Ser Ile Ala Thr Gly Ser Ile Ala Leu Ala Ile Thr
545                 550                 555                 560

Asn Thr Ala Arg Pro Trp His Ile Gly Leu Tyr Gly Asn Ala Gly Gly
                565                 570                 575

Leu Gly Pro Thr Gln Gly His Pro Leu Ser Ser Ala Thr Asp Glu Pro
            580                 585                 590

Glu Pro His Trp Gly Pro Phe Gly Gly Ala Ala Pro Val Ser Ala Gly
        595                 600                 605

Val Gly His Ala Ala Leu Val Gly Ala Leu Ser Val Pro His Ser Trp
    610                 615                 620

Thr Thr Ala Ala Pro Glu Ile Gln Leu Ala Val Gln Ala Thr Pro Thr
625                 630                 635                 640

Phe Ser Ser Ser Ala Gly Ala Asp Pro Thr Ala Leu Asn Gly Met Pro
                645                 650                 655

Ala Gly Leu Leu Ser Gly Met Ala Leu Ala Ser Leu Ala Ala Arg Gly
            660                 665                 670

Thr Thr Gly Gly Gly Gly Thr Arg Ser Gly Thr Ser Thr Asp Gly Gln
        675                 680                 685

Glu Asp Gly Arg Lys Pro Pro Val Val Val Ile Arg Glu Gln Pro Pro
    690                 695                 700

Pro Gly Asn Pro Pro Arg
705                 710

<210> 17
<211> 921
<212> DNA
<213> Artificial Sequence

<220>
<223> Description of Artificial Sequence:tri-fusion
      protein DPV-MTI-MSL (designated Mtb31f)

<220>
<221> CDS
<222> (1)..(900)

<400> 17

cat atg cat cac cat cac cat cac gat ccc gtg gac gcg gtc att aac 48

acc acc tgc aat tac ggg cag gta gta gct gcg ctc aac gcg acg gat 96

ccg ggg gct gcc gca cag ttc aac gcc tca ccg gtg gcg cag tcc tat 144

ttg cgc aat ttc ctc gcc gca ccg cca cct cag cgc gct gcc atg gcc 192

gcg caa ttg caa gct gtg ccg ggg gcg gca cag tac atc ggc ctt gtc 240

gag tcg gtt gcc ggc tcc tgc aac aac tat gag ctc atg acg att aat 288

tac cag ttc ggg gac gtc gac gct cat ggc gcc atg atc cgc gct cag 336

gcg gcg tcg ctt gag gcg gag cat cag gcc atc gtt cgt gat gtg ttg 384

gcc gcg ggt gac ttt tgg ggc ggc gcc ggt tcg gtg gct tgc cag gag 432

ttc att acc cag ttg ggc cgt aac ttc cag gtg atc tac gag cag gcc 480

aac gcc cac ggg cag aag gtg cag gct gcc ggc aac aac atg gcg caa 528

acc gac agc gcc gtc ggc tcc agc tgg gcc act agt atg agc ctt ttg 576

gat gct cat atc cca cag ttg gtg gcc tcc cag tcg gcg ttt gcc gcc 624

aag gcg ggg ctg atg cgg cac acg atc ggt cag gcc gag cag gcg gcg 672

atg tcg gct cag gcg ttt cac cag ggg gag tcg tcg gcg gcg ttt cag 720

gcc gcc cat gcc cgg ttt gtg gcg gcg gcc gcc aaa gtc aac acc ttg 768

ttg gat gtc gcg cag gcg aat ctg ggt gag gcc gcc ggt acc tat gtg 816

gcc gcc gat gct gcg gcc gcg tcg acc tat acc ggg ttc gat atc cat 864

cac act ggc ggc cgc tcg agc aga tcc ggc tgc taacaaagcc cgaaaggaag 917

ctga 921

<210> 18
<211> 299
<212> PRT
<213> Artificial Sequence
<223> Description of Artificial Sequence:tri-fusion
      protein DPV-MTI-MSL (designated Mtb31f)

<400> 18
His Met His His His His His His Asp Pro Val Asp Ala Val Ile Asn
 1               5                  10                  15

Thr Thr Cys Asn Tyr Gly Gln Val Val Ala Ala Leu Asn Ala Thr Asp
            20                  25                  30

Pro Gly Ala Ala Ala Gln Phe Asn Ala Ser Pro Val Ala Gln Ser Tyr
        35                  40                  45

Leu Arg Asn Phe Leu Ala Ala Pro Pro Pro Gln Arg Ala Ala Met Ala

50                  55                  60

Ala Gln Leu Gln Ala Val Pro Gly Ala Ala Gln Tyr Ile Gly Leu Val
65                  70                  75                  80

Glu Ser Val Ala Gly Ser Cys Asn Asn Tyr Glu Leu Met Thr Ile Asn
                85                  90                  95

Tyr Gln Phe Gly Asp Val Asp Ala His Gly Ala Met Ile Arg Ala Gln
            100                 105                 110

Ala Ala Ser Leu Glu Ala Glu His Gln Ala Ile Val Arg Asp Val Leu
        115                 120                 125

Ala Ala Gly Asp Phe Trp Gly Gly Ala Gly Ser Val Ala Cys Gln Glu
    130                 135                 140

Phe Ile Thr Gln Leu Gly Arg Asn Phe Gln Val Ile Tyr Glu Gln Ala
145                 150                 155                 160

Asn Ala His Gly Gln Lys Val Gln Ala Ala Gly Asn Asn Met Ala Gln
                165                 170                 175

Thr Asp Ser Ala Val Gly Ser Ser Trp Ala Thr Ser Met Ser Leu Leu
            180                 185                 190

Asp Ala His Ile Pro Gln Leu Val Ala Ser Gln Ser Ala Phe Ala Ala
        195                 200                 205

Lys Ala Gly Leu Met Arg His Thr Ile Gly Gln Ala Glu Gln Ala Ala
    210                 215                 220

Met Ser Ala Gln Ala Phe His Gln Gly Glu Ser Ser Ala Ala Phe Gln
225                 230                 235                 240

Ala Ala His Ala Arg Phe Val Ala Ala Ala Ala Lys Val Asn Thr Leu
                245                 250                 255

Leu Asp Val Ala Gln Ala Asn Leu Gly Glu Ala Ala Gly Thr Tyr Val
            260                 265                 270

Ala Ala Asp Ala Ala Ala Ala Ser Thr Tyr Thr Gly Phe Asp Ile His
        275                 280                 285

His Thr Gly Gly Arg Ser Ser Arg Ser Gly Cys
    290                 295

<210> 19
<211> 1801
<212> DNA
<213> Artificial Sequence

<220>
<223> Description of Artificial Sequence:tri-fusion
      protein TbH9-DPV-MTI (designated Mtb61f)

<220>
<221> CDS

<222> (1)..(1800)

<400> 19
cat atg cat cac cat cac cat cac atg gtg gat ttc ggg gcg tta cca 48

ccg gag atc aac tcc gcg agg atg tac gcc ggc ccg ggt tcg gcc tcg 96

ctg gtg gcc gcg gct cag atg tgg gac agc gtg gcg agt gac ctg ttt 144

tcg gcc gcg tcg gcg ttt cag tcg gtg gtc tgg ggt ctg acg gtg ggg 192

tcg tgg ata ggt tcg tcg gcg ggt ctg atg gtg gcg gcg gcc tcg ccg 240

tat gtg gcg tgg atg agc gtc acc gcg ggg cag gcc gag ctg acc gcc 288

gcc cag gtc cgg gtt gct gcg gcg gcc tac gag acg gcg tat ggg ctg 336

acg gtg ccc ccg ccg gtg atc gcc gag aac cgt gct gaa ctg atg att 384

ctg ata gcg acc aac ctc ttg ggg caa aac acc ccg gcg atc gcg gtc 432

aac gag gcc gaa tac ggc gag atg tgg gcc caa gac gcc gcc gcg atg 480

ttt ggc tac gcc gcg gcg acg gcg acg gcg acg gcg acg ttg ctg ccg 528

ttc gag gag gcg ccg gag atg acc agc gcg ggt ggg ctc ctc gag cag 576

gcc gcc gcg gtc gag gag gcc tcc gac acc gcc gcg gcg aac cag ttg 624

atg aac aat gtg ccc cag gcg ctg caa cag ctg gcc cag ccc acg cag 672

ggc acc acg cct tct tcc aag ctg ggt ggc ctg tgg aag acg gtc tcg 720

ccg cat cgg tcg ccg atc agc aac atg gtg tcg atg gcc aac aac cac 768

atg tcg atg acc aac tcg ggt gtg tcg atg acc aac acc ttg agc tcg 816

atg ttg aag ggc ttt gct ccg gcg gcg gcc gcc cag gcc gtg caa acc 864

gcg gcg caa aac ggg gtc cgg gcg atg agc tcg ctg ggc agc tcg ctg 912

ggt tct tcg ggt ctg ggc ggt ggg gtg gcc gcc aac ttg ggt cgg gcg 960

gcc tcg gtc ggt tcg ttg tcg gtg ccg cag gcc tgg gcc gcg gcc aac 1008

cag gca gtc acc ccg gcg gcg cgg gcg ctg ccg ctg acc agc ctg acc 1056

agc gcc gcg gaa aga ggg ccc ggg cag atg ctg ggc ggg ctg ccg gtg 1104

ggg cag atg ggc gcc agg gcc ggt ggt ggg ctc agt ggt gtg ctg cgt 1152

gtt ccg ccg cga ccc tat gtg atg ccg cat tct ccg gca gcc ggc aag 1200

ctt gat ccc gtg gac gcg gtc att aac acc acc tgc aat tac ggg cag 1248

gta gta gct gcg ctc aac gcg acg gat ccg ggg gct gcc gca cag ttc 1296

aac gcc tca ccg gtg gcg cag tcc tat ttg cgc aat ttc ctc gcc gca 1344

ccg cca cct cag cgc gct gcc atg gcc gcg caa ttg caa gct gtg ccg 1392

ggg gcg gca cag tac atc ggc ctt gtc gag tcg gtt gcc ggc tcc tgc 1440

aac aac tat gag ctc atg acg att aat tac cag ttc ggg gac gtc gac 1488

gct cat ggc gcc atg atc cgc gct cag gcg gcg tcg ctt gag gcg gag 1536

cat cag gcc atc gtt cgt gat gtg ttg gcc gcg ggt gac ttt tgg ggc 1584

ggc gcc ggt tcg gtg gct tgc cag gag ttc att acc cag ttg ggc cgt 1632

aac ttc cag gtg atc tac gag cag gcc aac gcc cac ggg cag aag gtg 1680

cag gct gcc ggc aac aac atg gcg caa acc gac agc gcc gtc ggc tcc 1728

agc tgg gcc act agt aac ggc cgc cag tgt gct gga att ctg cag ata 1776

tcc atc aca ctg gcg gcc gct cga g 1801

<210> 20
<211> 600
<212> PRT
<213> Artificial Sequence
<223> Description of Artificial Sequence:tri-fusion
      protein TbH9-DPV-MTI (designated Mtb61f)

<400> 20
His Met His His His His His His Met Val Asp Phe Gly Ala Leu Pro
1               5                   10                  15

Pro Glu Ile Asn Ser Ala Arg Met Tyr Ala Gly Pro Gly Ser Ala Ser
            20                  25                  30

Leu Val Ala Ala Ala Gln Met Trp Asp Ser Val Ala Ser Asp Leu Phe
        35                  40                  45

Ser Ala Ala Ser Ala Phe Gln Ser Val Val Trp Gly Leu Thr Val Gly
    50                  55                  60

Ser Trp Ile Gly Ser Ser Ala Gly Leu Met Val Ala Ala Ala Ser Pro
65                  70                  75                  80

Tyr Val Ala Trp Met Ser Val Thr Ala Gly Gln Ala Glu Leu Thr Ala
                85                  90                  95

Ala Gln Val Arg Val Ala Ala Ala Ala Tyr Glu Thr Ala Tyr Gly Leu
            100                 105                 110

Thr Val Pro Pro Pro Val Ile Ala Glu Asn Arg Ala Glu Leu Met Ile
        115                 120                 125

Leu Ile Ala Thr Asn Leu Leu Gly Gln Asn Thr Pro Ala Ile Ala Val
    130                 135                 140

Asn Glu Ala Glu Tyr Gly Glu Met Trp Ala Gln Asp Ala Ala Ala Met
145                 150                 155                 160

Phe Gly Tyr Ala Ala Ala Thr Ala Thr Ala Thr Ala Thr Leu Leu Pro

                165                 170                 175

Phe Glu Glu Ala Pro Glu Met Thr Ser Ala Gly Gly Leu Leu Glu Gln
            180                 185                 190

Ala Ala Ala Val Glu Glu Ala Ser Asp Thr Ala Ala Ala Asn Gln Leu
        195                 200                 205

Met Asn Asn Val Pro Gln Ala Leu Gln Gln Leu Ala Gln Pro Thr Gln
    210                 215                 220

Gly Thr Thr Pro Ser Ser Lys Leu Gly Gly Leu Trp Lys Thr Val Ser
225                 230                 235                 240

Pro His Arg Ser Pro Ile Ser Asn Met Val Ser Met Ala Asn Asn His
                245                 250                 255

Met Ser Met Thr Asn Ser Gly Val Ser Met Thr Asn Thr Leu Ser Ser
            260                 265                 270

Met Leu Lys Gly Phe Ala Pro Ala Ala Ala Ala Gln Ala Val Gln Thr
        275                 280                 285

Ala Ala Gln Asn Gly Val Arg Ala Met Ser Ser Leu Gly Ser Ser Leu
    290                 295                 300

Gly Ser Ser Gly Leu Gly Gly Gly Val Ala Ala Asn Leu Gly Arg Ala
305                 310                 315                 320

Ala Ser Val Gly Ser Leu Ser Val Pro Gln Ala Trp Ala Ala Ala Asn
                325                 330                 335

Gln Ala Val Thr Pro Ala Ala Arg Ala Leu Pro Leu Thr Ser Leu Thr
            340                 345                 350

Ser Ala Ala Glu Arg Gly Pro Gly Gln Met Leu Gly Gly Leu Pro Val
        355                 360                 365

Gly Gln Met Gly Ala Arg Ala Gly Gly Gly Leu Ser Gly Val Leu Arg
    370                 375                 380

Val Pro Pro Arg Pro Tyr Val Met Pro His Ser Pro Ala Ala Gly Lys
385                 390                 395                 400

Leu Asp Pro Val Asp Ala Val Ile Asn Thr Thr Cys Asn Tyr Gly Gln
                405                 410                 415

Val Val Ala Ala Leu Asn Ala Thr Asp Pro Gly Ala Ala Ala Gln Phe
            420                 425                 430

Asn Ala Ser Pro Val Ala Gln Ser Tyr Leu Arg Asn Phe Leu Ala Ala
        435                 440                 445

Pro Pro Pro Gln Arg Ala Ala Met Ala Ala Gln Leu Gln Ala Val Pro
    450                 455                 460

Gly Ala Ala Gln Tyr Ile Gly Leu Val Glu Ser Val Ala Gly Ser Cys
465                 470                 475                 480

Asn Asn Tyr Glu Leu Met Thr Ile Asn Tyr Gln Phe Gly Asp Val Asp

485                 490                 495

Ala His Gly Ala Met Ile Arg Ala Gln Ala Ala Ser Leu Glu Ala Glu
            500                 505                 510

His Gln Ala Ile Val Arg Asp Val Leu Ala Ala Gly Asp Phe Trp Gly
        515                 520                 525

Gly Ala Gly Ser Val Ala Cys Gln Glu Phe Ile Thr Gln Leu Gly Arg
    530                 535                 540

Asn Phe Gln Val Ile Tyr Glu Gln Ala Asn Ala His Gly Gln Lys Val
545                 550                 555                 560

Gln Ala Ala Gly Asn Asn Met Ala Gln Thr Asp Ser Ala Val Gly Ser
                565                 570                 575

Ser Trp Ala Thr Ser Asn Gly Arg Gln Cys Ala Gly Ile Leu Gln Ile
            580                 585                 590

Ser Ile Thr Leu Ala Ala Ala Arg
        595                 600

<210> 21
<211> 1104
<212> DNA
<213> Artificial Sequence

<220>
<223> Description of Artificial Sequence:tri-fusion
      Erd14-DPV-MTI (designated Mtb36f)

<220>
<221> CDS
<222> (1)..(1104)

<400> 21
cat atg cat cac cat cac cat cac atg gcc acc acc ctt ccc gtt cag    48

cgc cac ccg cgg tcc ctc ttc ccc gag ttt tct gag ctg ttc gcg gcc    96

ttc ccg tca ttc gcc gga ctc cgg ccc acc ttc gac acc cgg ttg atg    144

cgg ctg gaa gac gag atg aaa gag ggg cgc tac gag gta cgc gcg gag    192

ctt ccc ggg gtc gac ccc gac aag gac gtc gac att atg gtc cgc gat    240

ggt cag ctg acc atc aag gcc gag cgc acc gag cag aag gac ttc gac    288

ggt cgc tcg gaa ttc gcg tac ggt tcc ttc gtt cgc acg gtg tcg ctg    336

ccg gta ggt gct gac gag gac gac att aag gcc acc tac gac aag ggc    384

att ctt act gtg tcg gtg gcg gtt tcg gaa ggg aag cca acc gaa aag    432

cac att cag atc cgg tcc acc aac aag ctt gat ccc gtg gac gcg gtc    480

att aac acc acc tgc aat tac ggg cag gta gta gct gcg ctc aac gcg    528

acg gat ccg ggg gct gcc gca cag ttc aac gcc tca ccg gtg gcg cag 576

tcc tat ttg cgc aat ttc ctc gcc gca ccg cca cct cag cgc gct gcc 624

atg gcc gcg caa ttg caa gct gtg ccg ggg gcg gca cag tac atc ggc 672

ctt gtc gag tcg gtt gcc ggc tcc tgc aac aac tat gag ctc atg acg 720

att aat tac cag ttc ggg gac gtc gac gct cat ggc gcc atg atc cgc 768

gct cag gcg gcg tcg ctt gag gcg gag cat cag gcc atc gtt cgt gat 816

gtg ttg gcc gcg ggt gac ttt tgg ggc ggc gcc ggt tcg gtg gct tgc 864

cag gag ttc att acc cag ttg ggc cgt aac ttc cag gtg atc tac gag 912

cag gcc aac gcc cac ggg cag aag gtg cag gct gcc ggc aac aac atg 960

gcg caa acc gac agc gcc gtc ggc tcc agc tgg gcc act agt aac ggc 1008

cgc cag tgt gct gga att ctg cag ata tcc atc aca ctg gcg gcc gct 1056

cga gca gat ccg gct gct aac aaa gcc cga aag gaa gct gag ttg gct 1104

<210> 22
<211> 368
<212> PRT
<213> Artificial Sequence
<223> Description of Artificial Sequence:tri-fusion
      Erd14-DPV-MTI (designated Mtb36f)

<400> 22
His Met His His His His His His Met Ala Thr Thr Leu Pro Val Gln
  1               5                  10                  15

Arg His Pro Arg Ser Leu Phe Pro Glu Phe Ser Glu Leu Phe Ala Ala
             20                  25                  30

Phe Pro Ser Phe Ala Gly Leu Arg Pro Thr Phe Asp Thr Arg Leu Met
         35                  40                  45

Arg Leu Glu Asp Glu Met Lys Glu Gly Arg Tyr Glu Val Arg Ala Glu
     50                  55                  60

Leu Pro Gly Val Asp Pro Asp Lys Asp Val Asp Ile Met Val Arg Asp
 65                  70                  75                  80

Gly Gln Leu Thr Ile Lys Ala Glu Arg Thr Glu Gln Lys Asp Phe Asp
                 85                  90                  95

Gly Arg Ser Glu Phe Ala Tyr Gly Ser Phe Val Arg Thr Val Ser Leu
            100                 105                 110

Pro Val Gly Ala Asp Glu Asp Asp Ile Lys Ala Thr Tyr Asp Lys Gly
        115                 120                 125

Ile Leu Thr Val Ser Val Ala Val Ser Glu Gly Lys Pro Thr Glu Lys

130                 135                 140

His Ile Gln Ile Arg Ser Thr Asn Lys Leu Asp Pro Val Asp Ala Val
145                 150                 155                 160

Ile Asn Thr Thr Cys Asn Tyr Gly Gln Val Val Ala Ala Leu Asn Ala
                165                 170                 175

Thr Asp Pro Gly Ala Ala Ala Gln Phe Asn Ala Ser Pro Val Ala Gln
            180                 185                 190

Ser Tyr Leu Arg Asn Phe Leu Ala Ala Pro Pro Pro Gln Arg Ala Ala
        195                 200                 205

Met Ala Ala Gln Leu Gln Ala Val Pro Gly Ala Ala Gln Tyr Ile Gly
    210                 215                 220

Leu Val Glu Ser Val Ala Gly Ser Cys Asn Asn Tyr Glu Leu Met Thr
225                 230                 235                 240

Ile Asn Tyr Gln Phe Gly Asp Val Asp Ala His Gly Ala Met Ile Arg
                245                 250                 255

Ala Gln Ala Ala Ser Leu Glu Ala Glu His Gln Ala Ile Val Arg Asp
            260                 265                 270

Val Leu Ala Ala Gly Asp Phe Trp Gly Gly Ala Gly Ser Val Ala Cys
        275                 280                 285

Gln Glu Phe Ile Thr Gln Leu Gly Arg Asn Phe Gln Val Ile Tyr Glu
    290                 295                 300

Gln Ala Asn Ala His Gly Gln Lys Val Gln Ala Ala Gly Asn Asn Met
305                 310                 315                 320

Ala Gln Thr Asp Ser Ala Val Gly Ser Ser Trp Ala Thr Ser Asn Gly
                325                 330                 335

Arg Gln Cys Ala Gly Ile Leu Gln Ile Ser Ile Thr Leu Ala Ala Ala
            340                 345                 350

Arg Ala Asp Pro Ala Ala Asn Lys Ala Arg Lys Glu Ala Glu Leu Ala
        355                 360                 365

<210> 23
<211> 1797
<212> DNA
<213> Artificial Sequence

<220>
<223> Description of Artificial Sequence:bi-fusion
      protein TbH9-Ra35 (designated Mtb59f)

<220>
<221> CDS
<222> (1)..(1791)

<400> 23

cat atg cat cac cat cac cat cac atg gtg gat ttc ggg gcg tta cca 48

ccg gag atc aac tcc gcg agg atg tac gcc ggc ccg ggt tcg gcc tcg 96

ctg gtg gcc gcg gct cag atg tgg gac agc gtg gcg agt gac ctg ttt 144

tcg gcc gcg tcg gcg ttt cag tcg gtg gtc tgg ggt ctg acg gtg ggg 192

tcg tgg ata ggt tcg tcg gcg ggt ctg atg gtg gcg gcg gcc tcg ccg 240

tat gtg gcg tgg atg agc gtc acc gcg ggg cag gcc gag ctg acc gcc 288

gcc cag gtc cgg gtt gct gcg gcg gcc tac gag acg gcg tat ggg ctg 336

acg gtg ccc ccg ccg gtg atc gcc gag aac cgt gct gaa ctg atg att 384

ctg ata gcg acc aac ctc ttg ggg caa aac acc ccg gcg atc gcg gtc 432

aac gag gcc gaa tac ggc gag atg tgg gcc caa gac gcc gcc gcg atg 480

ttt ggc tac gcc gcg gcg acg gcg acg gcg acg gcg acg ttg ctg ccg 528

ttc gag gag gcg ccg gag atg acc agc gcg ggt ggg ctc ctc gag cag 576

gcc gcc gcg gtc gag gag gcc tcc gac acc gcc gcg gcg aac cag ttg 624

atg aac aat gtg ccc cag gcg ctg caa cag ctg gcc cag ccc acg cag 672

ggc acc acg cct tct tcc aag ctg ggt ggc ctg tgg aag acg gtc tcg 720

ccg cat cgg tcg ccg atc agc aac atg gtg tcg atg gcc aac aac cac 768

atg tcg atg acc aac tcg ggt gtg tcg atg acc aac acc ttg agc tcg 816

atg ttg aag ggc ttt gct ccg gcg gcg gcc gcc cag gcc gtg caa acc 864

gcg gcg caa aac ggg gtc cgg gcg atg agc tcg ctg ggc agc tcg ctg 912

ggt tct tcg ggt ctg ggc ggt ggg gtg gcc gcc aac ttg ggt cgg gcg 960

gcc tcg gtc ggt tcg ttg tcg gtg ccg cag gcc tgg gcc gcg gcc aac 1008

cag gca gtc acc ccg gcg gcg cgg gcg ctg ccg ctg acc agc ctg acc 1056

agc gcc gcg gaa aga ggg ccc ggg cag atg ctg ggc ggg ctg ccg gtg 1104

ggg cag atg ggc gcc agg gcc ggt ggt ggg ctc agt ggt gtg ctg cgt 1152

gtt ccg ccg cga ccc tat gtg atg ccg cat tct ccg gca gcc ggc gat 1200

atc gcc ccg ccg gcc ttg tcg cag gac cgg ttc gcc gac ttc ccc gcg 1248

ctg ccc ctc gac ccg tcc gcg atg gtc gcc caa gtg ggg cca cag gtg 1296

gtc aac atc aac acc aaa ctg ggc tac aac aac gcc gtg ggc gcc ggg 1344

acc ggc atc gtc atc gat ccc aac ggt gtc gtg ctg acc aac aac cac 1392

gtg atc gcg ggc gcc acc gac atc aat gcg ttc agc gtc ggc tcc ggc 1440

caa acc tac ggc gtc gat gtg gtc ggg tat gac cgc acc cag gat gtc 1488

gcg gtg ctg cag ctg cgc ggt gcc ggt ggc ctg ccg tcg gcg gcg atc 1536

ggt ggc ggc gtc gcg gtt ggt gag ccc gtc gtc gcg atg ggc aac agc 1584

ggt ggg cag ggc gga acg ccc cgt gcg gtg cct ggc agg gtg gtc gcg 1632

ctc ggc caa acc gtg cag gcg tcg gat tcg ctg acc ggt gcc gaa gag 1680

aca ttg aac ggg ttg atc cag ttc gat gcc gcg atc cag ccc ggt gat 1728

tcg ggc ggg ccc gtc gtc aac ggc cta gga cag gtg gtc ggt atg aac 1776

acg gcc gcg tcc taggatatc 1797

<210> 24
<211> 596
<212> PRT
<213> Artificial Sequence
<223> Description of Artificial Sequence:bi-fusion
      protein TbH9-Ra35 (designated Mtb59f)

<400> 24
His Met His His His His His His Met Val Asp Phe Gly Ala Leu Pro
 1               5                  10                  15

Pro Glu Ile Asn Ser Ala Arg Met Tyr Ala Gly Pro Gly Ser Ala Ser
            20                  25                  30

Leu Val Ala Ala Ala Gln Met Trp Asp Ser Val Ala Ser Asp Leu Phe
        35                  40                  45

Ser Ala Ala Ser Ala Phe Gln Ser Val Val Trp Gly Leu Thr Val Gly
    50                  55                  60

Ser Trp Ile Gly Ser Ser Ala Gly Leu Met Val Ala Ala Ala Ser Pro
65                  70                  75                  80

Tyr Val Ala Trp Met Ser Val Thr Ala Gly Gln Ala Glu Leu Thr Ala
                85                  90                  95

Ala Gln Val Arg Val Ala Ala Ala Ala Tyr Glu Thr Ala Tyr Gly Leu
            100                 105                 110

Thr Val Pro Pro Pro Val Ile Ala Glu Asn Arg Ala Glu Leu Met Ile
        115                 120                 125

Leu Ile Ala Thr Asn Leu Leu Gly Gln Asn Thr Pro Ala Ile Ala Val
    130                 135                 140

Asn Glu Ala Glu Tyr Gly Glu Met Trp Ala Gln Asp Ala Ala Ala Met
145                 150                 155                 160

Phe Gly Tyr Ala Ala Ala Thr Ala Thr Ala Thr Ala Thr Leu Leu Pro
                165                 170                 175

Phe Glu Glu Ala Pro Glu Met Thr Ser Ala Gly Gly Leu Leu Glu Gln

180                 185                 190

Ala Ala Ala Val Glu Glu Ala Ser Asp Thr Ala Ala Ala Asn Gln Leu
    195                 200                 205

Met Asn Asn Val Pro Gln Ala Leu Gln Gln Leu Ala Gln Pro Thr Gln
    210                 215                 220

Gly Thr Thr Pro Ser Ser Lys Leu Gly Gly Leu Trp Lys Thr Val Ser
225                 230                 235                 240

Pro His Arg Ser Pro Ile Ser Asn Met Val Ser Met Ala Asn Asn His
                245                 250                 255

Met Ser Met Thr Asn Ser Gly Val Ser Met Thr Asn Thr Leu Ser Ser
            260                 265                 270

Met Leu Lys Gly Phe Ala Pro Ala Ala Ala Ala Gln Ala Val Gln Thr
        275                 280                 285

Ala Ala Gln Asn Gly Val Arg Ala Met Ser Ser Leu Gly Ser Ser Leu
    290                 295                 300

Gly Ser Ser Gly Leu Gly Gly Gly Val Ala Ala Asn Leu Gly Arg Ala
305                 310                 315                 320

Ala Ser Val Gly Ser Leu Ser Val Pro Gln Ala Trp Ala Ala Ala Asn
                325                 330                 335

Gln Ala Val Thr Pro Ala Ala Arg Ala Leu Pro Leu Thr Ser Leu Thr
            340                 345                 350

Ser Ala Ala Glu Arg Gly Pro Gly Gln Met Leu Gly Gly Leu Pro Val
        355                 360                 365

Gly Gln Met Gly Ala Arg Ala Gly Gly Gly Leu Ser Gly Val Leu Arg
    370                 375                 380

Val Pro Pro Arg Pro Tyr Val Met Pro His Ser Pro Ala Ala Gly Asp
385                 390                 395                 400

Ile Ala Pro Pro Ala Leu Ser Gln Asp Arg Phe Ala Asp Phe Pro Ala
                405                 410                 415

Leu Pro Leu Asp Pro Ser Ala Met Val Ala Gln Val Gly Pro Gln Val
            420                 425                 430

Val Asn Ile Asn Thr Lys Leu Gly Tyr Asn Asn Ala Val Gly Ala Gly
        435                 440                 445

Thr Gly Ile Val Ile Asp Pro Asn Gly Val Val Leu Thr Asn Asn His
    450                 455                 460

Val Ile Ala Gly Ala Thr Asp Ile Asn Ala Phe Ser Val Gly Ser Gly
465                 470                 475                 480

Gln Thr Tyr Gly Val Asp Val Val Gly Tyr Asp Arg Thr Gln Asp Val
                485                 490                 495

Ala Val Leu Gln Leu Arg Gly Ala Gly Gly Leu Pro Ser Ala Ala Ile

500                 505                 510

Gly Gly Gly Val Ala Val Gly Glu Pro Val Val Ala Met Gly Asn Ser
        515                 520                 525

Gly Gly Gln Gly Gly Thr Pro Arg Ala Val Pro Gly Arg Val Val Ala
    530                 535                 540

Leu Gly Gln Thr Val Gln Ala Ser Asp Ser Leu Thr Gly Ala Glu Glu
545                 550                 555                 560

Thr Leu Asn Gly Leu Ile Gln Phe Asp Ala Ala Ile Gln Pro Gly Asp
                565                 570                 575

Ser Gly Gly Pro Val Val Asn Gly Leu Gly Gln Val Val Gly Met Asn
            580                 585                 590

Thr Ala Ala Ser
        595

<210> 25
<211> 702
<212> DNA
<213> Artificial Sequence

<220>
<223> Description of Artificial Sequence:bi-fusion
      protein Ra12-DPPD (designated Mtb24), reading
      frame 1

<220>
<221> CDS
<222> (1)..(696)
<223> bi-fusion protein Ra12-DPPD (designated Mtb24),
      reading frame 1

<220>
<221> CDS
<222> (2)..(700)
<223> reading frame 2

<220>
<221> CDS
<222> (3)..(701)
<223> reading frame 3

<400> 25
cat atg cat cac cat cac cat cac acg gcc gcg tcc gat aac ttc cag    48

ctg tcc cag ggt ggg cag gga ttc gcc att ccg atc ggg cag gcg atg    96

gcg atc gcg ggc cag atc cga tcg ggt ggg ggg tca ccc acc gtt cat    144

atc ggg cct acc gcc ttc ctc ggc ttg ggt gtt gtc gac aac aac ggc    192

aac ggc gca cga gtc caa cgc gtg gtc ggg agc gct ccg gcg gca agt    240

ctc ggc atc tcc acc ggc gac gtg atc acc gcg gtc gac ggc gct ccg    288

atc aac tcg gcc acc gcg atg gcg gac gcg ctt aac ggg cat cat ccc 336

ggt gac gtc atc tcg gtg acc tgg caa acc aag tcg ggc ggc acg cgt 384

aca ggg aac gtg aca ttg gcc gag gga ccc ccg gcc gaa ttc gac gac 432

gac gac aag gat cca cct gac ccg cat cag ccg gac atg acg aaa ggc 480

tat tgc ccg ggt ggc cga tgg ggt ttt ggc gac ttg gcc gtg tgc gac 528

ggc gag aag tac ccc gac ggc tcg ttt tgg cac cag tgg atg caa acg 576

tgg ttt acc ggc cca cag ttt tac ttc gat tgt gtc agc ggc ggt gag 624

ccc ctc ccc ggc ccg ccg cca ccg ggt ggt tgc ggt ggg gca att ccg 672

tcc gag cag ccc aac gct ccc tgagaattc 702

<210> 26
<211> 231
<212> PRT
<213> Artificial Sequence
<223> Description of Artificial Sequence:bi-fusion
      protein Ra12-DPPD (designated Mtb24), reading
      frame 1

<400> 26
His Met His His His His His His Thr Ala Ala Ser Asp Asn Phe Gln
 1               5                   10                  15

Leu Ser Gln Gly Gly Gln Gly Phe Ala Ile Pro Ile Gly Gln Ala Met
            20                  25                  30

Ala Ile Ala Gly Gln Ile Arg Ser Gly Gly Gly Ser Pro Thr Val His
        35                  40                  45

Ile Gly Pro Thr Ala Phe Leu Gly Leu Gly Val Val Asp Asn Asn Gly
    50                  55                  60

Asn Gly Ala Arg Val Gln Arg Val Val Gly Ser Ala Pro Ala Ala Ser
65                  70                  75                  80

Leu Gly Ile Ser Thr Gly Asp Val Ile Thr Ala Val Asp Gly Ala Pro
                85                  90                  95

Ile Asn Ser Ala Thr Ala Met Ala Asp Ala Leu Asn Gly His His Pro
            100                 105                 110

Gly Asp Val Ile Ser Val Thr Trp Gln Thr Lys Ser Gly Gly Thr Arg
        115                 120                 125

Thr Gly Asn Val Thr Leu Ala Glu Gly Pro Pro Ala Glu Phe Asp Asp
    130                 135                 140

Asp Asp Lys Asp Pro Pro Asp Pro His Gln Pro Asp Met Thr Lys Gly
145                 150                 155                 160

Tyr Cys Pro Gly Gly Arg Trp Gly Phe Gly Asp Leu Ala Val Cys Asp
                165                 170                 175

Gly Glu Lys Tyr Pro Asp Gly Ser Phe Trp His Gln Trp Met Gln Thr
            180                 185                 190

Trp Phe Thr Gly Pro Gln Phe Tyr Phe Asp Cys Val Ser Gly Gly Glu
        195                 200                 205

Pro Leu Pro Gly Pro Pro Pro Pro Gly Gly Cys Gly Gly Ala Ile Pro
    210                 215                 220

Ser Glu Gln Pro Asn Ala Pro
225                 230

<210> 27
<211> 87
<212> PRT
<213> Artificial Sequence
<223> Description of Artificial Sequence:peptide of
      reading frame 2

<400> 27
Ile Cys Ile Thr Ile Thr Ile Thr Arg Pro Arg Pro Ile Thr Ser Ser
  1               5                  10                  15

Cys Pro Arg Val Gly Arg Asp Ser Pro Phe Arg Ser Gly Arg Arg Trp
             20                  25                  30

Arg Ser Arg Ala Arg Ser Asp Arg Val Gly Gly His Pro Pro Phe Ile
         35                  40                  45

Ser Gly Leu Pro Pro Ser Ser Ala Trp Val Leu Ser Thr Thr Thr Ala
     50                  55                  60

Thr Ala His Glu Ser Asn Ala Trp Ser Gly Ala Leu Arg Arg Gln Val
 65                  70                  75                  80

Ser Ala Ser Pro Pro Ala Thr
                 85

<210> 28
<211> 29
<212> PRT
<213> Artificial Sequence
<223> Description of Artificial Sequence:peptide of
      reading frame 2

<400> 28
Ser Pro Arg Ser Thr Ala Leu Arg Ser Thr Arg Pro Pro Arg Trp Arg
  1               5                  10                  15

Thr Arg Leu Thr Gly Ile Ile Pro Val Thr Ser Ser Arg
             20                  25

<210> 29
<211> 13
<212> PRT
<213> Artificial Sequence
<223> Description of Artificial Sequence:peptide of
      reading frame 2

<400> 29
Pro Gly Lys Pro Ser Arg Ala Ala Arg Val Gln Gly Thr
  1               5                  10

<210> 30
<211> 24
<212> PRT
<213> Artificial Sequence
<223> Description of Artificial Sequence:peptide of
      reading frame 2

<400> 30
His Trp Pro Arg Asp Pro Arg Pro Asn Ser Thr Thr Thr Thr Arg Ile
  1               5                  10                  15

His Leu Thr Arg Ile Ser Arg Thr
             20

<210> 31
<211> 77
<212> PRT
<213> Artificial Sequence
<223> Description of Artificial Sequence:peptide of
      reading frame 2

<400> 31
Arg Lys Ala Ile Ala Arg Val Ala Asp Gly Val Leu Ala Thr Trp Pro
  1               5                  10                  15

Cys Ala Thr Ala Arg Ser Thr Pro Thr Ala Arg Phe Gly Thr Ser Gly
             20                  25                  30

Cys Lys Arg Gly Leu Pro Ala His Ser Phe Thr Ser Ile Val Ser Ala
         35                  40                  45

Ala Val Ser Pro Ser Pro Ala Arg Arg His Arg Val Val Ala Val Gly
     50                  55                  60

Gln Phe Arg Pro Ser Ser Pro Thr Leu Pro Glu Asn Ser
 65                  70                  75

<210> 32
<211> 13
<212> PRT
<213> Artificial Sequence
<223> Description of Artificial Sequence:peptide of

reading frame 3

<400> 32
Pro Tyr Ala Ser Pro Ser Pro Ser His Gly Arg Val Arg
1               5                   10

<210> 33
<211> 93
<212> PRT
<213> Artificial Sequence
<223> Description of Artificial Sequence:peptide of
      reading frame 3

<400> 33
Leu Pro Ala Val Pro Gly Trp Ala Gly Ile Arg His Ser Asp Arg Ala
1               5                   10                  15

Gly Asp Gly Asp Arg Gly Pro Asp Pro Ile Gly Trp Gly Val Thr His
            20                  25                  30

Arg Ser Tyr Arg Ala Tyr Arg Leu Pro Arg Leu Gly Cys Cys Arg Gln
        35                  40                  45

Gln Arg Gln Arg Arg Thr Ser Pro Thr Arg Gly Arg Glu Arg Ser Gly
    50                  55                  60

Gly Lys Ser Arg His Leu His Arg Arg Arg Asp His Arg Gly Arg Arg
65                  70                  75                  80

Arg Ser Asp Gln Leu Gly His Arg Asp Gly Gly Arg Ala
                85                  90

<210> 34
<211> 5
<212> PRT
<213> Artificial Sequence
<223> Description of Artificial Sequence:peptide of
      reading frame 3

<400> 34
Arg Ala Ser Ser Arg
1               5

<210> 35
<211> 36
<212> PRT
<213> Artificial Sequence
<223> Description of Artificial Sequence:peptide of
      reading frame 3

<400> 35
   Arg His Leu Gly Asp Leu Ala Asn Gln Val Gly Arg His Ala Tyr Arg
   1               5                   10                  15
   Glu Arg Asp Ile Gly Arg Gly Thr Pro Gly Arg Ile Arg Arg Arg Arg
               20                  25                  30
   Gln Gly Ser Thr
           35

<210> 36
<211> 56
<212> PRT
<213> Artificial Sequence
<223> Description of Artificial Sequence:peptide of
      reading frame 3

<400> 36
Pro Ala Ser Ala Gly His Asp Glu Arg Leu Leu Pro Gly Trp Pro Met
  1               5                  10                  15

Gly Phe Trp Arg Leu Gly Arg Val Arg Arg Arg Glu Val Pro Arg Arg
            20                  25                  30

Leu Val Leu Ala Pro Val Asp Ala Asn Val Val Tyr Arg Pro Thr Val
        35                  40                  45

Leu Leu Arg Leu Cys Gln Arg Arg
    50                  55

<210> 37
<211> 26
<212> PRT
<213> Artificial Sequence
<223> Description of Artificial Sequence:peptide of
      reading frame 3

<400> 37
Ala Pro Pro Arg Pro Ala Ala Thr Gly Trp Leu Arg Trp Gly Asn Ser
  1               5                  10                  15
Val Arg Ala Ala Gln Arg Ser Leu Arg Ile
            20                  25

<210> 38
<211> 374
<212> PRT
<213> Mycobacterium tuberculosis

<220>
<223> 38 kD antigen

<400> 38
Met Lys Ile Arg Leu His Thr Leu Leu Ala Val Leu Thr Ala Ala Pro
  1               5                  10                  15

Leu Leu Leu Ala Ala Ala Gly Cys Gly Ser Lys Pro Pro Ser Gly Ser
            20                  25                  30

Pro Glu Thr Gly Ala Gly Ala Gly Thr Val Ala Thr Thr Pro Ala Ser
            35                  40                  45

Ser Pro Val Thr Leu Ala Glu Thr Gly Ser Thr Leu Leu Tyr Pro Leu
    50                  55                  60

Phe Asn Leu Trp Gly Pro Ala Phe His Glu Arg Tyr Pro Asn Val Thr
65                  70                  75                  80

Ile Thr Ala Gln Gly Thr Gly Ser Gly Ala Gly Ile Ala Gln Ala Ala
                85                  90                  95

Ala Gly Thr Val Asn Ile Gly Ala Ser Asp Ala Tyr Leu Ser Glu Gly
            100                 105                 110

Asp Met Ala Ala His Lys Gly Leu Met Asn Ile Ala Leu Ala Ile Ser
        115                 120                 125

Ala Gln Gln Val Asn Tyr Asn Leu Pro Gly Val Ser Glu His Leu Lys
    130                 135                 140

Leu Asn Gly Lys Val Leu Ala Ala Met Tyr Gln Gly Thr Ile Lys Thr
145                 150                 155                 160

Trp Asp Asp Pro Gln Ile Ala Ala Leu Asn Pro Gly Val Asn Leu Pro
                165                 170                 175

Gly Thr Ala Val Val Pro Leu His Arg Ser Asp Gly Ser Gly Asp Thr
            180                 185                 190

Phe Leu Phe Thr Gln Tyr Leu Ser Lys Gln Asp Pro Glu Gly Trp Gly
        195                 200                 205

Lys Ser Pro Gly Phe Gly Thr Thr Val Asp Phe Pro Ala Val Pro Gly
    210                 215                 220

Ala Leu Gly Glu Asn Gly Asn Gly Gly Met Val Thr Gly Cys Ala Glu
225                 230                 235                 240

Thr Pro Gly Cys Val Ala Tyr Ile Gly Ile Ser Phe Leu Asp Gln Ala
                245                 250                 255

Ser Gln Arg Gly Leu Gly Glu Ala Gln Leu Gly Asn Ser Ser Gly Asn
            260                 265                 270

Phe Leu Leu Pro Asp Ala Gln Ser Ile Gln Ala Ala Ala Ala Gly Phe
        275                 280                 285

Ala Ser Lys Thr Pro Ala Asn Gln Ala Ile Ser Met Ile Asp Gly Pro
    290                 295                 300

Ala Pro Asp Gly Tyr Pro Ile Ile Asn Tyr Glu Tyr Ala Ile Val Asn
305                 310                 315                 320

Asn Arg Gln Lys Asp Ala Ala Thr Ala Gln Thr Leu Gln Ala Phe Leu
                325                 330                 335

His Trp Ala Ile Thr Asp Gly Asn Lys Ala Ser Phe Leu Asp Gln Val
            340                 345                 350

His Phe Gln Pro Leu Pro Pro Ala Val Val Lys Leu Ser Asp Ala Leu
        355                 360                 365

Ile Ala Thr Ile Ser Ser
    370

<210> 39
<211> 5
<212> PRT
<213> Artificial Sequence

<220>
<223> Description of Artificial Sequence:flexible
      polylinker

<400> 39
Gly Gly Gly Gly Ser
  1               5

<210> 40
<211> 9
<212> PRT
<213> Artificial Sequence

<220>
<223> Description of Artificial Sequence:peptide from
      transcription of pET polylinker and XhoI
      restriction site at positions 2143-2168 of
      SEQ ID NO:15

<400> 40
Ile His His Thr Gly Gly Arg Ser Ser
  1               5

<210> 41
<211> 6
<212> PRT
<213> Artificial Sequence

<220>
<223> Description of Artificial Sequence:peptide
      transcribed from positions 901-918 of
      SEQ ID NO:17

<400> 41
Gln Ser Pro Lys Gly Ser
  1               5

## Description

### 1. INTRODUCTION

The present invention relates to fusion proteins containing at least two *Mycobacterium tuberculosis* antigens. In particular, it relates to bi- fusion proteins which contain two individual *M. tuberculosis* antigens, tri- fusion proteins which contain three *M*. *tuberculosis* antigens, tetra-fusion proteins which contain four *M. tuberculosis* antigens, and penta-fusion proteins which contain five *M*. *tuberculosis* antigens, and methods for their use in the diagnosis, treatment and prevention of tuberculosis infection.

### 2. BACKGROUND OF THE INVENTION

Tuberculosis is a chronic infectious disease caused by infection with *M*. *tuberculosis.* It is a major disease in developing countries, as well as an increasing problem in developed areas of the world, with about 8 million new cases and 3 million deaths each year. Although the infection may be asymptomatic for a considerable period of time, the disease is most commonly manifested as an acute inflammation of the lungs, resulting in fever and a nonproductive cough. If untreated, serious complications and death typically result.

Although tuberculosis can generally be controlled using extended antibiotic therapy, such treatment is not sufficient to prevent the spread of the disease. Infected individuals may be asymptomatic, but contagious, for some time. In addition, although compliance with the treatment regimen is critical, patient behavior is difficult to monitor. Some patients do not complete the course of treatment, which can lead to ineffective treatment and the development of drug resistance.

In order to control the spread of tuberculosis, effective vaccination and accurate early diagnosis of the disease are of utmost importance. Currently, vaccination with live bacteria is the most efficient method for inducing protective immunity. The most common Mycobacterium employed for this purpose is *Bacillus* Calmette-Guerin (BCG), an avirulent strain of *M. bovis.* However, the safety and efficacy of BCG is a source of controversy and some countries, such as the United States, do not vaccinate the general public with this agent.

Diagnosis of tuberculosis is commonly achieved using a skin test, which involves intradermal exposure to tuberculin PPD (protein-purified derivative). Antigen-specific T cell responses result in measurable induration at the injection site by 48-72 hours after injection, which indicates exposure to Mycobacterial antigens. Sensitivity and specificity have, however, been a problem with this test, and individuals vaccinated with BCG cannot be distinguished from infected individuals.

While macrophages have been shown to act as the principal effectors of *M*. *tuberculosis* immunity, T cells are the predominant inducers of such immunity. The essential role of T cells in protection against *M. tuberculosis* infection is illustrated by the frequent occurrence *of M. tuberculosis* in Acquired Immunodeficiency Syndrome patients, due to the depletion of CD4⁺ T cells associated with human immunodeficiency virus (HIV) infection. Mycobacterium-reactive CD4⁺ T cells have been shown to be potent producers of gamma-interferon (IFN-γ), which, in turn, has been shown to trigger the anti-mycobacterial effects of macrophages in mice. While the role of IFN-γ in humans is less clear, studies have shown that 1,25-dihydroxy-vitamin D3, either alone or in combination with IFN-γ or tumor necrosis factor-alpha, activates human macrophages to inhibit *M. tuberculosis* infection. Furthermore, it is known that IFN-γ stimulates human macrophages to make 1,25-dihydroxy-vitamin D3. Similarly, interleukin-12 (IL-12) has been shown to play a role in stimulating resistance to *M*. *tuberculosis* infection. For a review of the immunology of *M. tuberculosis* infection, see Chan and Kaufinann, 1994, Tuberculosis: Pathogenesis, Protection and Control, Bloom (ed.), ASM Press, Washington, DC.

Accordingly, there is a need for improved vaccines, and improved methods for diagnosis, preventing and treating tuberculosis.

### 3. SUMMARY OF THE INVENTION

The present invention relates to fusion proteins *of M. tuberculosis* antigens. In particular, it relates to fusion polypeptides that contain two or more *M. tuberculosis* antigens, polynucleotides encoding such polypeptides, methods of using the polypeptides and polynucleotides in the diagnosis, treatment and prevention *of M. tuberculosis* infection.

The present invention is based, in part, on the inventors' discovery that polynucleotides which contain two to five *M. tuberculosis* coding sequences produce recombinant fusion proteins that retain the immunogenicity and antigenicity of their individual components. The fusion proteins described herein induced both T cell and B cell responses, as measured by T cell proliferation, cytokine production, and antibody production. Furthermore, a fusion protein was used as an immunogen with adjuvants *in vivo* to elicit both cell-mediated and humoral immunity to *M. tuberculosis.* Additionally, a fusion protein was made by a fusion construct and used in a vaccine formulation with an adjuvant to afford long-term protection in animals against the development of tuberculosis. The fusion protein was a more effective immunogen than a mixture of its individual protein components.

In a specific embodiment of the invention, the isolated or purified *M. tuberculosis* polypeptides of the invention may be formulated as pharmaceutical compositions for administration into a subject in the prevention and/or treatment of *M. tuberculosis* infection. The immunogenicity of the fusion protein may be enhanced by the inclusion of an adjuvant.

In another aspect of the invention, the isolated or purified polynucleotides are used to produce recombinant fusion polypeptide antigens *in vitro.* Alternatively, the polynucleotides may be administered directly into a subject as DNA vaccines to cause antigen expression in the subject, and the subsequent induction of an anti-*M*. *tuberculosis* immune response.

It is also an object of the invention that the polypeptides be used in *in vitro* assays for detecting humoral antibodies or cell-mediated immunity against *M. tuberculosis* for diagnosis of infection or monitor of disease progression. Additionally, the polypeptides may be used as an *in vivo* diagnostic agent in the form of an intradermal skin test. Alternatively, the polypeptides may be used as immunogens to generate anti-*M*. *tuberculosis* antibodies in a non-human animal. The antibodies can be used to detect the target antigens *in vivo* and *in vitro.*

### 4. BRIEF DESCRIPTION OF THE DRAWINGS

- Figure 1A and 1B.: The nucleotide sequence (SEQ ID NO:1) and amino acid sequence (SEQ ID NO:2) of tri-fusion protein Ra12-TbH9-Ra35 (designated Mtb32A).
- Figure 2 :: The nucleotide sequence (SEQ ID NO:3) and amino acid sequence (SEQ ID NO:4) of tri-fusion protein Erd14-DPV-MTI (designated Mtb39A).
- Figure 3A - 3D:: The nucleotide sequence (SEQ ID NO:5) and amino acid sequence (SEQ ID NO:6) of tri-fusion protein TbRa3-38kD- Tb38-1.
- Figure 4A - 4D:: The nucleotide sequence (SEQ ID NO:7) and amino acid sequence (SEQ ID NO:8) of bi-fusion protein TbH9-Tb38-1.
- Figure 5A - 5J:: The nucleotide sequence (SEQ ID NO:9) and amino acid sequence (SEQ ID NO: 10) of tetra-fusion protein TbRa3-38kD-Tb38-1-DPEP (designated TbF-2).
- Figure 6A and 6B:: The nucleotide sequence (SEQ ID NO:11) and amino acid sequence (SEQ ID NO: 12) of penta-fusion protein Erd14-DPV-MTI-MSL-MTCC2 (designated Mtb88f).
- Figure 7A and 7B:: The nucleotide sequence (SEQ ID NO: 13) and amino acid sequence (SEQ ID NO: 14) of tetra-fusion protein Erd14-DPV-MTI-MSL (designated Mtb46f).
- Figure 8A and 8B:: The nucleotide sequence (SEQ ID NO:15) and amino acid sequence (SEQ ID NO:16) of tetra-fusion protein DPV-MTI-MSL-MTCC2 (designated Mtb71f).
- Figure 9A and 9B:: The nucleotide sequence (SEQ ID NO:17) and amino acid sequence (SEQ ID NO:18) of tri-fusion protein DPV-MTI-MSL (designated Mtb31f).
- Figure 10A and 10B:: The nucleotide sequence (SEQ ID NO:19) and amino acid sequence (SEQ ID NO:20) of tri-fusion protein TbH9-DPV-MTI (designated Mtb61f).
- Figure 11A and 11B:: The nucleotide sequence (SEQ ID NO:21) and amino acid sequence (SEQ ID NO:22) of tri-fusion protein Erd14-DPV-MTI (designated Mtb36f).
- Figure 12A and 12B:: The nucleotide sequence (SEQ ID NO:23) and amino acid sequence (SEQ ID NO:24) of bi-fusion protein TbH9-Ra35 (designated Mtb59f).
- Figure 13A and 13B:: The nucleotide sequence (SEQ ID NO:25) and amino acid sequence (SEQ ID NO:26) of bi-fusion protein Ra12-DPPD (designated Mtb24).
- Figure 14A-14F:: T cell proliferation responses of six PPD+ subjects when stimulated with two fusion proteins and their individual components.
- Figure 15A-15F:: IFN-γ production of six PPD+ subjects when stimulated with two fusion proteins and their individual components.
- Figure 16A-16F:: T cell proliferation of mice immunized with a fusion protein or its individual components and an adjuvant.
- Figure 17:: IFN-γ production of mice immunized with a fusion protein or its individual components and an adjuvant.
- Figure 18:: IL-4 production of mice immunized with a fusion protein or its individual components and an adjuvant.
- Figure 19A-19F:: Serum antibody concentrations of mice immunized with a fusion protein or its individual components and an adjuvant.
- Figure 20A-20C:: Survival of guinea pigs after aerosol challenge of *M. tuberculosis.* Fusion proteins, Mtb32A and Mtb39A, were formulated in adjuvant SBAS1c (20A), SBAS2 (20B) or SBAS7 (20C), and used as an immunogen in guinea pigs prior to challenge with bacteria. BCG is the positive control.
- Figure 21A and 21B:: Stimulation of proliferation and IFN-γ production in TbH9-specific T cells by the fusion protein TbH9-Tb38-1.
- Figure 22A and 22B:: Stimulation of proliferation and IFN-γ production in Tb38-1-specific T cells by the fusion protein TbH9-Tb38-1.
- Figure 23A and 23B:: Stimulation of proliferation and IFN-γ production in T cells previously shown to respond to both TbH-9 and Tb38-1 antigens by the fusion protein TbH9-Tb38-1.

### 5. DETAILED DESCRIPTION OF THE INVENTION

The present invention relates to antigens useful for the treatment and prevention of tuberculosis, polynucleotides encoding such antigens, and methods for their use. The antigens of the present invention are fusion polypeptides of *M. tuberculosis* antigens and variants thereof. More specifically, the antigens of the present invention comprise at least two polypeptides of *M. tuberculosis* that are fused into a larger fusion polypeptide molecule. The antigens of the present invention may further comprise other components designed to enhance the immunogenicity of the antigens or to improve these antigens in other aspects, for example, the isolation of these antigens through addition of a stretch of histidine residues at one end of the antigen.

### 5.1. M. TUBERCULOSIS SPECIFIC ANTIGENS

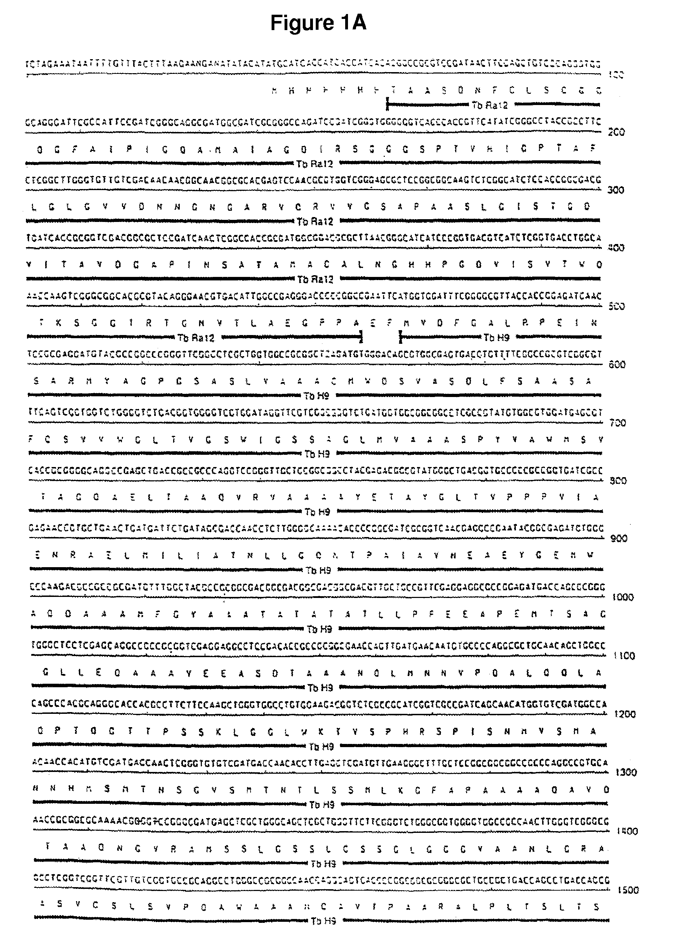

The antigens of the present invention are exemplified in Figures 1A through 13B, including homologues and variants of those antigens. These antigens may be modified, for example, by adding linker peptide sequences as described below. These linker peptides may be inserted between one or more polypeptides which make up each of the fusion proteins presented in Figures 1A through 13B. Other antigens of the present invention are antigens described in Figures 1A through 13B which have been linked to a known antigen of *M. tuberculosis,* such as the previously described 38 kD (SEQ ID NO:27) antigen (Andersen and Hansen,1989, Infect. Immun. 57:2481-2488; Genbank Accession No. M30046).

### 5.2. IMMUNOGENICITY ASSAYS

Antigens described herein, and immunogenic portions thereof, have the ability to induce an immunogenic response. More specifically, the antigens have the ability to induce proliferation and/or cytokine production (*i.e.,* interferon-γ and/or interleukin-12 production) in T cells, NK cells, B cells and/or macrophages derived from an *M. tuberculosis-immune* individual. The selection of cell type for use in evaluating an immunogenic response to a antigen will depend on the desired response. For example, interleukin-12 production is most readily evaluated using preparations containing B cells and/or macrophages. An *M. tuberculosis-immune* individual is one who is considered to be resistant to the development of tuberculosis by virtue of having mounted an effective T cell response to *M. tuberculosis* (*i.e.,* substantially free of disease symptoms). Such individuals may be identified based on a strongly positive (*i.e.,* greater than about 10 mm diameter induration) intradermal skin test response to tuberculosis proteins (PPD) and an absence of any signs or symptoms of tuberculosis disease. T cells, NK cells, B cells and macrophages derived from *M. tuberculosis-immune* individuals may be prepared using methods known to those of ordinary skill in the art. For example, a preparation of PBMCs (*i.e*., peripheral blood mononuclear cells) may be employed without further separation of component cells. PBMCs may generally be prepared, for example, using density centrifugation through "FICOLL" (Winthrop Laboratories, NY). T cells for use in the assays described herein may also be purified directly from PBMCs. Alternatively, an enriched T cell line reactive against mycobacterial proteins, or T cell clones reactive to individual mycobacterial proteins, may be employed. Such T cell clones may be generated by, for example, culturing PBMCs from *M. tuberculosis-immune* individuals with mycobacterial proteins for a period of 2-4 weeks. This allows expansion of only the mycobacterial protein-specific T cells, resulting in a line composed solely of such cells. These cells may then be cloned and tested with individual proteins, using methods known to those of ordinary skill in the art, to more accurately define individual T cell specificity. In general, antigens that test positive in assays for proliferation and/or cytokine production (*i.e*., interferon-γ and/or interleukin-12 production) performed using T cells, NK cells, B cells and/or macrophages derived from an *M. tuberculosis*-immune individual are considered immunogenic. Such assays may be performed, for example, using the representative procedures described below. Immunogenic portions of such antigens may be identified using similar assays, and may be present within the polypeptides described herein.

The ability of a polypeptide (*e.g*., an immunogenic antigen, or a portion or other variant thereof) to induce cell proliferation is evaluated by contacting the cells (*e.g*., T cells and/or NK cells) with the polypeptide and measuring the proliferation of the cells. In general, the amount of polypeptide that is sufficient for evaluation of about 10⁵ cells ranges from about 10 ng/mL to about 100 µg/mL and preferably is about 10 µg/mL. The incubation of polypeptide with cells is typically performed at 37°C for about six days. Following incubation with polypeptide, the cells are assayed for a proliferative response, which may be evaluated by methods known to those of ordinary skill in the art, such as exposing cells to a pulse of radiolabeled thymidine and measuring the incorporation of label into cellular DNA. In general, a polypeptide that results in at least a three fold increase in proliferation above background (*i.e*., the proliferation observed for cells cultured without polypeptide) is considered to be able to induce proliferation.

The ability of a polypeptide to stimulate the production of interferon-γ and/or interleukin-12 in cells may be evaluated by contacting the cells with the polypeptide and measuring the level of interferon-γ or interleukin-12 produced by the cells. In general, the amount of polypeptide that is sufficient for the evaluation of about 10⁵ cells ranges from about 10 ng/mL to about 100 µg/mL and preferably is about 10 µg/mL. The polypeptide may be, but need not be, immobilized on a solid support, such as a bead or a biodegradable microsphere, such as those described in U.S. Patent Nos. 4,897,268 and 5,075,109. The incubation of polypeptide with the cells is typically performed at 37°C for about six days. Following incubation with polypeptide, the cells are assayed for interferon-γ and/or interleukin-12 (or one or more subunits thereof), which may be evaluated by methods known to those of ordinary skill in the art, such as an enzyme-linked immunosorbent assay (ELISA) or, in the case of IL-12 P70 subunit, a bioassay such as an assay measuring proliferation of T cells. In general, a polypeptide that results in the production of at least 50 pg of interferon-γ per mL of cultured supernatant (containing 10⁴-10⁵ T cells per mL) is considered able to stimulate the production of interferon-γ. A polypeptide that stimulates the production of at least 10 pg/mL of IL-12 P70 subunit, and/or at least 100 pg/mL of IL-12 P40 subunit, per 10⁵ macrophages or B cells (or per 3 x 10⁵ PBMC) is considered able to stimulate the production of IL-12.

In general, immunogenic antigens are those antigens that stimulate proliferation and/or cytokine production (*i.e*., interferon-γ and/or interleukin-12 production) in T cells, NK cells, B cells and/or macrophages derived from at least about 25% of *M. tuberculosis-*immune individuals. Among these immunogenic antigens, polypeptides having superior therapeutic properties may be distinguished based on the magnitude of the responses in the above assays and based on the percentage of individuals for which a response is observed. In addition, antigens having superior therapeutic properties will not stimulate proliferation and/or cytokine production *in vitro* in cells derived from more than about 25% of individuals who are not *M. tuberculosis-immune,* thereby eliminating responses that are not specifically due to *M. tuberculosis*-responsive cells. Those antigens that induce a response in a high percentage of T cell, NK cell, B cell and/or macrophage preparations from *M. tuberculosis-immune* individuals (with a low incidence of responses in cell preparations from other individuals) have superior therapeutic properties.

Antigens with superior therapeutic properties may also be identified based on their ability to diminish the severity *of M. tuberculosis* infection in experimental animals, when administered as a vaccine. Suitable vaccine preparations for use on experimental animals are described in detail below. Efficacy may be determined based on the ability of the antigen to provide at least about a 50% reduction in bacterial numbers and/or at least about a 40% decrease in mortality following experimental infection. Suitable experimental animals include mice, guinea pigs and primates.

### 5.3. ISOLATION OF CODING SEQUENCES

The present invention also relates to nucleic acid molecules that encode fusion polypeptides of *M. tuberculosis*. In a specific embodiment by way of example in Section 6, *infra,* thirteen *M. tuberculosis* fusion coding sequences were constructed. In accordance with the invention, any nucleotide sequence which encodes the amino acid sequence of the fusion protein can be used to generate recombinant molecules which direct the expression of the coding sequence.

In order to clone full-length coding sequences or homologous variants to generate the fusion polynucleotides, labeled DNA probes designed from any portion of the nucleotide sequences or their complements disclosed herein may be used to screen a genomic or cDNA library made from various strains of *M. tuberculosis* to identify the coding sequence of each individual component. Isolation of coding sequences may also be carried out by the polymerase chain reactions (PCR) using two degenerate oligonucleotide primer pools designed on the basis of the coding sequences disclosed herein.

The invention also relates to isolated or purified polynucleotides complementary to the nucleotide sequences of SEQ ID NOS:1, 3, 5, 7, 9, 11, 13, 15, 17, 19, 21, 23 and 25, and polynucleotides that selectively hybridize to such complementary sequences. In a preferred embodiment, a polynucleotide which hybridizes to the sequence of SEQ ID NOS:1, 3, 5, 7, 9, 11, 13, 15, 17, 19, 21, 23 and 25 or its complementary sequence under conditions of low stringency and encodes a protein that retains the immunogenicity of the fusion proteins of SEQ ID NOS:2, 4, 6, 8, 10, 12, 14, 16, 18, 20, 22, 24 and 26 is provided. By way of example and not limitation, exemplary conditions of low stringency are as follows (see also Shilo and Weinberg, 1981, Proc. Natl. Acad. Sci. USA 78:6789-6792): Filters containing DNA are pretreated for 6 h at 40°C in a solution containing 35% formamide, 5X SSC, 50 mM Tris-HCl (pH 7.5), 5mM EDTA, 0.1% PVP, 0.1% Ficoll, 1% BSA, and 500 µg/mℓ denatured salmon sperm DNA. Hybridizations are carried out in the same solution with the following modifications: 0.02% PVP, 0.02% Ficoll, 0.2% BSA, 100 µg/mℓ salmon sperm DNA, 10% (wt/vol) dextran sulfate, and 5-20 X 10⁶ cpm ³²P-labeled probe is used. Filters are incubated in hybridization mixture for 18-20 h at 40°C, and then washed for 1.5 h at 55°C in a solution containing 2X SSC, 25 mM Tris-HCl (pH 7.4), 5 mM EDTA, and 0.1% SDS. The wash solution is replaced with fresh solution and incubated an additional 1.5 h at 60°C. Filters are blotted dry and exposed for autoradiography. If necessary, filters are washed for a third time at 65-68°C and re-exposed to film. Other conditions of low stringency which may be used are well known in the art (*e.g*., as employed for cross-species hybridizations).

In another preferred embodiment, a polynucleotide which hybridizes to the coding sequence of SEQ ID NOS:1, 3, 5, 7, 9, 11, 13, 15, 17, 19, 21, 23 and 25 or its complementary sequence under conditions of high stringency and encodes a protein that retains the immunogenicity of the fusion proteins of SEQ ID NOS:2, 4, 6, 8, 10, 12, 14, 16, 18, 20, 22, 24 and 26 is provided. By way of example and not limitation, exemplary conditions of high stringency are as follows: Prehybridization of filters containing DNA is carried out for 8 h to overnight at 65°C in buffer composed of 6X SSC, 50 mM Tris-HCl (pH 7.5), 1 mM EDTA, 0.02% PVP, 0.02% Ficoll, 0.02% BSA, and 500 µg/mL denatured salmon sperm DNA. Filters are hybridized for 48 h at 65 °C in prehybridization mixture containing 100 µg/mL denatured salmon sperm DNA and 5-20 X 10⁶ cpm of ³²P-labeled probe. Washing of filters is done at 37°C for 1 h in a solution containing 2X SSC, 0.01% PVP, 0.01% Ficoll, and 0.01% BSA. This is followed by a wash in 0.1X SSC at 50°C for 45 min before autoradiography. Other conditions of high stringency which may be used are well known in the art.

In yet another preferred embodiment, a polynucleotide which hybridizes to the coding sequence of SEQ ID NOS:1, 3, 5, 7, 9, 11, 13, 15, 17, 19, 21, 23 and 25 or its complementary sequence under conditions of moderate stringency and encodes a protein that retains the immunogenicity of the fusion proteins of SEQ ID NOS:2, 4, 6, 8, 10, 12, 14, 16, 18, 20, 22, 24 and 26 is provided. Exemplary conditions of moderate stringency are as follows: Filters containing DNA are pretreated for 6 h at 55°C in a solution containing 6X SSC, 5X Denhart's solution, 0.5% SDS and 100 µg/mL denatured salmon sperm DNA. Hybridizations are carried out in the same solution and 5-20 X 10⁶ cpm ³²P-labeled probe is used. Filters are incubated in hybridization mixture for 18-20 h at 55 °C, and then washed twice for 30 minutes at 60°C in a solution containing 1X SSC and 0.1% SDS. Filters are blotted dry and exposed for autoradiography. Other conditions of moderate stringency which may be used are well-known in the art. Washing of filters is done at 37°C for 1 h in a solution containing 2X SSC, 0.1% SDS.

### 5.4. POLYPEPTIDES ENCODED BY THE CODING SEQUENCES

In accordance with the invention, a polynucleotide of the invention which encodes a fusion protein, fragments thereof, or functional equivalents thereof may be used to generate recombinant nucleic acid molecules that direct the expression of the fusion protein, fragments thereof, or functional equivalents thereof, in appropriate host cells. The fusion polypeptide products encoded by such polynucleotides may be altered by molecular manipulation of the coding sequence.

Due to the inherent degeneracy of the genetic code, other DNA sequences which encode substantially the same or a functionally equivalent amino acid sequence, may be used in the practice of the invention for the expression of the fusion polypeptides. Such DNA sequences include those which are capable of hybridizing to the coding sequences or their complements disclosed herein under low, moderate or high stringency conditions described in Sections 5.3, *supra.*

Altered nucleotide sequences which may be used in accordance with the invention include deletions, additions or substitutions of different nucleotide residues resulting in a sequence that encodes the same or a functionally equivalent gene product. The gene product itself may contain deletions, additions or substitutions of amino acid residues, which result in a silent change thus producing a functionally equivalent antigenic epitope. Such conservative amino acid substitutions may be made on the basis of similarity in polarity, charge, solubility, hydrophobicity, hydrophilicity, and/or the amphipathic nature of the residues involved. For example, negatively charged amino acids include aspartic acid and glutamic acid; positively charged amino acids include lysine, histidine and arginine; amino acids with uncharged polar head groups having similar hydrophilicity values include the following: glycine, asparagine, glutamine, serine, threonine and tyrosine; and amino acids with nonpolar head groups include alanine, valine, isoleucine, leucine, phenylalanine, proline, methionine and tryptophan.

The nucleotide sequences of the invention may be engineered in order to alter the fusion protein coding sequence for a variety of ends, including but not limited to, alterations which modify processing and expression of the gene product. For example, mutations may be introduced using techniques which are well known in the art, *e.g*., site-directed mutagenesis, to insert new restriction sites, to alter glycosylation patterns, phosphorylation, etc.

In an alternate embodiment of the invention, the coding sequence of a fusion protein could be synthesized in whole or in part, using chemical methods well known in the art. See, e.g., Caruthers et al., 1980, Nuc. Acids Res. Symp. Ser. 7:215-233; Crea and Horn, 180, Nuc. Acids Res. 9(10):2331*;* Matteucci and Caruthers, 1980, Tetrahedron Letter 21:719; and Chow and Kempe, 1981, Nuc. Acids Res. 9(12):2807-2817. Alternatively, the polypeptide itself could be produced using chemical methods to synthesize an amino acid sequence in whole or in part. For example, peptides can be synthesized by solid phase techniques, cleaved from the resin, and purified by preparative high performance liquid chromatography. (See Creighton, 1983, Proteins Structures And Molecular Principles, W.H. Freeman and Co., N.Y. pp. 50-60). The composition of the synthetic polypeptides may be confirmed by amino acid analysis or sequencing (*e.g*., the Edman degradation procedure; see Creighton, 1983, Proteins, Structures and Molecular Principles, W.H. Freeman and Co., N.Y., pp. 34-49).

Additionally, the coding sequence of a fusion protein can be mutated *in vitro* or *in vivo,* to create and/or destroy translation, initiation, and/or termination sequences, or to create variations in coding regions and/or form new restriction endonuclease sites or destroy preexisting ones, to facilitate further *in vitro* modification. Any technique for mutagenesis known in the art can be used, including but not limited to, chemical mutagenesis, *in vitro* site-directed mutagenesis (Hutchinson, C., et al., 1978, J. Biol. Chem 253:6551), use of TAB® linkers (Pharmacia), and the like. It is important that the manipulations do not destroy immunogenicity of the fusion polypeptides.

In addition, nonclassical amino acids or chemical amino acid analogs can be introduced as a substitution or addition into the sequence. Non-classical amino acids include, but are not limited to, the D-isomers of the common amino acids, α-amino isobutyric acid, 4-aminobutyric acid, Abu, 2-amino butyric acid, γ-Abu, ∈-Ahx, 6-amino hexanoic acid, Aib, 2-amino isobutyric acid, 3-amino propionic acid, ornithine, norleucine, norvaline, hydroxyproline, sarcosine, citrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogs in general. Furthermore, the amino acid can be D (dextrorotary) or L (levorotary).

In a specific embodiment, the coding sequences of each antigen in the fusion protein are joined at their amino- or carboxy-terminus via a peptide bond in any order. Alternatively, a peptide linker sequence may be employed to separate the individual polypeptides that make-up a fusion polypeptide by a distance sufficient to ensure that each polypeptide folds into a secondary and tertiary structure that maximizes its antigenic effectiveness for preventing and treating tuberculosis. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may be from 1 to about 50 amino acids in length. Peptide sequences are not required when the first and second polypeptides have non-essential N-terminal amino acid regions that can be used to separate the functional domains and prevent steric interference. For example, the antigens in a fusion protein may be connected by a flexible polylinker such as Gly-Cys-Gly or Gly-Gly-Gly-Gly-Ser repeated 1 to 3 times (Bird et al., 1988, Science 242:423-426; Chaudhary et al., 1990, Proc. Natl. Acad. Sci. U.S.A. 87:1066-1070).

In one embodiment, such a protein is produced by recombinant expression of a nucleic acid encoding the protein. Such a fusion product can be made by ligating the appropriate nucleic acid sequences encoding the desired amino acid sequences to each other by methods known in the art, in the proper coding frame, and expressing the product by methods known in the art. Alternatively, such a product may be made by protein synthetic techniques, e.g., by use of a peptide synthesizer. Coding sequences for other molecules such as a cytokine or an adjuvant can be added to the fusion polynucleotide as well.

### 5.5. PRODUCTION OF FUSION PROTEINS

In order to produce a *M*. *tuberculosis* fusion protein of the invention, the nucleotide sequence coding for the protein, or a functional equivalent, is inserted into an appropriate expression vector, *i.e*., a vector which contains the necessary elements for the transcription and translation of the inserted coding sequence. The host cells or cell lines transfected or transformed with recombinant expression vectors can be used for a variety of purposes. These include, but are not limited to, large scale production of the fusion protein.

Methods which are well known to those skilled in the art can be used to construct expression vectors containing a fusion coding sequence and appropriate transcriptional/translational control signals. These methods include *in vitro* recombinant DNA techniques, synthetic techniques and *in vivo* recombination/genetic recombination. (*See, e.g.,* the techniques described in Sambrook et al., 1989, Molecular Cloning A Laboratory Manual, Cold Spring Harbor Laboratory, N.Y. and Ausubel et al., 1989, Current Protocols in Molecular Biology, Greene Publishing Associates and Wiley Interscience, N.Y.). RNA capable of encoding a polypeptide may also be chemically synthesized (Gait, ed, 1984, Oligonucleoide Synthesis, IRL Press, Oxford).

### 5.5.1. EXPRESSION SYSTEMS

A variety of host-expression vector systems may be utilized to express a fusion protein coding sequence. These include, but are not limited to, microorganisms such as bacteria (*e.g., E. coli, B. subtilis*) transformed with recombinant bacteriophage DNA, plasmid DNA or cosmid DNA expression vectors containing a coding sequence; yeast (*e.g*., *Saccharomycdes, Pichia*) transformed with recombinant yeast expression vectors containing a coding sequence; insect cell systems infected with recombinant virus expression vectors (*e.g*., baculovirus) containing a coding sequence; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing a coding sequence; or mammalian cell systems (*e.g*., COS, CHO, BHK, 293, 3T3 cells). The expression elements of these systems vary in their strength and specificities.

Depending on the host/vector system utilized, any of a number of suitable transcription and translation elements, including constitutive and inducible promoters, may be used in the expression vector. For example, when cloning in bacterial systems, inducible promoters such as pL of bacteriophage λ, plac, ptrp, ptac (ptrp-lac hybrid promoter; cytomegalovirus promoter) and the like may be used; when cloning in insect cell systems, promoters such as the baculovirus polyhedron promoter may be used; when cloning in plant cell systems, promoters derived from the genome of plant cells (*e.g*., heat shock promoters; the promoter for the small subunit of RUBISCO; the promoter for the chlorophyll α/β binding protein) or from plant viruses (*e*.*g*., the 35S RNA promoter of CaMV; the coat protein promoter of TMV) may be used; when cloning in mammalian cell systems, promoters derived from the genome of mammalian cells (*e.g.*, metallothionein promoter) or from mammalian viruses (*e.g*., the adenovirus late promoter; the vaccinia virus 7.5K promoter) may be used; when generating cell lines that contain multiple copies of a the antigen coding sequence, SV40-, BPV- and EBV-based vectors may be used with an appropriate selectable marker.

Bacterial systems are preferred for the expression *of M. tuberculosis* antigens. For *in vivo* delivery, a bacterium such as *Bacillus-Calmette-Guerrin* may be engineered to express a fusion polypeptide of the invention on its cell surface. A number of other bacterial expression vectors may be advantageously selected depending upon the use intended for the expressed products. For example, when large quantities of the fusion protein are to be produced for formulation of pharmaceutical compositions, vectors which direct the expression of high levels of fusion protein products that are readily purified may be desirable. Such vectors include, but are not limited to, the *E*. *coli* expression vector pUR278 (Ruther et al., 1983, EMBO J. 2:1791), in which a coding sequence may be ligated into the vector in frame with the *lacZ* coding region so that a hybrid protein is produced; pIN vectors (Inouye and Inouye, 1985, Nucleic Acids Res. 13:3101-3109; Van Heeke and Schuster, 1989, J. Biol. Chem. 264:5503-5509); and the like. pGEX vectors may also be used to express foreign polypeptides as fusion proteins with glutathione S-transferase (GST). In general, such fusion proteins are soluble and can be purified easily from lysed cells by adsorption to glutathione-agarose beads followed by elution in the presence of free glutathione. The pGEX vectors are designed to include thrombin or factor Xa protease cleavage sites so that the cloned fusion polypeptide of interest can be released from the GST moiety.

### 5.5.2. PROTEIN PURIFICATION

Once a recombinant protein is expressed, it can be identified by assays based on the physical or functional properties of the product, including radioactive labeling of the product followed by analysis by gel electrophoresis, radioimmunoassay, ELISA, bioassays, etc.

Once the encoded protein is identified, it may be isolated and purified by standard methods including chromatography (*e.g*., high performance liquid chromatography, ion exchange, affinity, and sizing column chromatography), centrifugation, differential solubility, or by any other standard technique for the purification of proteins. The actual conditions used will depend, in part, on factors such as net charge, hydrophobicity, hydrophilicity, etc., and will be apparent to those having skill in the art. The functional properties may be evaluated using any suitable assay such as antibody binding, induction of T cell proliferation, stimulation of cytokine production such as IL2, IL-4 and IFN-γ. For the practice of the present invention, it is preferred that each fusion protein is at least 80% purified from other proteins. It is more preferred that they are at least 90% purified. For *in vivo* administration, it is preferred that the proteins are greater than 95% purified.

### 5.6. USES OF THE FUSION PROTEIN CODING SEQUENCE

The fusion protein coding sequence of the invention may be used to encode a protein product for use as an immunogen to induce and/or enhance immune responses to *M*. *tuberculosis.* In addition, such coding sequence may be ligated with a coding sequence of another molecule such as cytokine or an adjuvant. Such polynucleotides may be used *in vivo* as a DNA vaccine (U.S. Patent Nos. 5,589,466; 5,679,647; 5,703,055). In this embodiment of the invention, the polynucleotide expresses its encoded protein in a recipient to directly induce an immune response. The polynucleotide may be injected into a naive subject to prime an immune response to its encoded product, or administered to an infected or immunized subject to enhance the secondary immune responses.

In a preferred embodiment, a therapeutic composition comprises a fusion protein coding sequence or fragments thereof that is part of an expression vector. In particular, such a polynucleotide contains a promoter operably linked to the coding region, said promoter being inducible or constitutive, and, optionally, tissue-specific. In another embodiment, a polynucleotide contains a coding sequence flanked by regions that promote homologous recombination at a desired site in the genome, thus providing for intrachromosomal expression of the coding sequence (Koller and Smithies, 1989, Proc. Natl. Acad. Sci. USA 86:8932-8935; Zijlstra et al., 1989, Nature 342:435-438).

Delivery of the nucleic acid into a subject may be either direct, in which case the subject is directly exposed to the nucleic acid or nucleic acid-carrying vector, or indirect, in which case, cells are first transformed with the nucleic acid *in vitro,* then transplanted into the subject. These two approaches are known, respectively, as *in vivo* or *ex vivo* gene transfer.

In a specific embodiment, the nucleic acid is directly administered *in vivo,* where it is expressed to produce the encoded fusion protein product. This can be accomplished by any of numerous methods known in the art, *e.g*., by constructing it as part of an appropriate nucleic acid expression vector and administering it so that it becomes intracellular, *e.g*., by infection using a defective or attenuated retroviral or other viral vector (*see,* U.S. Patent No. 4,980,286), or by direct injection of naked DNA, or by use of microparticle bombardment (e.g., a gene gun; Biolistic, Dupont), or coating with lipids or cell-surface receptors or transfecting agents, encapsulation in liposomes, microparticles, or microcapsules (United States Patent Nos. 5,407,609; 5,853,763; 5,814,344 and 5,820,883), or by administering it in linkage to a peptide which is known to enter the nucleus, by administering it in linkage to a ligand subject to receptor-mediated endocytosis (*see, e.g.,* Wu and Wu, 1987, J. Biol. Chem. 262:4429-4432) which can be used to target cell types specifically expressing the receptors, etc. In another embodiment, a nucleic acid-ligand complex can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor (*see, e.g.,* PCT Publications WO 92/06180 dated April 16, 1992; WO 92/22635 dated December 23, 1992; WO92/20316 dated November 26, 1992; WO93/14188 dated July 22, 1993; WO 93/20221 dated October 14, 1993). Alternatively, the nucleic acid can be introduced intracellularly and incorporated within host cell DNA for expression, by homologous recombination (Koller and Smithies, 1989, Proc. Natl. Acad. Sci. USA 86:8932-8935; Zijlstra et al., 1989, Nature 342:435-438).

In a specific embodiment, a viral vector such as a retroviral vector can be used (*see,* Miller et al., 1993, Meth. Enzymol. 217:581-599). Retroviral vectors have been modified to delete retroviral sequences that are not necessary for packaging of the viral genome and integration into host cell DNA. A fusion coding sequence is cloned into the vector, which facilitates delivery of the nucleic acid into a recipient. More detail about retroviral vectors can be found in Boesen et al., 1994, Biotherapy 6:291-302, which describes the use of a retroviral vector to deliver the *mdr1* gene to hematopoietic stem cells in order to make the stem cells more resistant to chemotherapy. Other references illustrating the use of retroviral vectors in gene therapy are: Clowes et al., 1994, J. Clin. Invest. 93:644-651; Kiem et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Adeno-associated virus (AAV) has also been proposed for use in *in vivo* gene transfer (Walsh et al., 1993, Proc. Soc. Exp. Biol. Med. 204:289-300.

Another approach involves transferring a construct to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a subject.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see *e.g*., Loeffler and Behr, 1993, Meth. Enzymol. 217:599-618; Cohen et al., 1993, Meth. Enzymol. 217:618-644; Cline, 1985, Pharmac. Ther. 29:69-92) and may be used in accordance with the present invention.

The polynucleotides of the invention may also be used in the diagnosis of tuberculosis for detection of polynucleotide sequences specific to *M. tuberculosis* in a patient. Such detection may be accomplished, for example, by isolating polynucleotides from a biological sample obtained from a patient suspected of being infected with the bacteria. Upon isolation of polynucleotides from the biological sample, a labeled polynucleotide of the invention that is complementary to one or more of the polynucleotides, will be allowed to hybridize to polynucleotides in the biological sample using techniques of nucleic acid hybridization known to those of ordinary skill in the art. For example, such hybridization may be carried out in solution or with one hybridization partner on a solid support.

### 5.7. THERAPEUTIC AND PROPHYLACTIC USES OF THE FUSION PROTEIN

Purified or partially purified fusion proteins or fragments thereof may be formulated as a vaccine or therapeutic composition. Such composition may include adjuvants to enhance immune responses. In addition, such proteins may be further suspended in an oil emulsion to cause a slower release of the proteins *in vivo* upon injection. The optimal ratios of each component in the formulation may be determined by techniques well known to those skilled in the art.

Any of a variety of adjuvants may be employed in the vaccines of this invention to enhance the immune response. Most adjuvants contain a substance designed to protect the antigen from rapid catabolism, such as aluminum hydroxide or mineral oil, and a nonspecific stimulator of immune responses, such as lipid A, *Bortadella pertussis* or *Mycobacterium tuberculosis.* Suitable adjuvants are commercially available and include, for example, Freund's Incomplete Adjuvant and Freund's Complete Adjuvant (Difco Laboratories) and Merck Adjuvant 65 (Merck and Company, Inc., Rahway, NJ). Other suitable adjuvants include alum, biodegradable microspheres, monophosphoryl lipid A, quil A, SBAS1c SBAS2 (Ling et al., 1997, Vaccine 15:1562-1567), SBAS7 and Al(OH)₃.

In the vaccines of the present invention, it is preferred that the adjuvant induces an immune response comprising Th1 aspects. Suitable adjuvant systems include, for example, a combination of monophosphoryl lipid A, preferably 3-de-O-acylated monophosphoryl lipid A (3D-MLP) together with an aluminum salt. An enhanced system involves the combination of a monophosphoryl lipid A and a saponin derivative, particularly the combination of 3D-MLP and the saponin QS21 as disclosed in WO 94/00153, or a less reactogenic composition where the QS21 is quenched with cholesterol as disclosed in WO 96/33739. Previous experiments have demonstrated a clear synergistic effect of combinations of 3D-MLP and QS21 in the induction of both humoral and Th1 type cellular immune responses. A particularly potent adjuvant formation involving QS21, 3D-MLP and tocopherol in an oil-in-water emulsion is described in WO 95/17210 and is a preferred formulation.

Formulations containing an antigen of the present invention may be administered to a subject *per se* or in the form of a pharmaceutical or therapeutic composition. Pharmaceutical compositions comprising the proteins may be manufactured by means of conventional mixing, dissolving, granulating, dragee-making, levigating, emulsifying, encapsulating, entrapping or lyophilizing processes. Pharmaceutical compositions may be formulated in conventional manner using one or more physiologically acceptable carriers, diluents, excipients or auxiliaries which facilitate processing of the polypeptides into preparations which can be used pharmaceutically. Proper formulation is dependent upon the route of administration chosen.

For topical administration, the proteins may be formulated as solutions, gels, ointments, creams, suspensions, etc. as are well-known in the art.

Systemic formulations include those designed for administration by injection, e.g. subcutaneous, intravenous, intramuscular, intrathecal or intraperitoneal injection, as well as those designed for transdermal, transmucosal, oral or pulmonary administration.

For injection, the proteins may be formulated in aqueous solutions, preferably in physiologically compatible buffers such as Hanks's solution, Ringer's solution, or physiological saline buffer. The solution may contain formulatory agents such as suspending, stabilizing and/or dispersing agents. Alternatively, the proteins may be in powder form for constitution with a suitable vehicle, *e.g.*, sterile pyrogen-free water, before use.

For transmucosal administration, penetrants appropriate to the barrier to be permeated are used in the formulation. Such penetrants are generally known in the art.

For oral administration, a composition can be readily formulated by combining the proteins with pharmaceutically acceptable carriers well known in the art. Such carriers enable the proteins to be formulated as tablets, pills, dragees, capsules, liquids, gels, syrups, slurries, suspensions and the like, for oral ingestion by a subject to be treated. For oral solid formulations such as, for example, powders, capsules and tablets, suitable excipients include fillers such as sugars, such as lactose, sucrose, mannitol and sorbitol; cellulose preparations such as maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP); granulating agents; and binding agents. If desired, disintegrating agents may be added, such as the cross-linked polyvinylpyrrolidone, agar, or alginic acid or a salt thereof such as sodium alginate.

If desired, solid dosage forms may be sugar-coated or enteric-coated using standard techniques.

For oral liquid preparations such as, for example, suspensions, elixirs and solutions, suitable carriers, excipients or diluents include water, glycols, oils, alcohols, etc. Additionally, flavoring agents, preservatives, coloring agents and the like may be added.

For buccal administration, the proteins may take the form of tablets, lozenges, etc. formulated in conventional manner.

For administration by inhalation, the proteins for use according to the present invention are conveniently delivered in the form of an aerosol spray from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g.,* gelatin for use in an inhaler or insufflator may be formulated containing a powder mix of the proteins and a suitable powder base such as lactose or starch.

The proteins may also be formulated in rectal or vaginal compositions such as suppositories or retention enemas, *e.g.,* containing conventional suppository bases such as cocoa butter or other glycerides.

In addition to the formulations described previously, the proteins may also be formulated as a depot preparation. Such long acting formulations may be administered by implantation (for example subcutaneously or intramuscularly) or by intramuscular injection. Thus, for example, the proteins may be formulated with suitable polymeric or hydrophobic materials (for example as an emulsion in an acceptable oil) or ion exchange resins, or as sparingly soluble derivatives, for example, as a sparingly soluble salt.

Alternatively, other pharmaceutical delivery systems may be employed. Liposomes and emulsions are well known examples of delivery vehicles that may be used to deliver an antigen. Certain organic solvents such as dimethylsulfoxide also may be employed, although usually at the cost of greater toxicity. The fusion proteins may also be encapsulated in microspheres (United States Patent Nos. 5,407,609; 5,853,763; 5,814,344 and 5,820,883). Additionally, the proteins may be delivered using a sustained-release system, such as semipermeable matrices of solid polymers containing the therapeutic or vaccinating agent. Various sustained-release materials have been established and are well known by those skilled in the art. Sustained-release capsules may, depending on their chemical nature, release the proteins for a few weeks up to over 100 days. Depending on the chemical nature and the biological stability of the reagent, additional strategies for protein stabilization may be employed.

Determination of an effective amount of the fusion protein for inducing an immune response in a subject is well within the capabilities of those skilled in the art, especially in light of the detailed disclosure provided herein.

An effective dose can be estimated initially from *in vitro* assays. For example, a dose can be formulated in animal models to achieve an induction of an immune response using techniques that are well known in the art. One having ordinary skill in the art could readily optimize administration to humans based on animal data. Dosage amount and interval may be adjusted individually. For example, when used as a vaccine, the polypeptides and/or polynucleotides of the invention may be administered in about 1 to 3 doses for a 1-36 week period. Preferably, 3 doses are administered, at intervals of about 3-4 months, and booster vaccinations may be given periodically thereafter. Alternate protocols may be appropriate for individual patients. A suitable dose is an amount of polypeptide or DNA that, when administered as described above, is capable of raising an immune response in an immunized patient sufficient to protect the patient from *M. tuberculosis* infection for at least 1-2 years. In general, the amount of polypeptide present in a dose (or produced *in situ* by the DNA in a dose) ranges from about 1 pg to about 100 mg per kg of host, typically from about 10 pg to about 1 mg, and preferably from about 100 pg to about 1 µg. Suitable dose range will vary with the size of the patient, but will typically range from about 0.1 mL to about 5 mL.

### 5.8 DIAGNOSTIC USES OF THE FUSION PROTEIN

The fusion polypeptides of the invention are useful in the diagnosis of tuberculosis infection *in vitro* and *in vivo.* The ability of a polypeptide of the invention to induce cell proliferation or cytokine production can be assayed by the methods disclosed in Section 5.2, *supra.*

In another aspect, this invention provides methods for using one or more of the fusion polypeptides to diagnose tuberculosis using a skin test *in vivo.* As used herein, a skin test is any assay performed directly on a patient in which a delayed-type hypersensitivity (DTH) reaction (such as swelling, reddening or dermatitis) is measured following intradermal injection of one or more polypeptides as described above. Such injection may be achieved using any suitable device sufficient to contact the polypeptide with dermal cells of the patient, such as, for example, a tuberculin syringe or 1 mL syringe. Preferably, the reaction is measured at least about 48 hours after injection, more preferably about 48 to about 72 hours after injection.

The DTH reaction is a cell-mediated immune response, which is greater in patients that have been exposed previously to the test antigen (*i.e*., the immunogenic portion of the polypeptide employed, or a variant thereof). The response may be measured visually, using a ruler. In general, a response that is greater than about 0.5 cm in diameter, preferably greater than about 1.0 cm in diameter, is a positive response, indicative of tuberculosis infection, which may or may not be manifested as an active disease.

The fusion polypeptides of this invention are preferably formulated, for use in a skin test, as pharmaceutical compositions containing a polypeptide and a physiologically acceptable carrier. Such compositions typically contain one or more of the above polypeptides in an amount ranging from about 1 µg to about 100 µg, preferably from about 10 µg to about 50 µg in a volume of 0.1 mL. Preferably, the carrier employed in such pharmaceutical compositions is a saline solution with appropriate preservatives, such as phenol and/or Tween 80™.

In another aspect, the present invention provides methods for using the polypeptides to diagnose tuberculosis. In this aspect. methods are provided for detecting *M. tuberculosis* infection in a biological sample using the fusion polypeptides alone or in combination. As used herein, a "biological sample" is any antibody-containing sample obtained from a patient. Preferably, the sample is whole blood, sputum, serum, plasma, saliva cerebrospinal fluid or urine. More preferably, the sample is a blood, serum or plasma sample obtained from a patient or a blood supply. The polypeptide(s) are used in an assay, as described below, to determine the presence or absence of antibodies to the polypeptide(s) in the sample relative to a predetermined cut-off value. The presence of such antibodies indicates previous sensitization to mycobacterial antigens which may be indicative of tuberculosis.

In embodiments in which more than one fusion polypeptide is employed, the polypeptides used are preferably complementary (*i.e*., one component polypeptide will tend to detect infection in samples where the infection would not be detected by another component polypeptide). Complementary polypeptides may generally be identified by using each polypeptide individually to evaluate serum samples obtained from a series of patients known to be infected with *M. tuberculosis.* After determining which samples test positive (as described below) with each polypeptide, combinations of two or more fusion polypeptides may be formulated that are capable of detecting infection in most, or all, of the samples tested. Such polypeptides are complementary. Approximately 25-30% of sera from tuberculosis-infected individuals are negative for antibodies to any single protein. Complementary polypeptides may, therefore, be used in combination to improve sensitivity of a diagnostic test.

There are a variety of assay formats known to those of ordinary skill in the art for using one or more polypeptides to detect antibodies in a sample. *See, e.g.,* Harlow and Lane, Antibodies: A Laboratory Manual, Cold Spring Harbor Laboratory, 1988, which is incorporated herein by reference. In a preferred embodiment, the assay involves the use of polypeptide immobilized on a solid support to bind to and remove the antibody from the sample. The bound antibody may then be detected using a detection reagent that contains a reporter group. Suitable detection reagents include antibodies that bind to the antibody/polypeptide complex and free polypeptide labeled with a reporter group (*e.g*., in a semi-competitive assay). Alternatively, a competitive assay may be utilized, in which an antibody that binds to the polypeptide is labeled with a reporter group and allowed to bind to the immobilized antigen after incubation of the antigen with the sample. The extent to which components of the sample inhibit the binding of the labeled antibody to the polypeptide is indicative of the reactivity of the sample with the immobilized polypeptide.

The solid support may be any solid material known to those of ordinary skill in the art to which the antigen may be attached. For example. the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example. in U.S. Patent No. 5,359,681.

The polypeptides may be bound to the solid support using a variety of techniques known to those of ordinary skill in the art. In the context of the present invention, the term "bound" refers to both noncovalent association, such as adsorption, and covalent attachment (which may be a direct linkage between the antigen and functional groups on the support or may be a linkage by way of a cross-linking agent). Binding by adsorption to a well in a microtiter plate or to a membrane is preferred. *In* such cases. adsorption may be achieved by contacting the polypeptide, in a suitable buffer, with the solid support for a suitable amount of time. The contact time varies with temperature, but is typically between about 1 hour and 1 day. In general, contacting a well of a plastic microtiter plate (such as polystyrene or polyvinylchloride) with an amount of polypeptide ranging from about 10 ng to about 1 µg, and preferably about 100 ng, is sufficient to bind an adequate amount of antigen.

Covalent attachment of polypeptide to a solid support may generally be achieved by first reacting the support with a bifunctional reagent that will react with both the support and a functional group, such as a hydroxyl or amino group, on the polypeptide. For example, the polypeptide may be bound to supports having an appropriate polymer coating using benzoquinone or by condensation of an aldehyde group on the support with an amine and an active hydrogen on the polypeptide (*see, e.g.,* Pierce Immunotechnology Catalog and Handbook. 1991, at A12-A13).

In certain embodiments. the assay is an enzyme linked immunosorbent 1assay (ELISA). This assay may be performed by first contacting a fusion polypeptide antigen that has been immobilized on a solid support, commonly the well of a microtiter plate, with the sample, such that antibodies to the polypeptide within the sample are allowed to bind to the immobilized polypeptide. Unbound sample is then removed from the immobilized polypeptide and a detection reagent capable of binding to the immobilized antibody-polypeptide complex is added. The amount of detection reagent that remains bound to the solid support is then determined using a method appropriate for the specific detection reagent.

More specifically, once the polypeptide is immobilized on the support as described above, the remaining protein binding sites on the support are typically blocked. Any suitable blocking agent known to those of ordinary skill in the art, such as bovine serum albumin or Tween 24™ (Sigma Chemical Co., St. Louis, MO) may be employed. The immobilized polypeptide is then incubated with the sample, and antibody is allowed to bind to the antigen. The sample may be diluted with a suitable diluent, such as phosphate-buffered saline (PBS) prior to incubation. In general, an appropriate contact time is that period of time that is sufficient to detect the presence of antibody within a *M*. *tuberculosis-*infected sample. Preferably, the contact time is sufficient to achieve a level of binding that is at least *95*% of that achieved at equilibrium between bound and unbound antibody. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1 % Tween 20™. Detection reagent may then be added to the solid support. An appropriate detection reagent is any compound that binds to the immobilized antibody-polypeptide complex and that can be detected by any of a variety of means known to those in the art. Preferably, the detection reagent contains a binding agent (for example, Protein A, Protein G, lectin or free antigen) conjugated to a reporter group. Preferred reporter groups include enzymes (such as horseradish peroxidase), substrates, cofactors, inhibitors, dyes, radionuclides, luminescent groups, fluorescent groups, biotin and colloidal particles, such as colloidal gold and selenium. The conjugation of binding agent to reporter group may be achieved using standard methods known to those of ordinary skill in the art. Common binding agents may also be purchased conjugated to a variety of reporter groups from many commercial sources (*e.g*.. Zymed Laboratories, San Francisco, CA, and Pierce, Rockford. IL).

The detection reagent is then incubated with the immobilized antibody-polypeptide complex for an amount of time sufficient to detect the bound antibody. An appropriate amount of time may generally be determined from the manufacturer's instructions or by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time). followed by spectroscopic or other analysis of the reaction products.

To determine the presence or absence of anti -*M. tuberculosis* antibodies in the sample, the signal detected from the reporter group that remains bound to the solid support is generally compared to a signal that corresponds to a predetermined cut-off value. In one preferred embodiment, the cut-off value is the average mean signal obtained when the immobilized antigen is incubated with samples from an uninfected patient. In general, a sample generating a signal that is three standard deviations above the predetermined cut-off value is considered positive for tuberculosis. In an alternate preferred embodiment, the cut-off value is determined using a Receiver Operator Curve. according to the method of Sackett et al., 1985, Clinical Epidemiology: A Basic Science for Clinical Medicine, Little Brown and Co., pp. 106-107. Briefly, in this embodiment, the cut-off value may be determined from a plot of pairs of true positive rates (*i.e.*, sensitivity) and false positive rates (100%-specificity) that correspond to each possible cut-off value for the diagnostic test result. The cut-off value on the plot that is the closest to the upper left-hand corner (*i.e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for tuberculosis.

In a related embodiment, the assay is performed in a rapid flow-through or strip test format, wherein the antigen is immobilized on a membrane, such as nitrocellulose. In the flow-through test, antibodies within the sample bind to the immobilized polypeptide as the sample passes through the membrane. A detection reagent (*e.g*., protein A-colloidal gold) then binds to the antibody-polypeptide complex as the solution containing the detection reagent flows through the membrane. The detection of bound detection reagent may then be performed as described above. In the strip test format, one end of the membrane to which polypeptide is bound is immersed in a solution containing the sample. The sample migrates along the membrane through a region containing detection reagent and to the area of immobilized polypeptide. Concentration of detection reagent at the polypeptide indicates the presence of anti- *M. tuberculosis* antibodies in the sample. Typically, the concentration of detection reagent at that site generates a pattern, such as a line, that can be read visually. The absence of such a pattern indicates a negative result. In general, the amount of polypeptide immobilized on the membrane is selected to generate a visually discernible pattern when the biological sample contains a level of antibodies that would be sufficient to generate a positive signal in an ELISA, as discussed above. Preferably, the amount of polypeptide immobilized on the membrane ranges from about 5 ng to about 1 µg, and more preferably from about 50 ng to about 500 ng. Such tests can typically be performed with a very small amount (*e.g*., one drop) of patient serum or blood.

The invention having been described, the following examples are offered by way of illustration and not limitation.

### 6. EXAMPLE: FUSION PROTEINS OF M. TUBERCULOSIS ANTIGENS RETAIN IMMUNOGENICITY OF THE INDIVIDUAL COMPONENTS

### 6.1. MATERIALS AND METHODS

### 6.1.1. CONSTRUCTION OF FUSION PROTEINS

Coding sequences of *M. tuberculosis* antigens were modified by PCR in order to facilitate their fusion and subsequent expression of fusion protein. DNA amplification was performed using 10 µl 10X Pfu buffer, 2 µl 10 mM dNTPs, 2 µl each of the PCR primers at 10 µM concentration, 81.5 µl water, 1.5 µl Pfu DNA polymerase (Stratagene, La Jolla, CA) and 1 µl DNA at either 70 ng/µl (for TbRa3 antigen) or 50 ng/µl (for 38 kD and Tb38-1 antigens). For TbRa3 antigen, denaturation at 94°C was performed for 2 min, followed by 40 cycles of 96°C for 15 sec and 72°C for 1 min, and lastly by 72°C for 4 min. For 38 kD antigen, denaturation at 96°C was performed for 2 min, followed by 40 cycles of 96°C for 30 sec, 68°C for 15 sec and 72°C for 3 min, and finally by 72°C for 4 min. For Tb38-1 antigen, denaturation at 94°C for 2 min was followed by 10 cycles of 96°C for 15 sec, 68°C for 15 sec and 72°C for 1.5 min, 30 cycles of 96°C for 15 sec, 64°C for 15 sec and 72°C for 1.5, and finally by 72°C for 4 min.

Following digestion with a restriction endonuclease to yield the desired cohesive or blunt ends, a polynucleotide specific for each fusion polypeptide was ligated into an expression plasmid. Each resulting plasmid contained the coding sequences of the individual antigens of each fusion polypeptide. The expression vectors used were pET-12b and pT7^L2 IL 1.

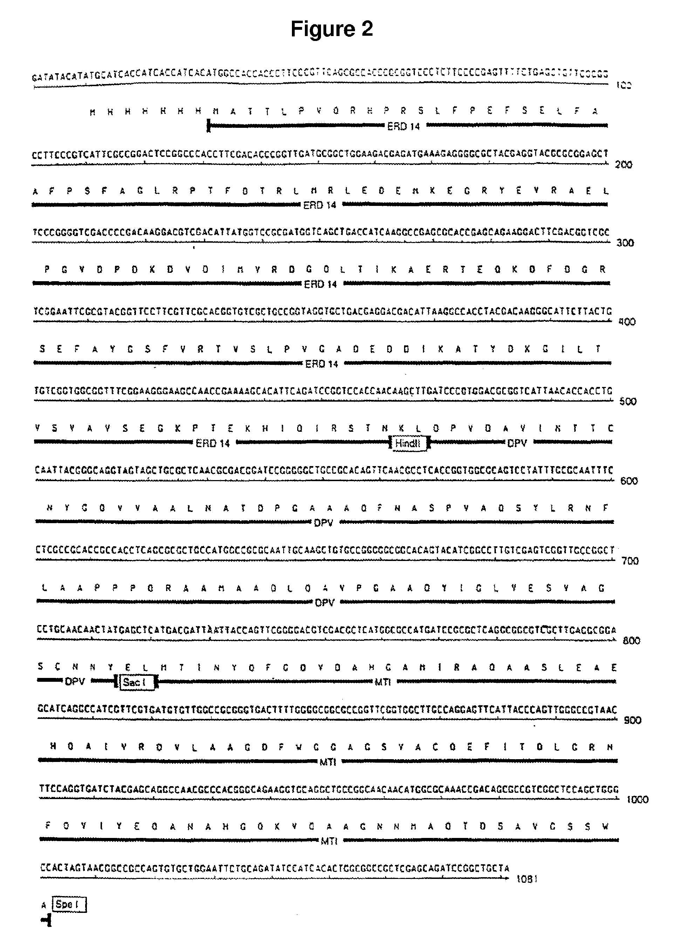

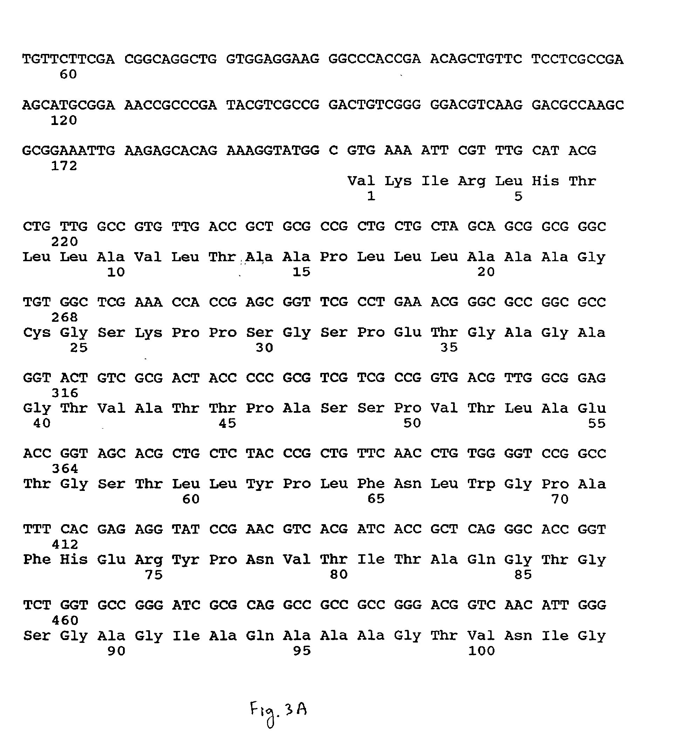

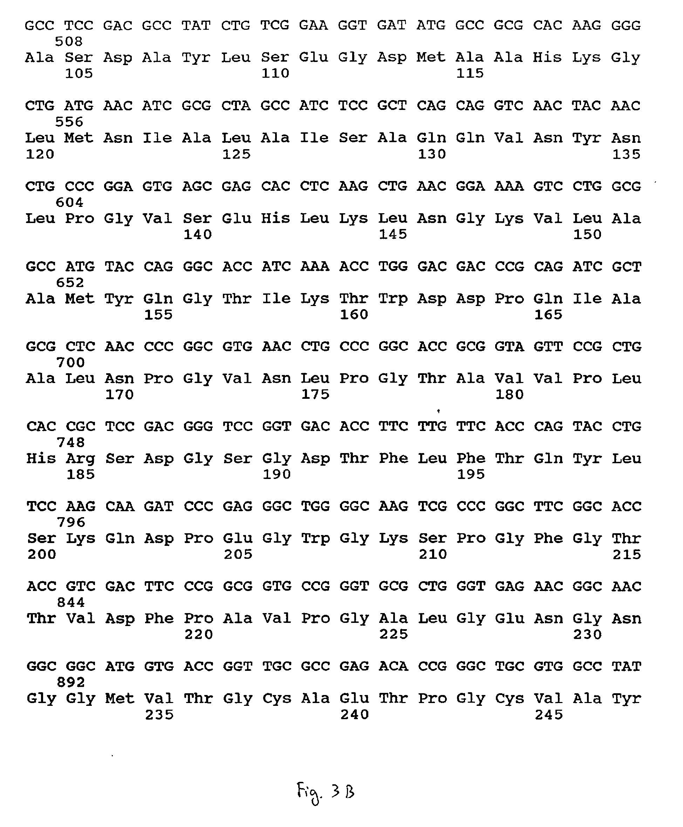

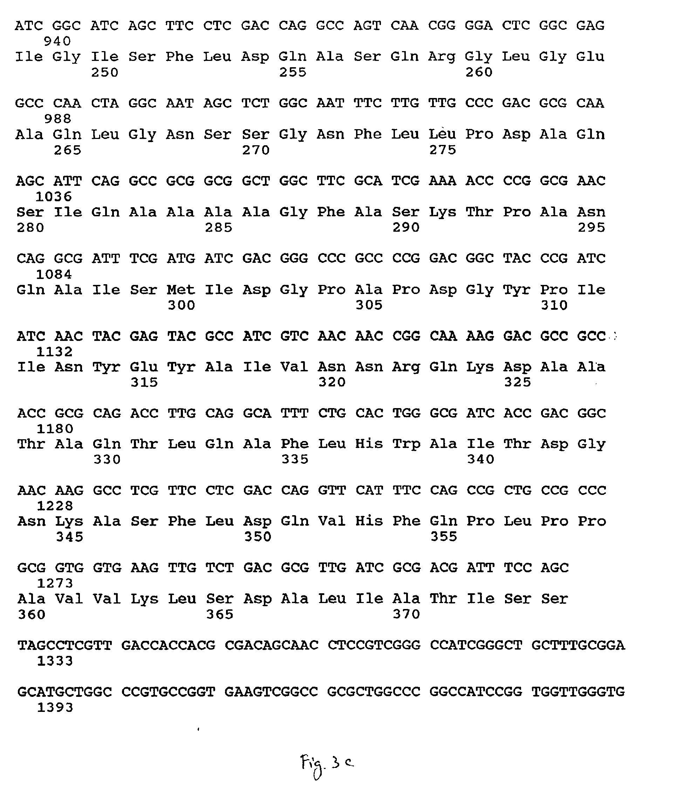

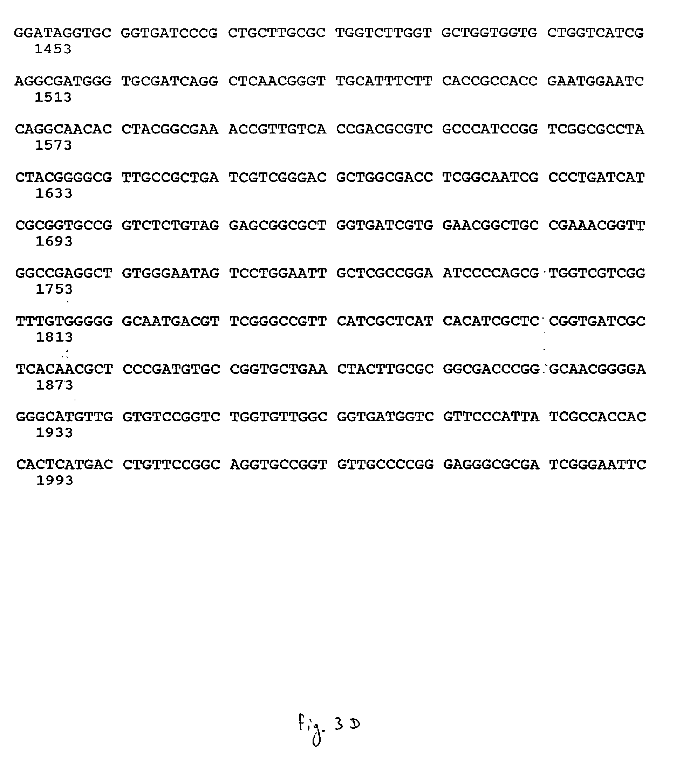

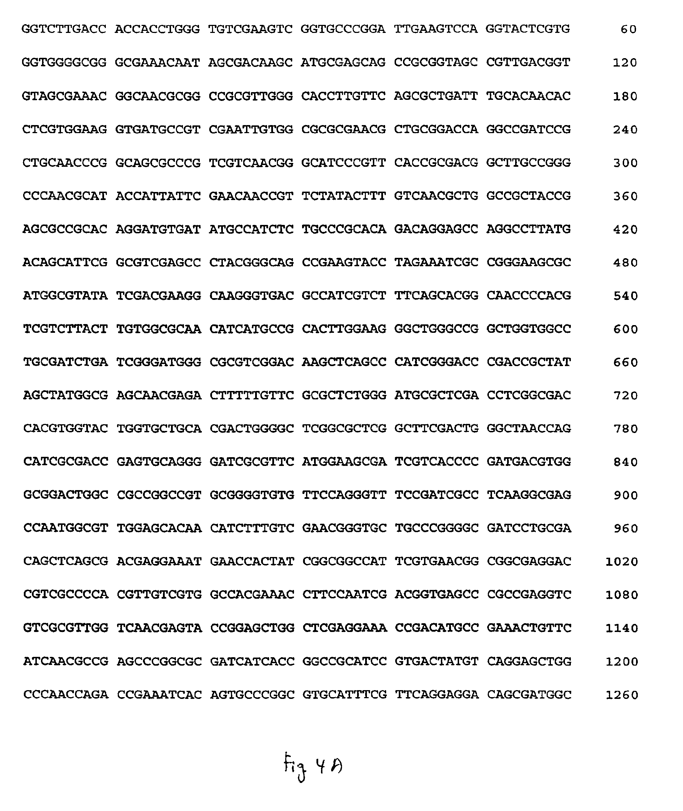

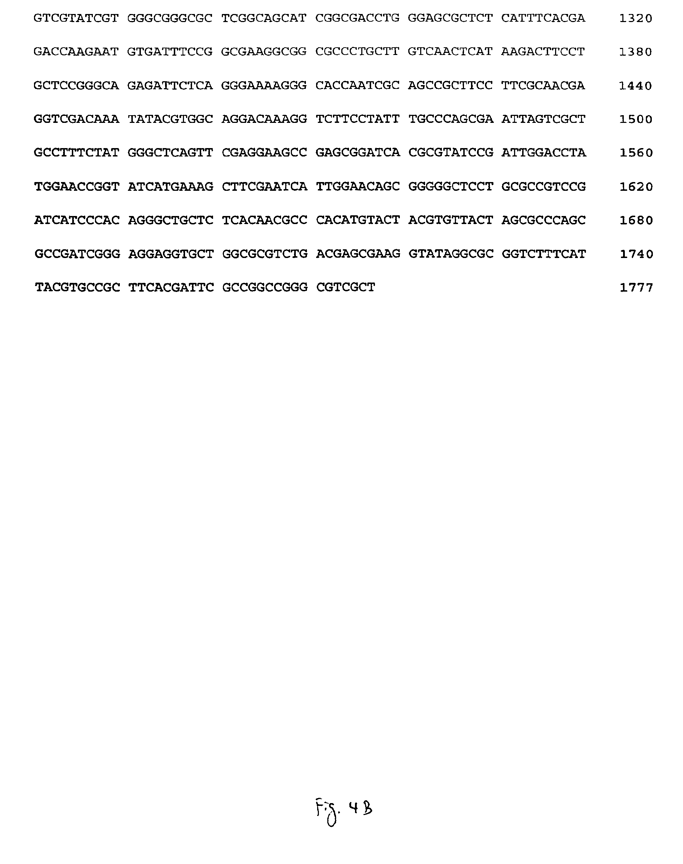

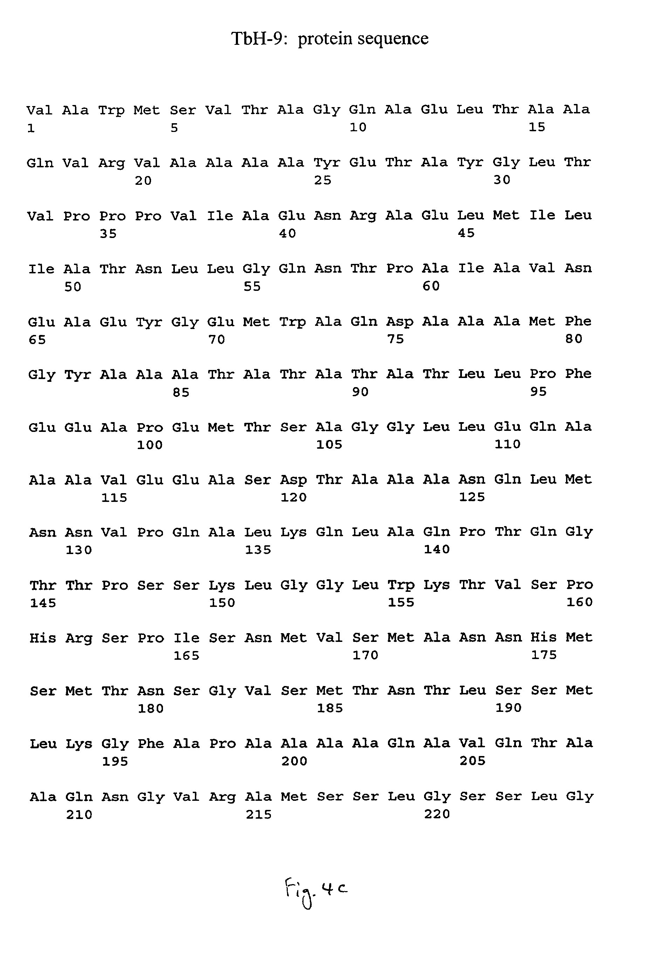

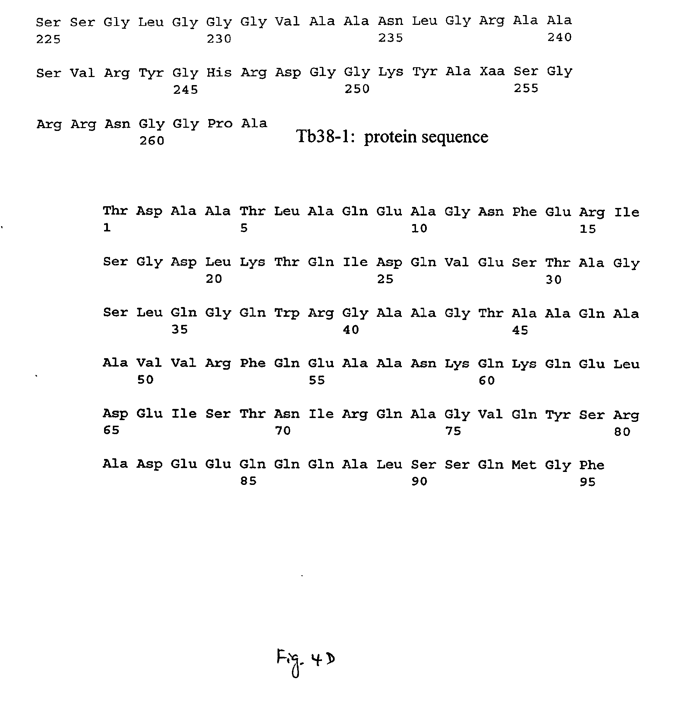

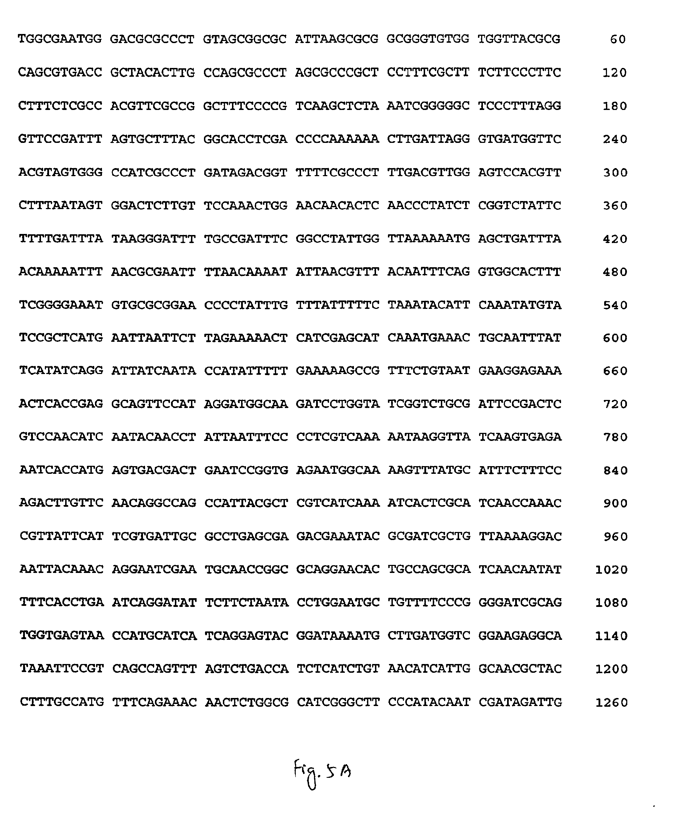

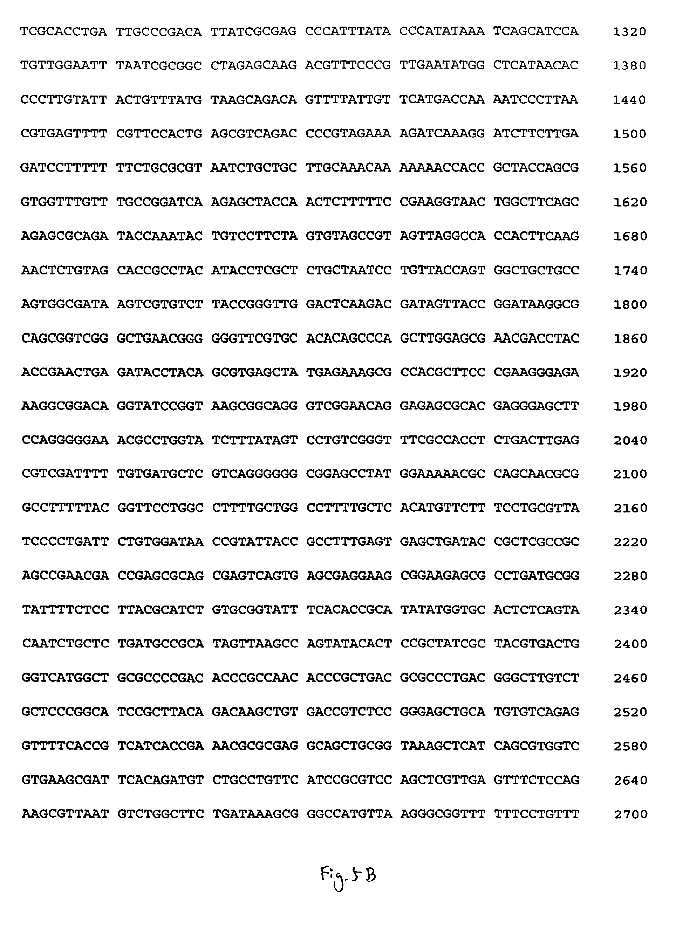

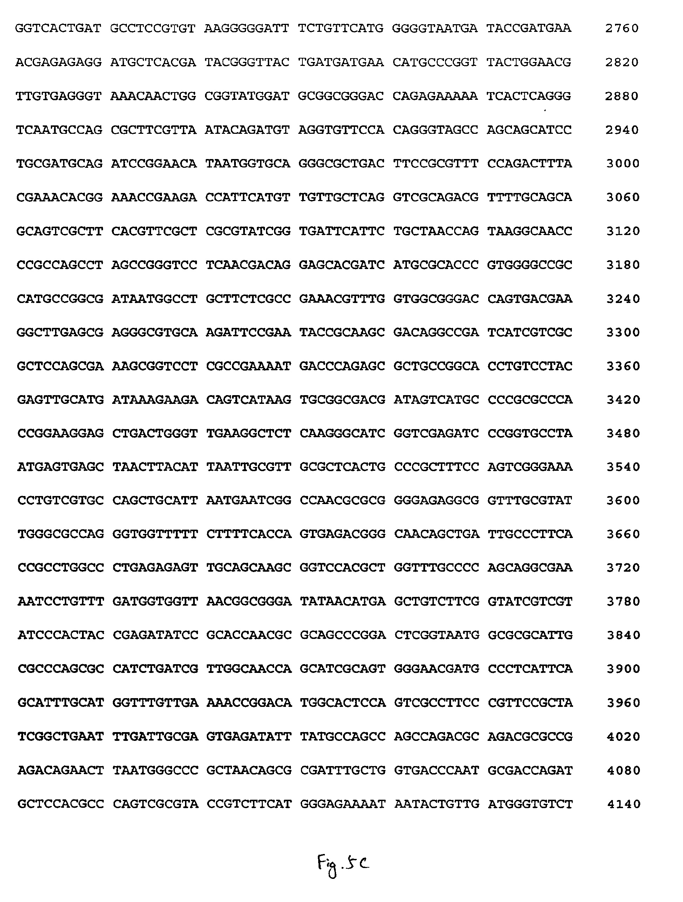

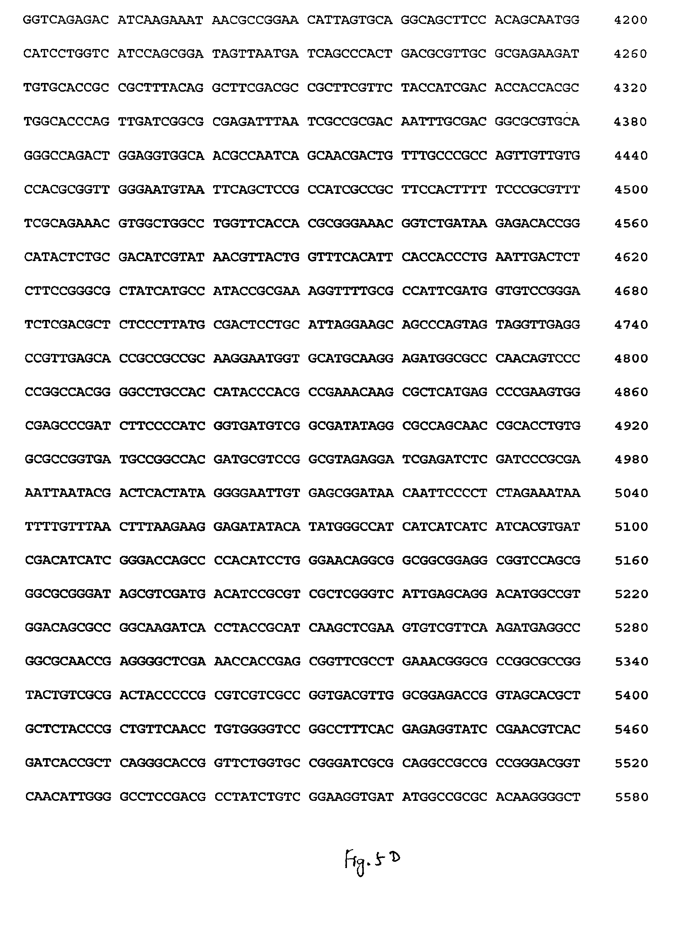

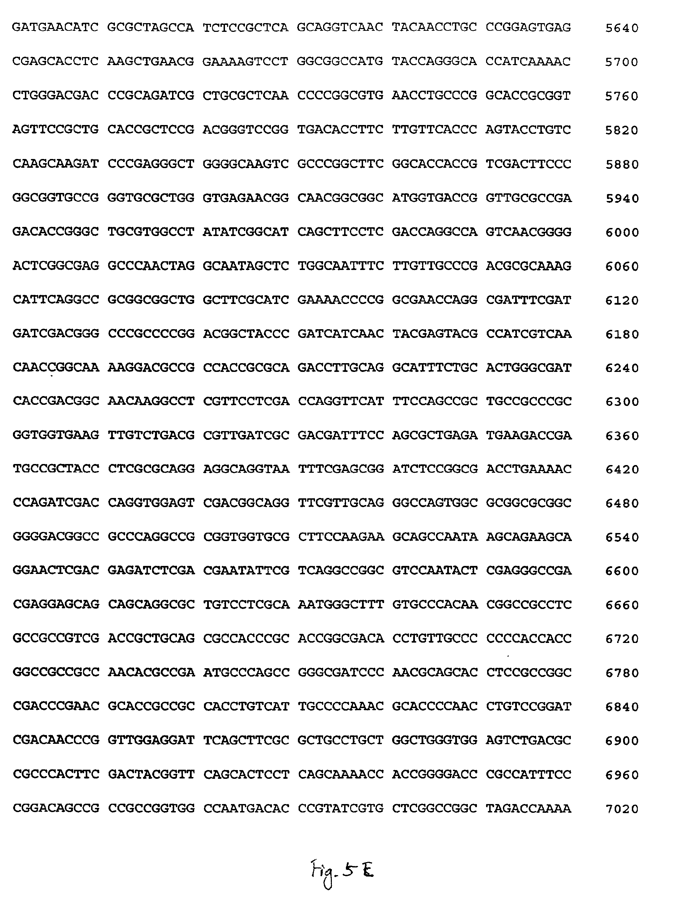

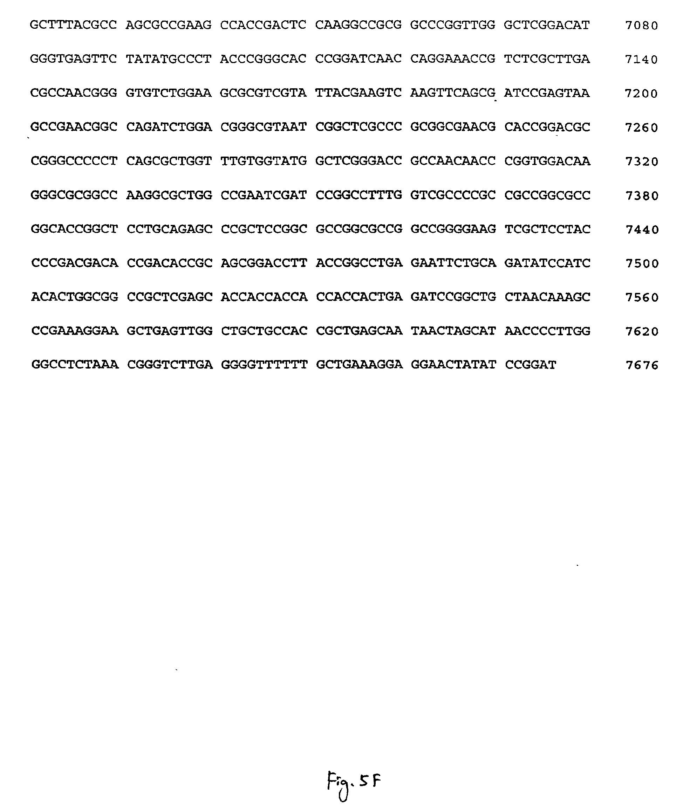

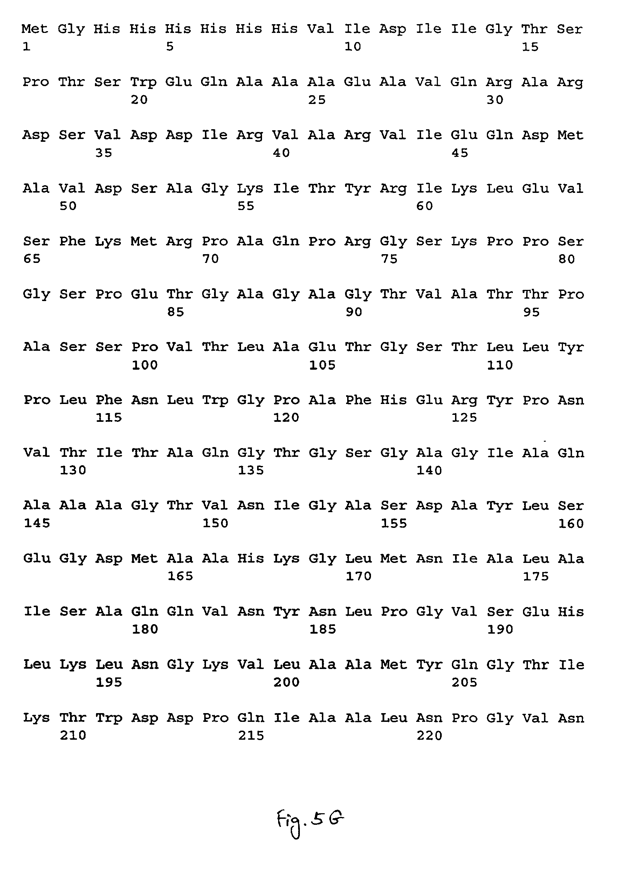

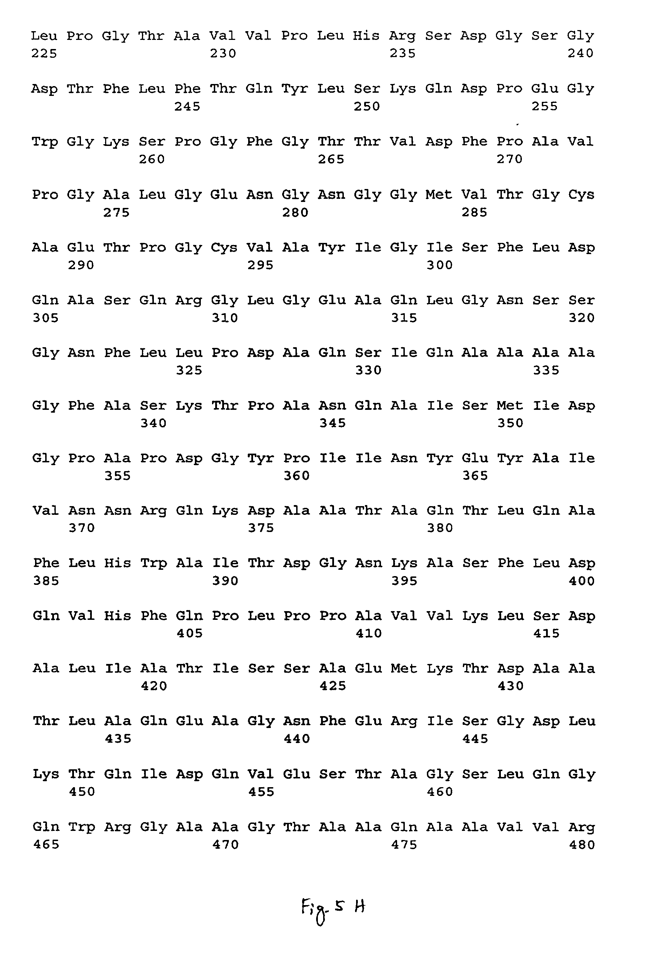

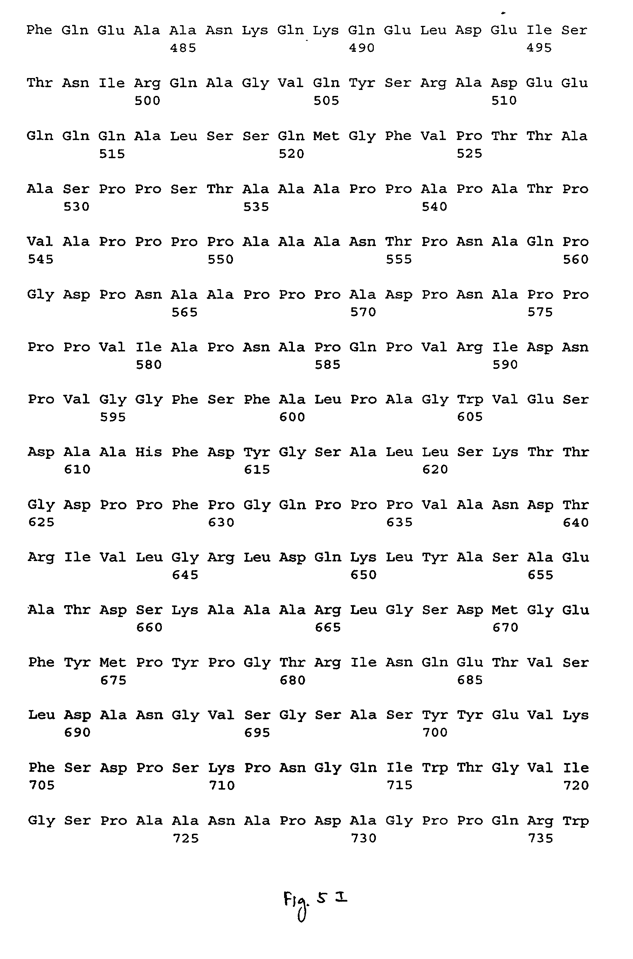

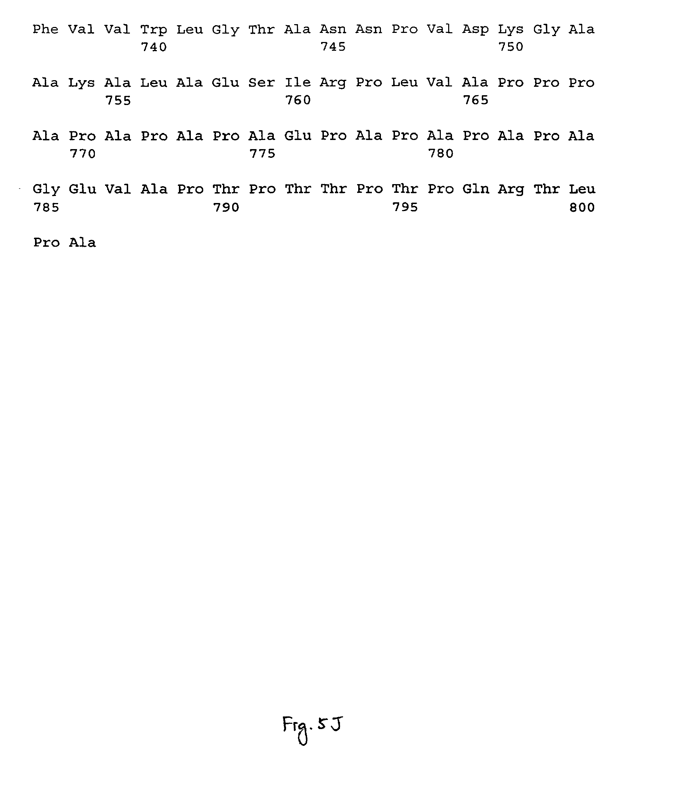

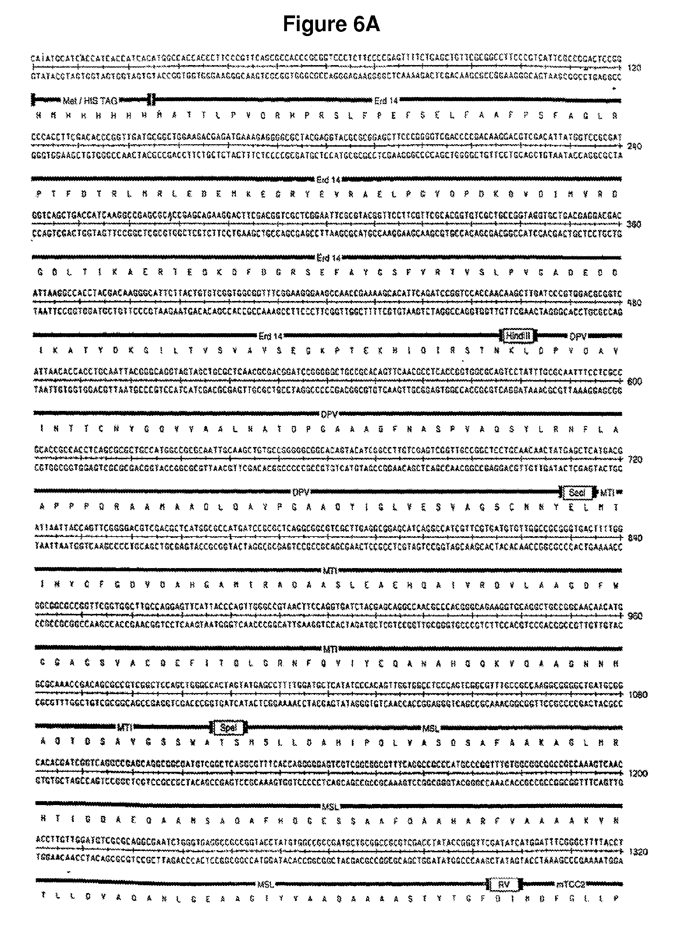

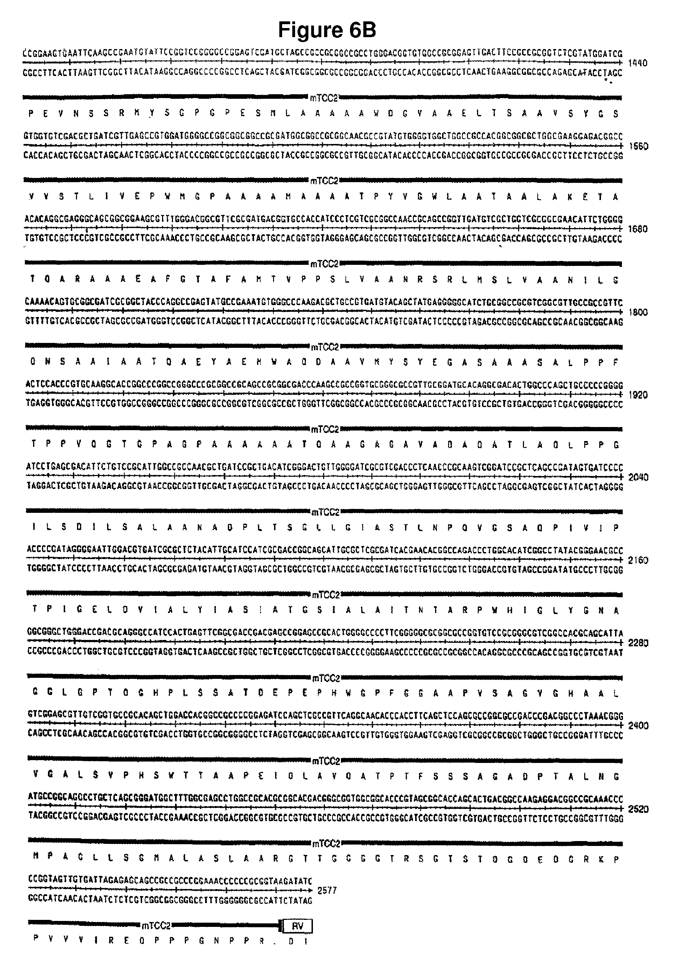

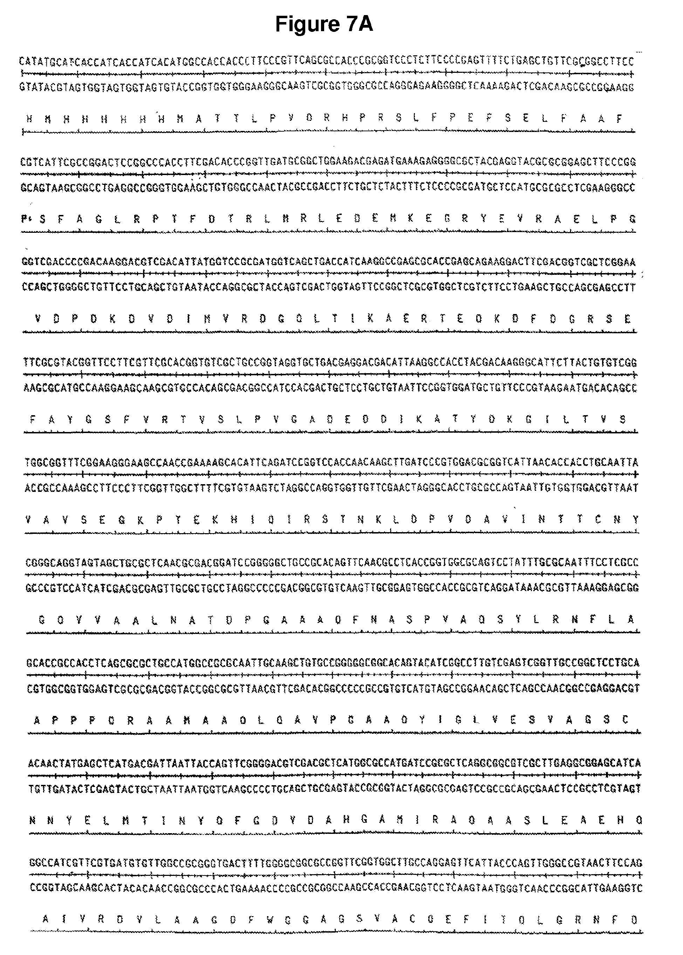

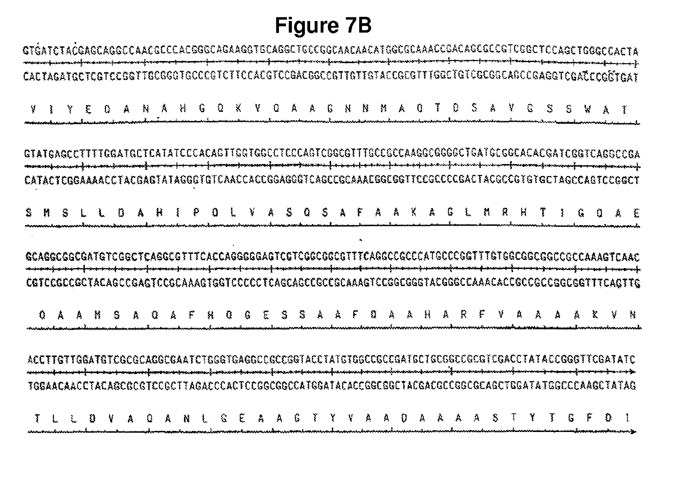

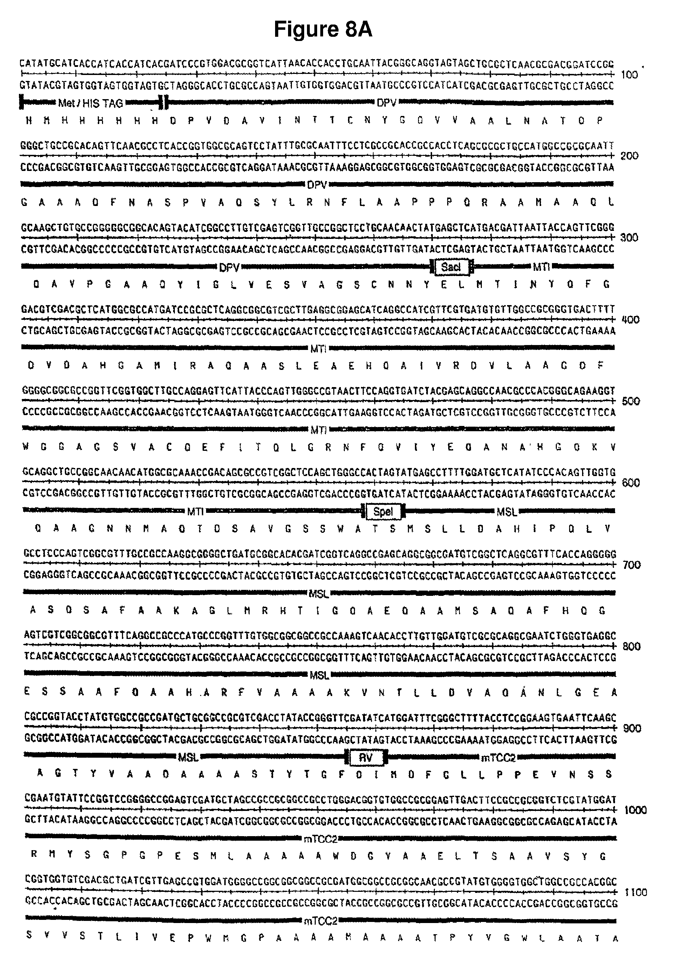

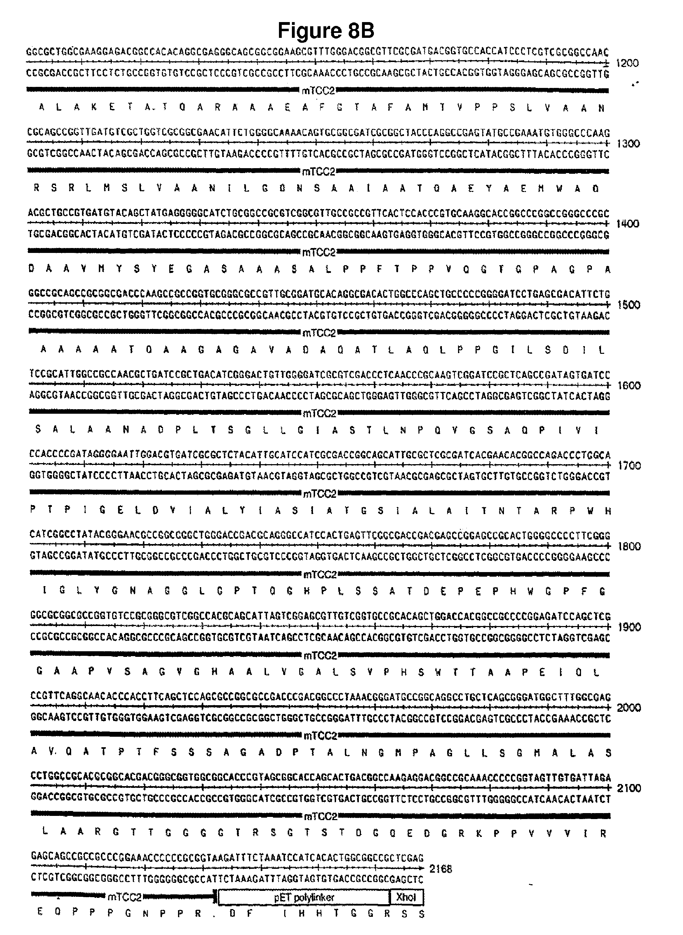

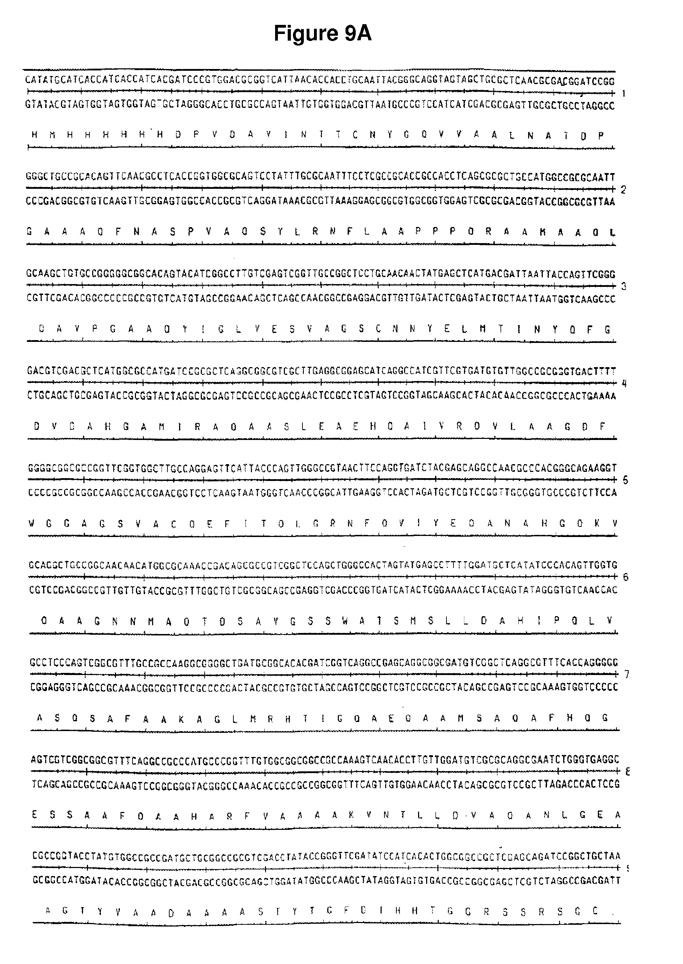

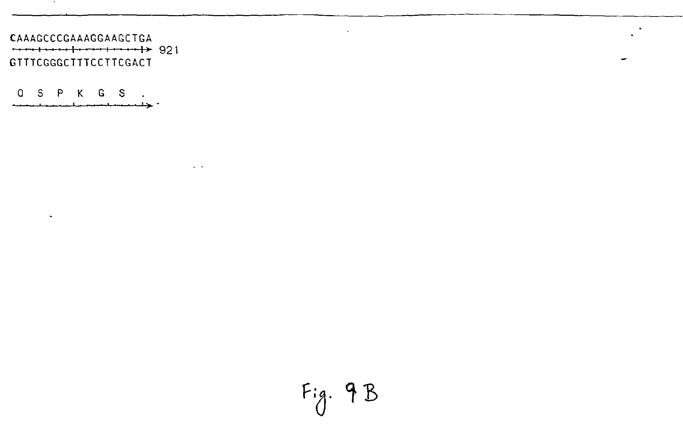

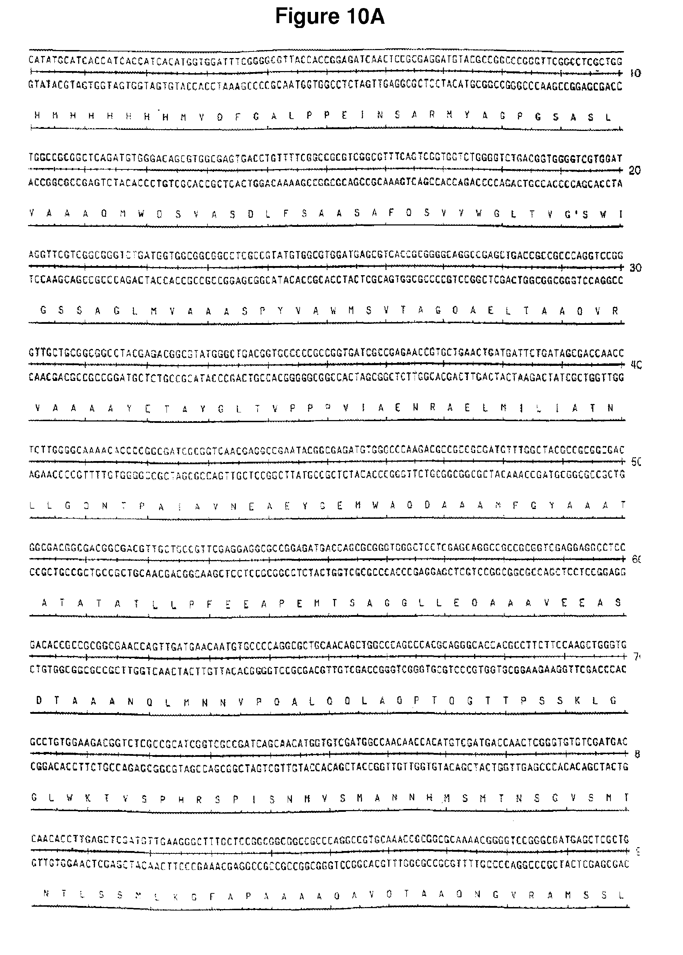

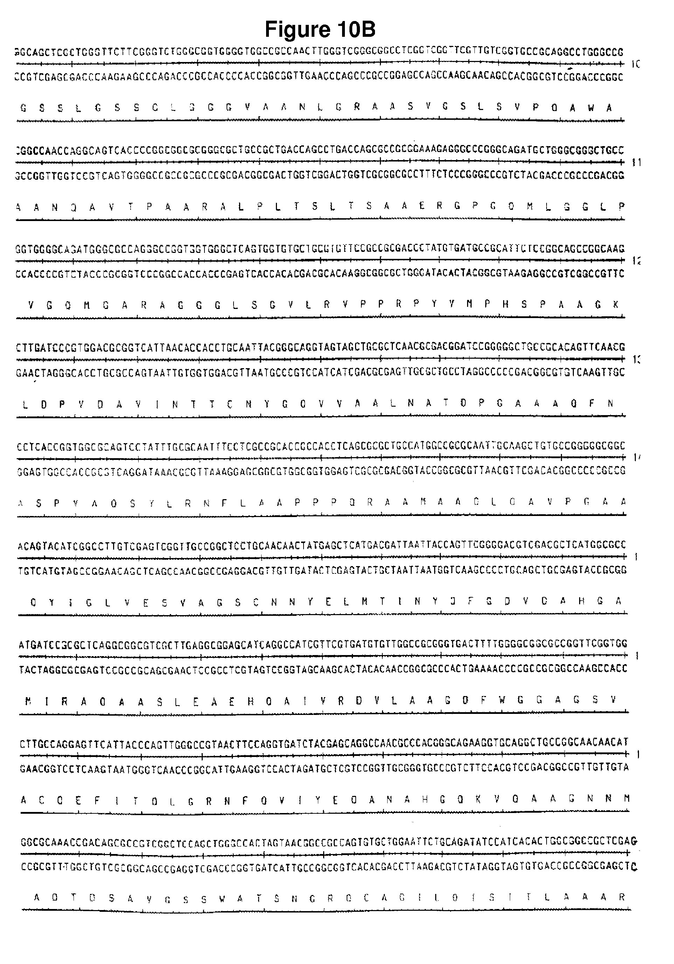

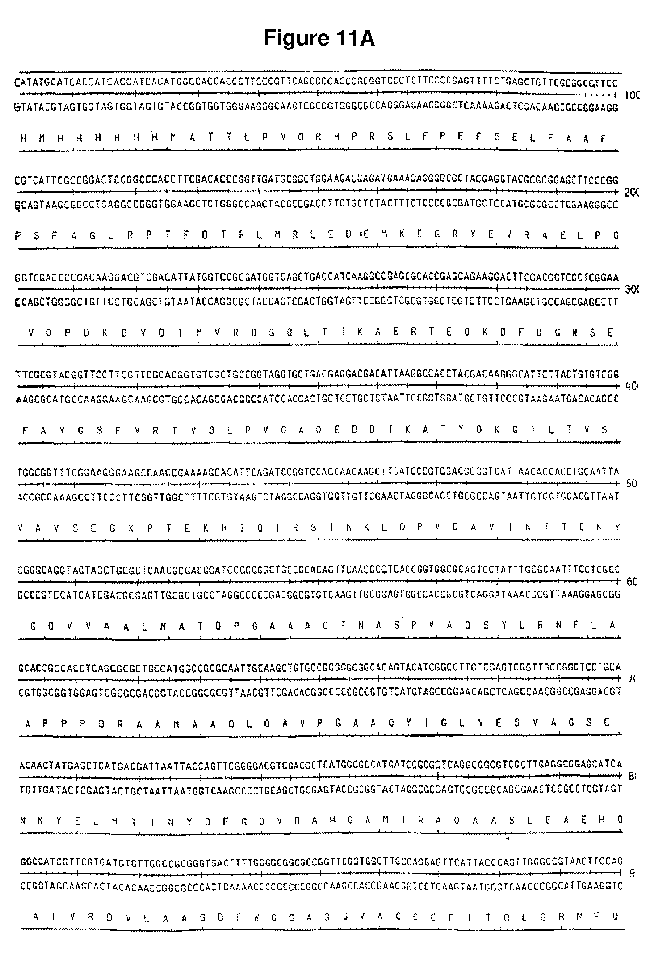

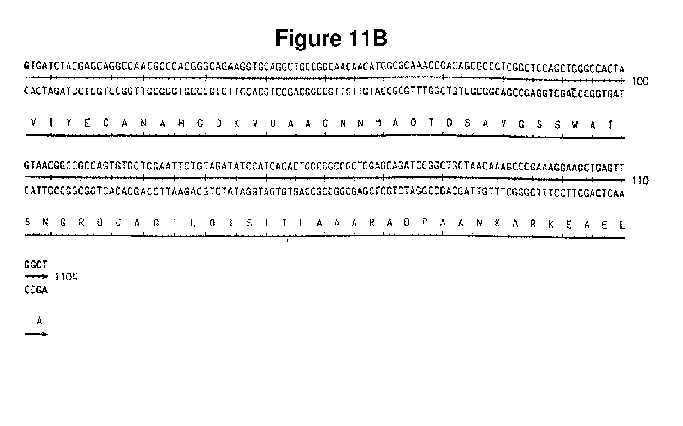

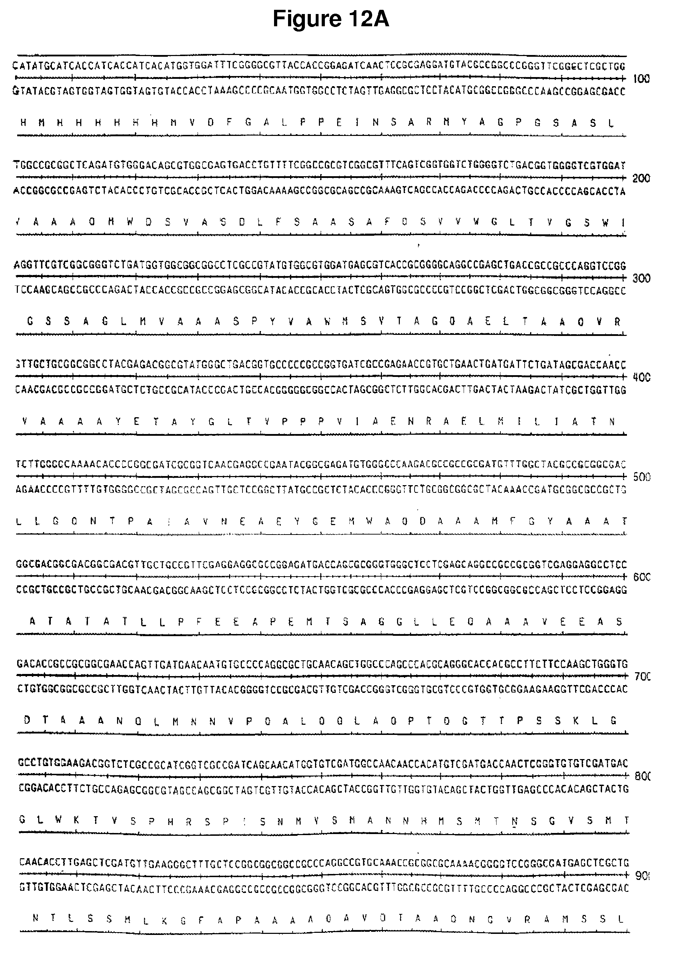

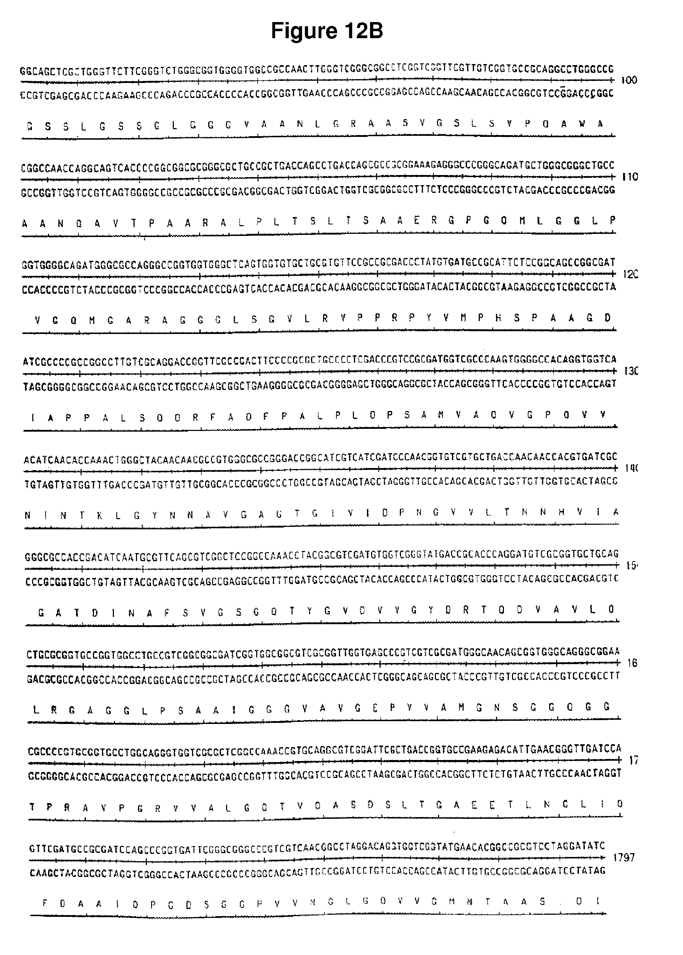

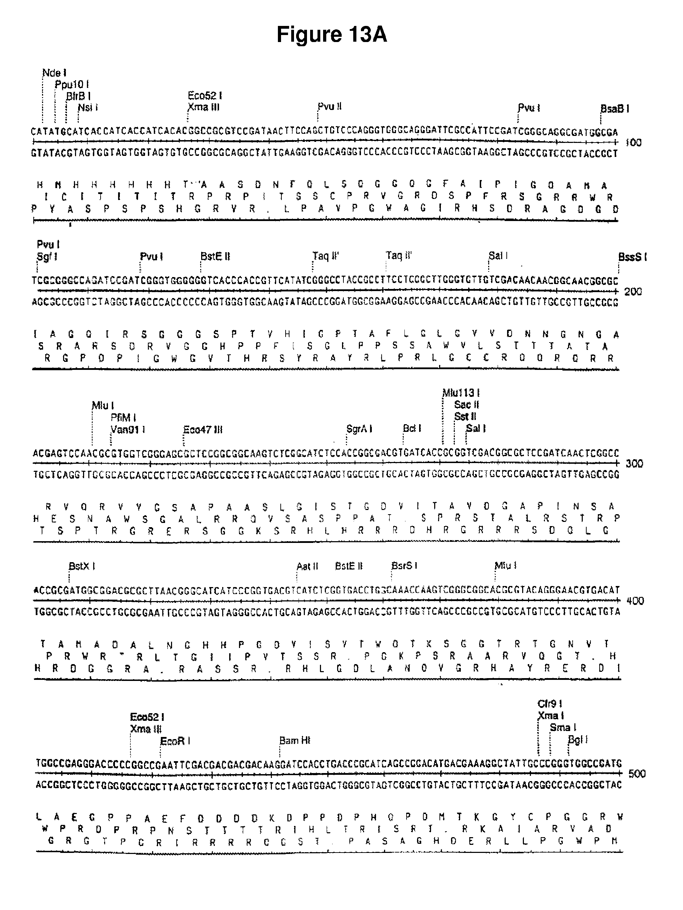

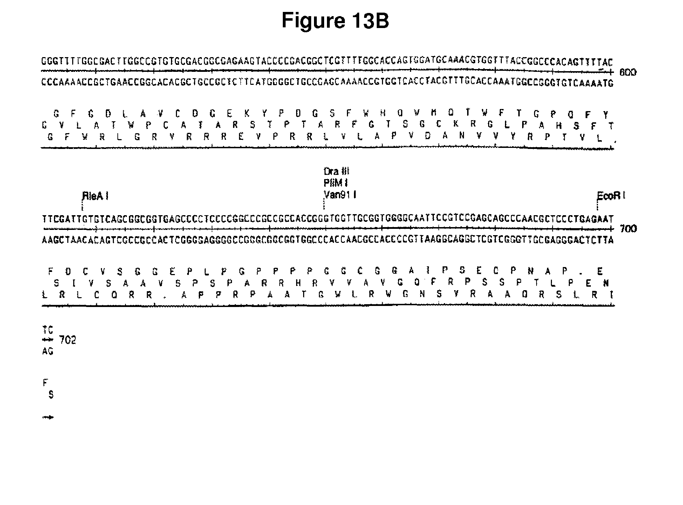

Three coding sequences for antigens Ra12, TbH9 and Ra35 were ligated to encode one fusion protein (SEQ ID NOS:1 and 2) (Fig. 1A and 2B). Another three coding sequences for antigens Erd14, DPV and MTI were ligated to encode a second fusion protein (SEQ ID NOS:3 and 4) (Fig. 2). Three coding sequences for antigens TbRa3, 38kD and Tb38-1 were ligated to encode one fusion protein (SEQ ID NOS:5 and 6) (Fig. 3A - 3D). Two coding sequences for antigens TbH9 and Tb38-1 were ligated to encode one fusion protein (SEQ ID NOS:7 and 8) (Fig. 4A - 4D). Four coding sequences for antigens TbRa3, 38kD, Tb38-1 and DPEP were ligated to encode one fusion protein (SEQ ID NOS:9 and 10) (Fig. 5A - 5J). Five coding sequences for antigens Erd14, DPV, MTI, MSL and MTCC2 were ligated to encode one fusion protein (SEQ ID NOS:11 and 12) (Fig. 6A and 6B). Four coding sequences for antigens Erd14, DPV, MTI and MSL were ligated to encode one fusion protein (SEQ ID NOS:13 and 14) (Fig. 7A and 7B). Four coding sequences for antigens DPV, MTI, MSL and MTCC2 were ligated to encode one fusion protein (SEQ ID NOS:15 and 16) (Fig. 8A and 8B). Three coding sequences for antigens DPV, MTI and MSL were ligated to encode one fusion protein (SEQ ID NOS:17 and 18) (Fig. 9A and 9B). Three coding sequences for antigens TbH9, DPV and MTI were ligated to encode one fusion protein (SEQ ID NOS:19 and 20) (Fig. 10A and 10B). Three coding sequences for antigens Erd14, DPV and MTI were ligated to encode one fusion protein (SEQ ID NOS:21 and 22) (Fig. 11A and 11B). Two coding sequences for antigens TbH9 and Ra35 were ligated to encode one fusion protein (SEQ ID NOS:23 and 24) (Fig. 12A and 12B). Two coding sequences for antigens Ra12 and DPPD were ligated to encode one fusion protein (SEQ ID NOS:25 and 26) (Fig. 13A and 13B).

The recombinant proteins were expressed in *E*. *coli* with six histidine residues at the amino-terminal portion using the pET plasmid vector (pET-17b) and a T7 RNA polymerase expression system (Novagen, Madison, WI). *E*. *coli* strain BL21 (DE3) pLysE (Novagen) was used for high level expression. The recombinant (His-Tag) fusion proteins were purified from the soluble supernatant or the insoluble inclusion body of 500 ml of IPTG induced batch cultures by affinity chromatography using the one step QIAexpress Ni-NTA Agarose matrix (QIAGEN, Chatsworth, CA) in the presence of 8M urea. Briefly, 20 ml of an overnight saturated culture of BL21 containing the pET construct was added into 500 ml of 2xYT media containing 50 µg/ml ampicillin and 34 µg/ml chloramphenicol, grown at 37°C with shaking. The bacterial cultures were induced with 2mM IPTG at an OD *560* of 0.3 and grown for an additional 3 h (OD =1.3 to 1.9). Cells were harvested from 500 ml batch cultures by centrifugation and resuspended in 20 ml of binding buffer (0.1 M sodium phosphate, pH 8.0; 10 mM Tris-HCl, pH 8.0) containing 2mM PMSF and 20 µg/ml leupeptin plus one complete protease inhibitor tablet (Boehringer Mannheim) per 25 ml. *E*. *coli* was lysed by freeze-thaw followed by brief sonication, then spun at 12 k rpm for 30 min to pellet the inclusion bodies.

The inclusion bodies were washed three times in 1% CHAPS in 10 mM Tris-HCl (pH 8.0). This step greatly reduced the level of contaminating LPS. The inclusion body was finally solubilized in 20 ml of binding buffer containing 8 M urea or 8M urea was added directly into the soluble supernatant. Recombinant fusion proteins with His-Tag residues were batch bound to Ni-NTA agarose resin (5 ml resin per 500 ml inductions) by rocking at room temperature for 1 h and the complex passed over a column. The flow through was passed twice over the same column and the column washed three times with 30 ml each of wash buffer (0.1 M sodium phosphate and 10 mM Tris-HCL, pH 6.3) also containing 8 M urea. Bound protein was eluted with 30 ml of 150 mM immidazole in wash buffer and 5 ml fractions collected. Fractions containing each recombinant fusion protein were pooled, dialyzed against 10 mM TrisHCl (pH 8.0) bound one more time to the Ni-NTA matrix, eluted and dialyzed in 10 mM Tris-HCL (pH 7.8). The yield of recombinant protein varies from 25 - 150 mg per liter of induced bacterial culture with greater than 98% purity. Recombinant proteins were assayed for endotoxin contamination using the *Limulus* assay (BioWhittaker) and were shown to contain < 10 E.U.Img.

### 6.1.2. T-CELL PROLIFERATION ASSAY

Purified fusion polypeptides were tested for the ability to induce T-cell proliferation in peripheral blood mononuclear cell (PBMC) preparations. The PBMCs from donors known to be PPD skin test positive and whose T-cells were shown to proliferate in response to PPD and crude soluble proteins from *M. tuberculosis* were cultured in RPMI 1640 supplemented with 10% pooled human serum and 50 µg/ml gentamicin. Purified polypeptides were added in duplicate at concentrations of 0.5 to 10 µg/ml. After six days of culture in 96-well round-bottom plates in a volume of 200 µl, 50 µl of medium was removed from each well for determination of IFN-γ levels, as described below in Section 6.1.3. The plates were then pulsed with 1 µCi/well of tritiated thymidine for a further 18 hours, harvested and tritium uptake determined using a gas scintillation counter. Fractions that resulted in proliferation in both replicates three fold greater than the proliferation observed in cells cultured in medium alone were considered positive.

### 6.1.3. INTERFERON-γ ASSAY

Spleens from mice were removed asceptically and single cell suspension prepared in complete RPMI following lysis of red blood cells. 100 µl of cells (2x10⁻⁵ cells) were plated per well in a 96-well flat bottom microtiter plate. Cultures were stimulated with the indicated recombinant proteins for 24h and the supernatant assayed for IFN-γ.

The levels of supernatant IFN-γ was analysed by sandwich ELISA, using antibody pairs and procedures available from PharMingen. Standard curves were generated using recombinant mouse cytokines. ELISA plates (Corning) were coated with 50 µl/well (1 µg/ml, in 0.1 M bicarbonate coating buffer, pH9.6) of a cytokine capture mAb (rat antimouse IFN-γ (PharMingen; Cat. # 18181D)), and incubated for 4 h at room temp. Shake out plate contents and block with PBS-0.05% Tween, 1.0% BSA (200 µl/well) overnight at 4°C and washed for 6X in PBS-0.1% Tween. Standards (mouse IFN-γ) and supernatant samples diluted in PBS-0.05% Tween, 0.1% BSA were then added for 2 hr at room temp. The plates were washed as above and then incubated for 2 hr at room temperature with 100 µl/well of a second Ab (biotin rat α mouse IFN-γ (Cat. # 18112D; PharMingen) at 0.5 µg/ml diluted in PBS-0.05% Tween, 0.1 % BSA. After washing, plates were incubated with 100 µl/well of streptavidin-HRP (Zymed) at a 1:2500 dilution in PBS-0.05% Tween, 0.1% BSA at room temp for lhr. The plates were washed one last time and developed with 100 µl/well TMB substrate (3,3',5,5' - tetramethylbenzidine, Kirkegaard and Perry, Gaithersburg, MD) and the reaction stopped after color developed, with H₂S0₄, 50 µl/well. Absorbance (OD) were determined at 450 nm using 570 nm as a reference wavelength and the cytokine concentration evaluated using the standard curve.

### 6.2. RESULTS

### 6.2.1. TRI-FUSION PROTEINS INDUCED IMMUNE RESPONSES

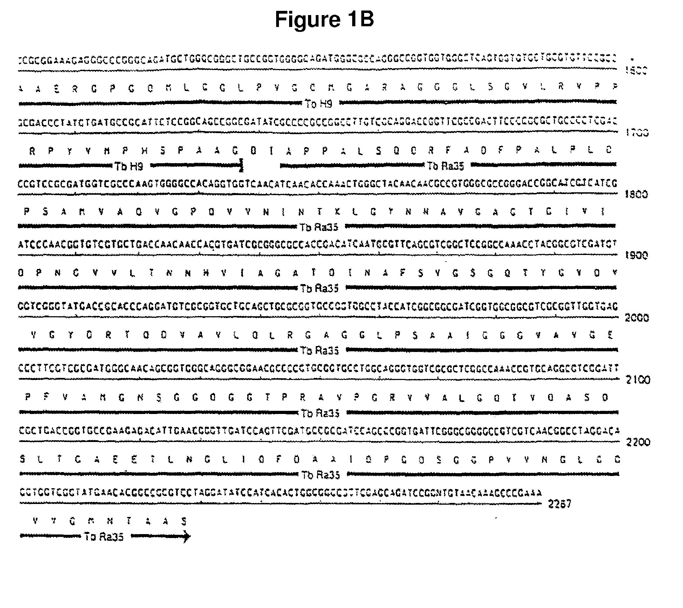

Three coding sequences for *M*. *tuberculosis* antigens were inserted into an expression vector for the production of a fusion protein. The antigens designated Ra12, TbH9 and Ra35 were produced as one recombinant fusion protein (Figure 1A and 1B). Antigens Erd14, DPV and MTI were produced as a second fusion protein (Figure 2). The two fusion proteins were affinity purified for use in *in vitro* and *in vivo* assays.

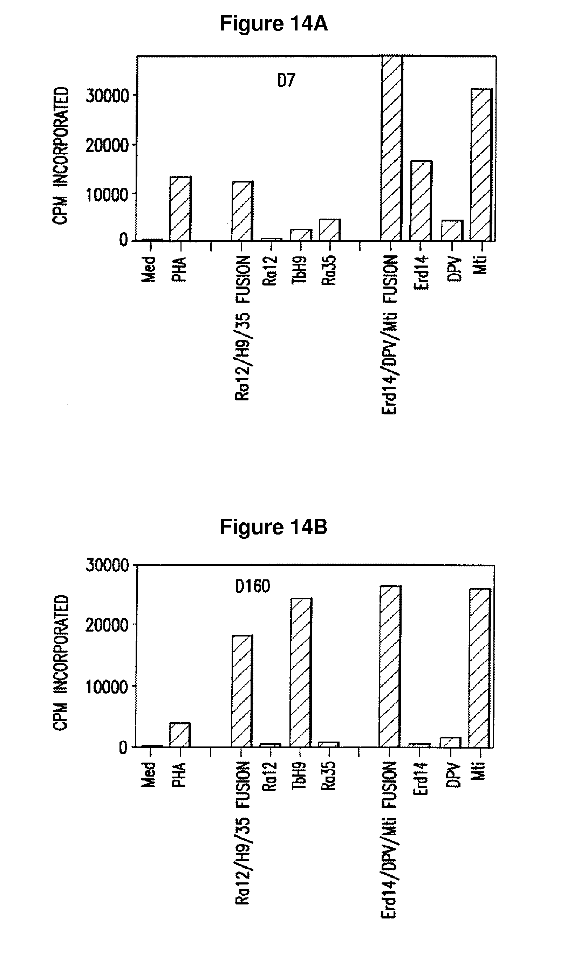

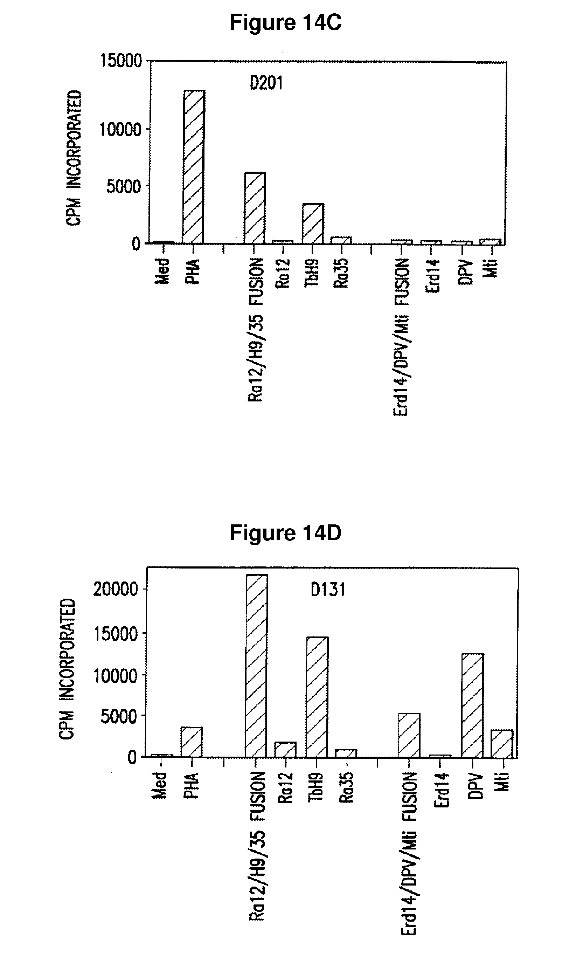

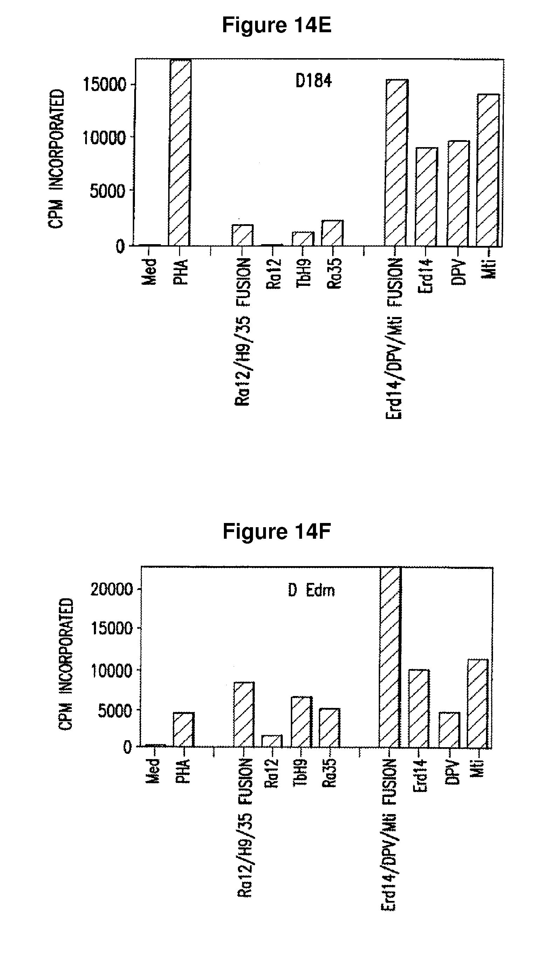

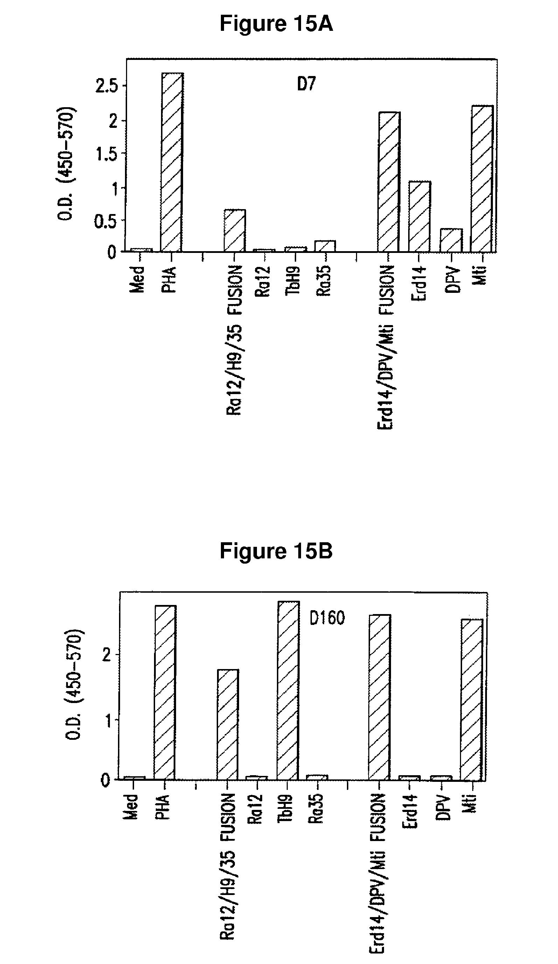

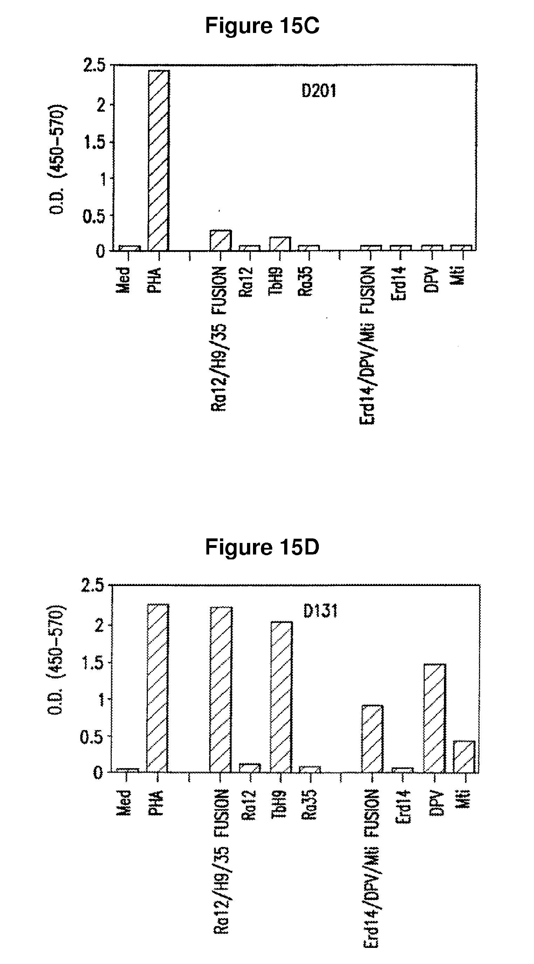

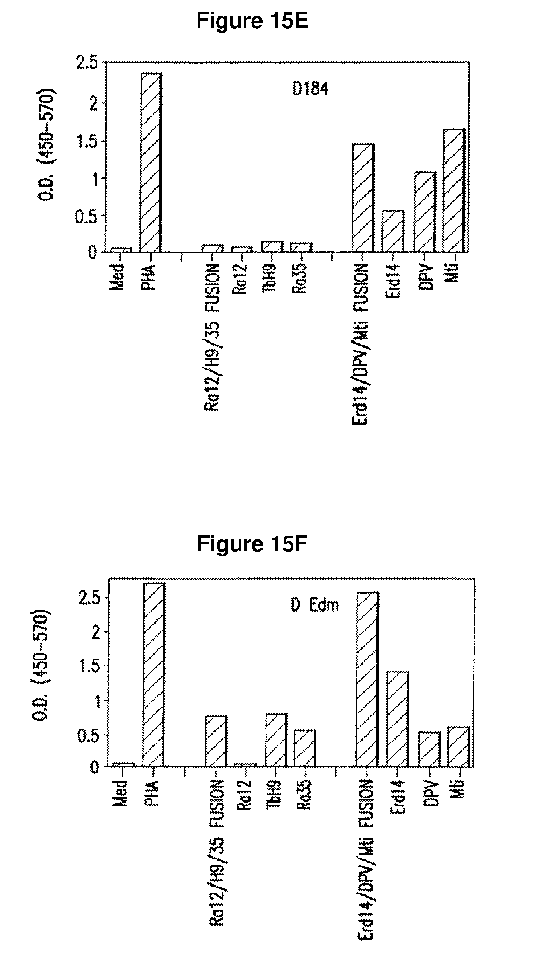

The two fusion proteins were tested for their ability to stimulate T cell responses from six PPD⁺ subjects. When T cell proliferation was measured, both fusion proteins exhibited a similar reactivity pattern as their individual components (Figure 14A-14F). A similar result was obtained when IFN-γ production was measured (Figure 15A-15F). For example, subject D160 responded to antigens TbH9 and MTI individually. Subject D160 also responded to the fusion proteins that contained these antigens (Figure 14B and 15B). In contrast, no T cell response from D160 was observed to other antigens individually. Another subject, D201, who did not react with antigens Erd14, DPV or MTI individually, was also unresponsive to the fusion protein containing these antigens. It should be noted that when the T cell responses to the individual components of the two fusion proteins were not particularly strong, the fusion proteins stimulated responses that were equal to or higher than that induced by the individual antigens in most cases.

The Ra12-TbH9-Ra35 tri-fusion protein was also tested as an immunogen *in vivo.* In these experiments, the fusion protein was injected into the footpads of mice for immunization. Each group of three mice received the protein in a different adjuvant formulation: SBAS1c SBAS2 (Ling et al., 1997, Vaccine 15:1562-1567), SBAS7 and AL(OH)₃. After two subcutaneous immunizations at three week intervals, the animals were sacrificed one week later, and their draining lymph nodes were harvested for use as responder cells in T cell proliferation and cytokine production assays.

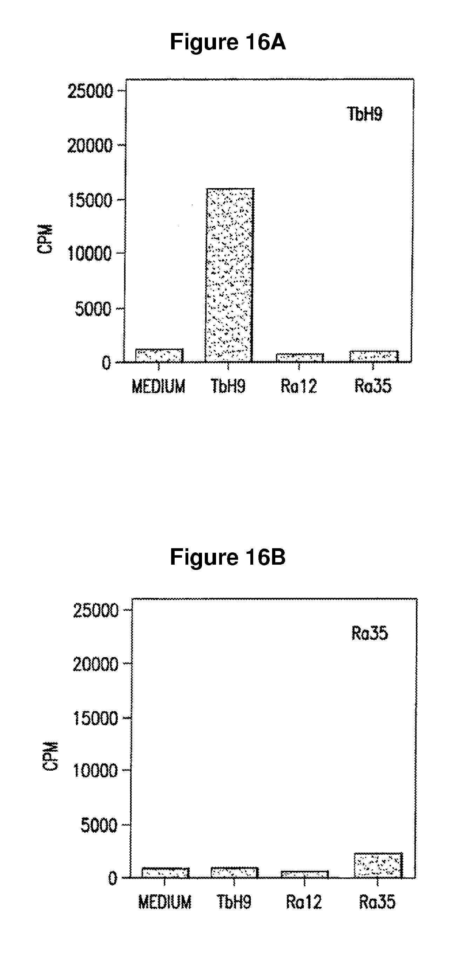

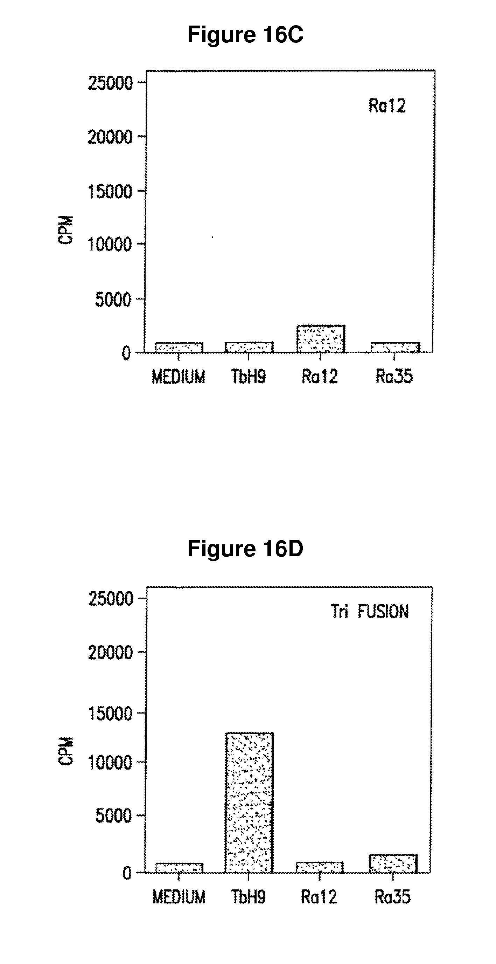

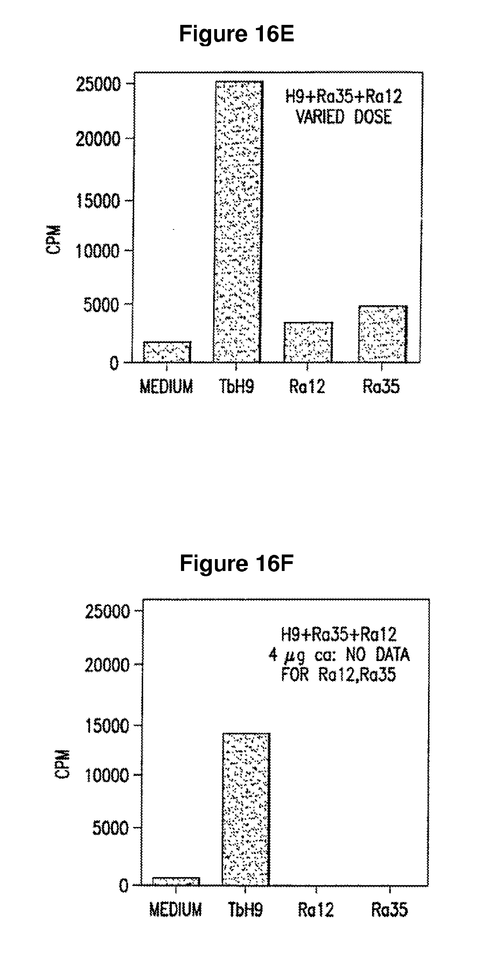

Regardless which adjuvant was used in the immunization, strong T cell proliferation responses were induced against TbH9 when it was used as an individual antigen (Figure 16A). Weaker responses were induced against Ra35 and Ra12 (Figure 16B and 16C). When the Ra12-TbH9-Ra35 fusion protein was used as immunogen, a response similar to that against the individual components was observed.

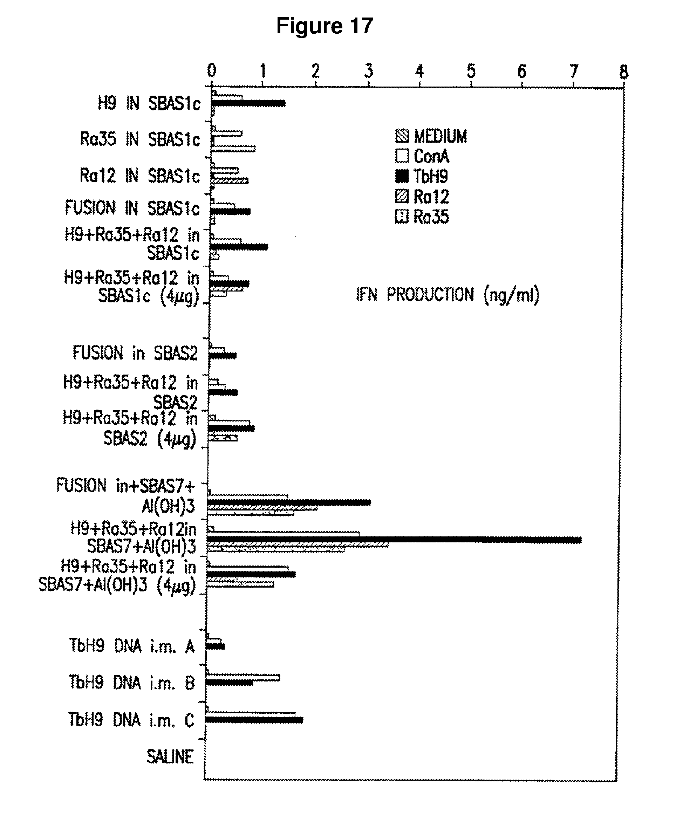

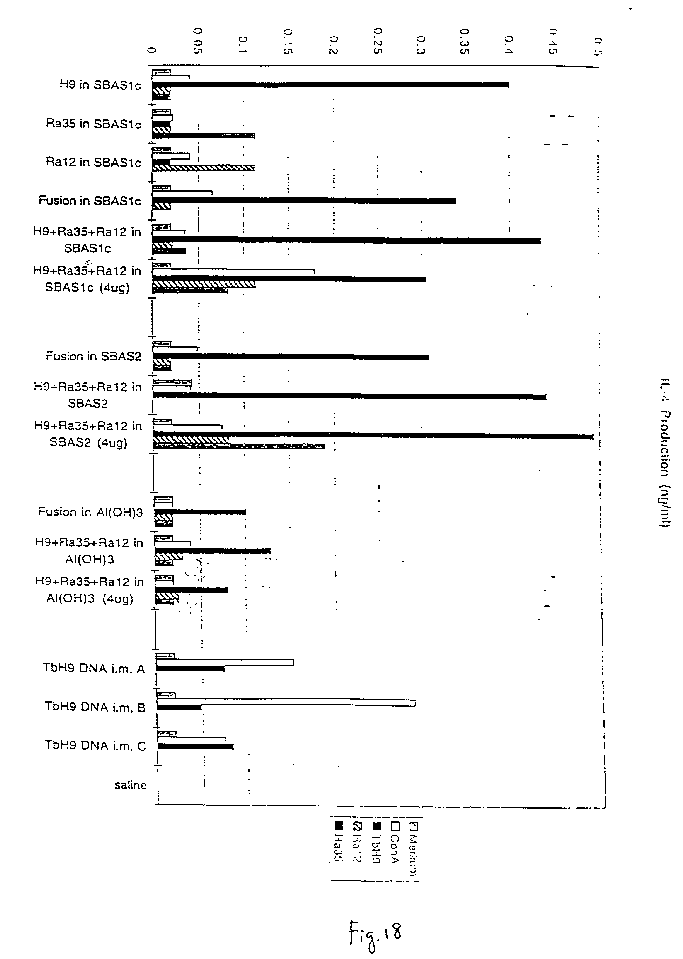

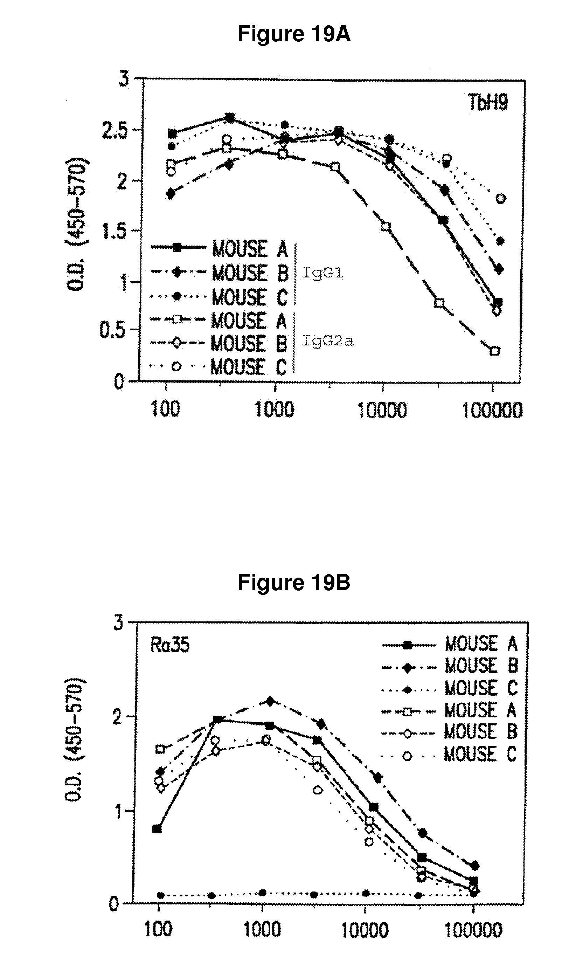

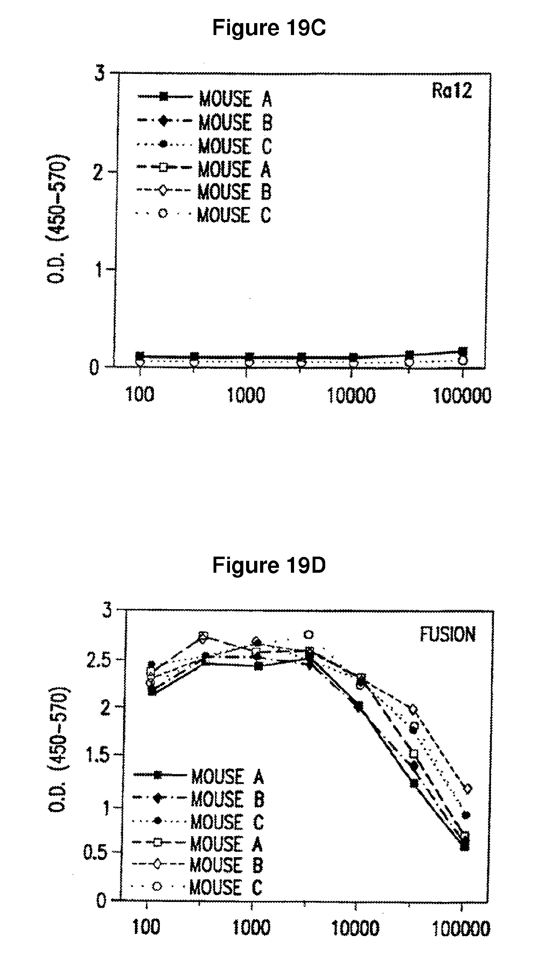

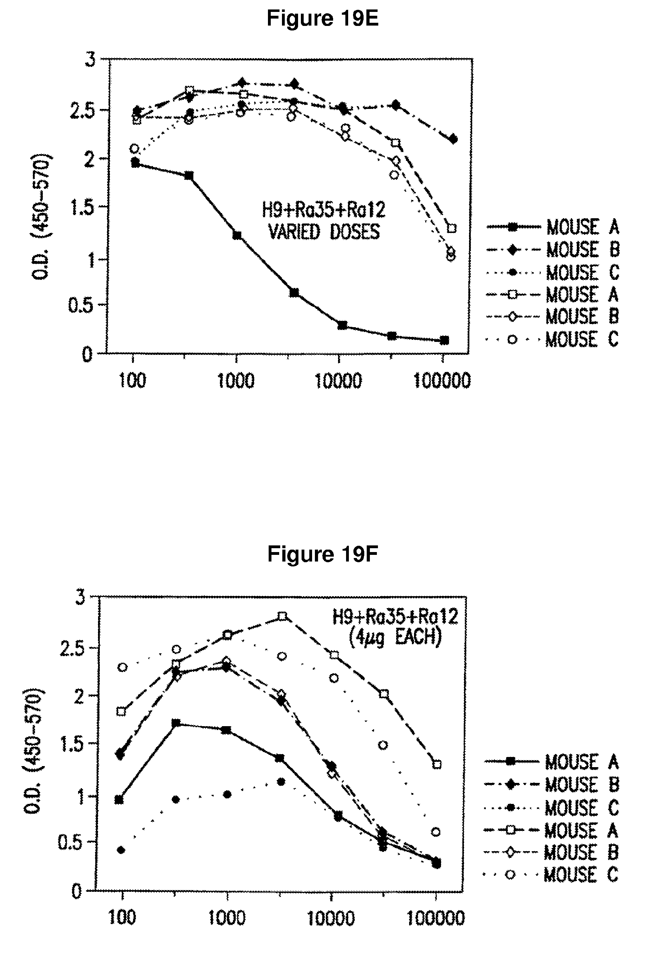

When cytokine production was measured, adjuvants SBAS1c and SBAS2 produced similar IFN-γ (Figure 17) and IL-4 responses (Figure 18). However, the combination of SBAS7 and aluminum hydroxide produced the strongest IFN-γ responses and the lowest level of IL-4 production for all three antigens. With respect to the humoral antibody response *in vivo,* Figure 19A-19F shows that the fusion protein elicited both IgG₁ and IgG₂ₐ antigen-specific responses when it was used with any of the three adjuvants.

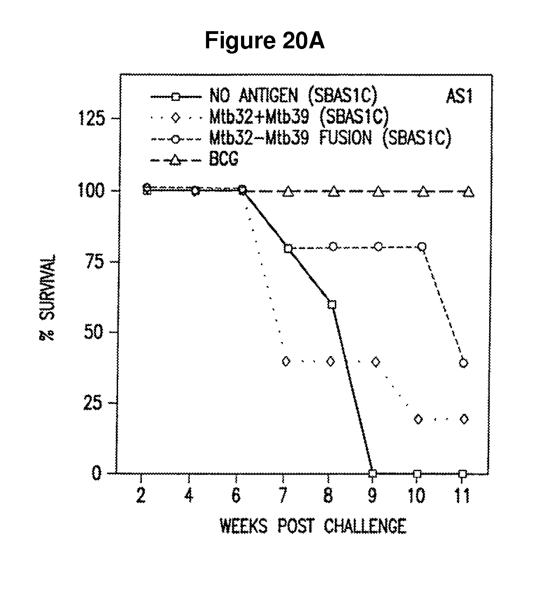

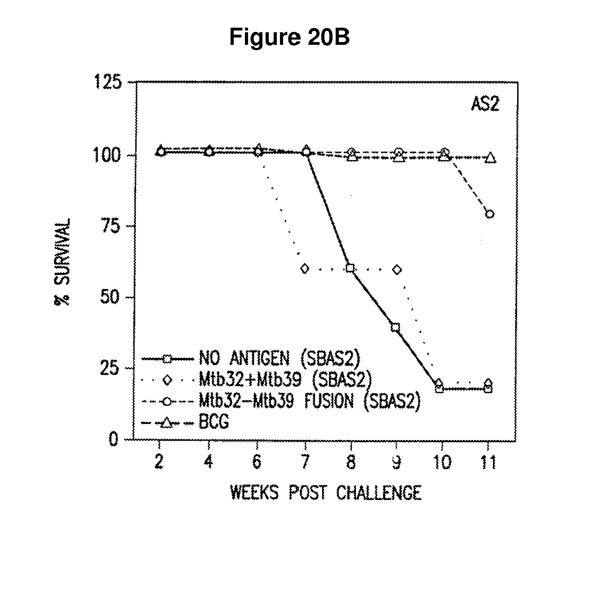

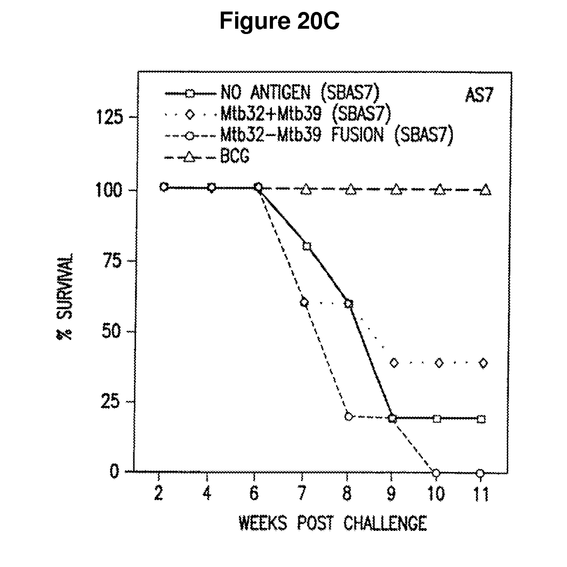

Additionally, C57BL/6 mice were immunized with a combination of two expression constructs each containing Ra12-TbH9-Ra35 (Mtb32A) or Erd14-DPV-MTI (Mtb39A) coding sequence as DNA vaccines. The immunized animals exhibited significant protection against tuberculosis upon a subsequent aerosol challenge of live bacteria. Based on these results, a fusion construct of Mtb32A and Mtb39A coding sequences was made, and its encoded product tested in a guinea pig long term protection model. In these studies, guinea pigs were immunized with a single recombinant fusion protein or a mixture of Mtb32A and Mtb39A proteins in formulations containing an adjuvant. Figure 20A-20C shows that guinea pigs immunized with the fusion protein in SBAS1c or SBAS2 were better protected against the development of tuberculosis upon subsequent challenge, as compared to animals immunized with the two antigens in a mixture in the same adjuvant formulation. The fusion proteins in SBAS2 formulation afforded the greatest protection in the animals. Thus, fusion proteins of various *M. tuberculosis* antigens may be used as more effective immunogens in vaccine formulations than a mixture of the individual components.

### 6.2.2. BI-FUSION PROTEIN INDUCED IMMUNE RESPONSES

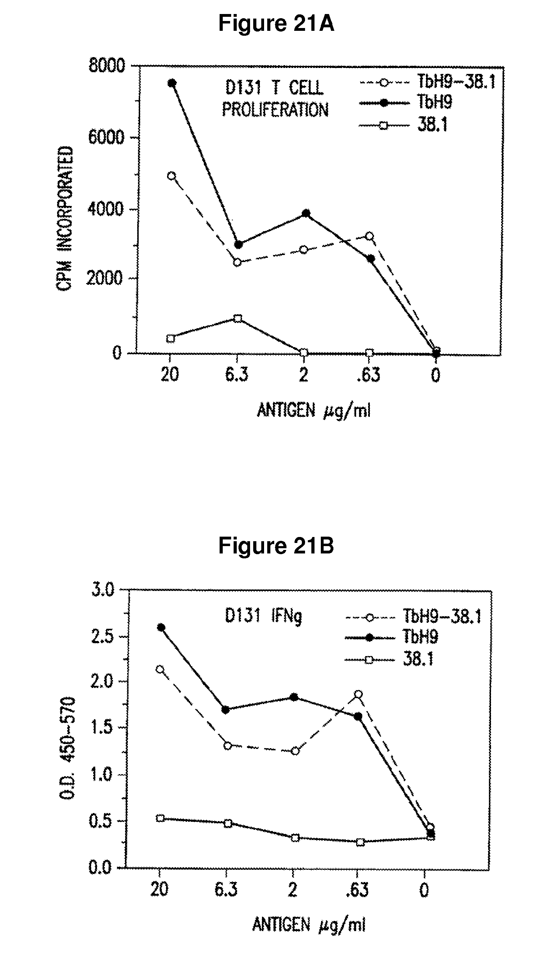

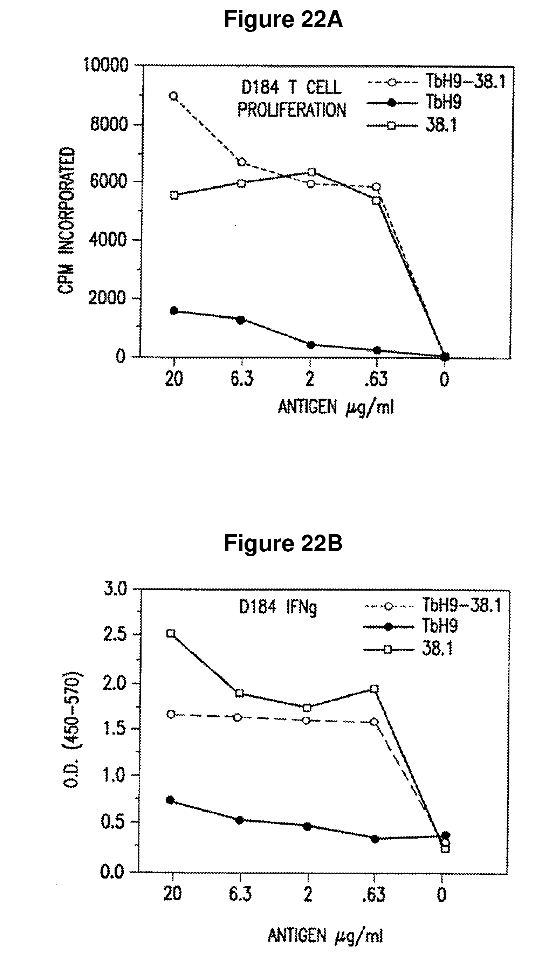

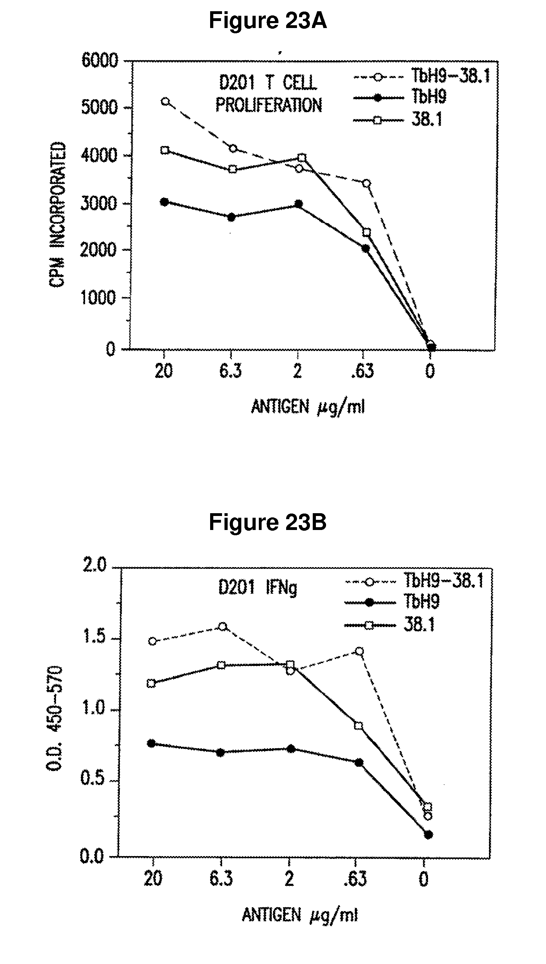

A bi-fusion fusion protein containing the TbH-9 and Tb38-1 antigens without a hinge sequence was produced by recombinant methods. The ability of the TbH9-Tb38-1 fusion protein to induce T cell proliferation and IFN-γ production was examined. PBMC from three donors were employed: one donor had been previously shown to respond to TbH9 but not to Tb38-1 (donor 131); one had been shown to respond to Tb38-1 but not to TbH9 (donor 184); and one had been shown to respond to both antigens (donor 201). The results of these studies demonstrate the functional activity of both the antigens in the fusion protein (Figures 21A and 21B, 22A and 22B, and 23A and 23B).

### 6.2.3. A TETRA-FUSION PROTEIN REACTED WITH TUBERCULOSIS PATIENT SERA

A fusion protein containing TbRa3, 38KD antigen, Tb38-1 and DPEP was produced by recombinant methods. The reactivity of this tetra-fusion protein referred to as TbF-2 with sera from *M. tuberculosis*-infected patients was examined by ELISA. The results of these studies (Table 1) demonstrate that all four antigens function independently in the fusion protein.

One of skill in the art will appreciate that the order of the individual antigens within each fusion protein may be changed and that comparable activity would be expected provided that each of the epitopes is still functionally available. In addition, truncated forms of the proteins containing active epitopes may be used in the construction of fusion proteins.

The present invention is not to be limited in scope by the exemplified embodiments which are intended as illustrations of single aspects of the invention, and any clones, nucleotide or amino acid sequences which are functionally equivalent are within the scope of the invention. Indeed, various modifications of the invention in addition to those described herein will become apparent to those skilled in the art from the foregoing description and accompanying drawings. Such modifications are intended to fall within the scope of the appended claims. It is also to be understood that all base pair sizes given for nucleotides are approximate and are used for purposes of description.

All publications cited herein are incorporated by reference in their entirety.

**TABLE 1**

| REACTIVITY OF TBF-2 FUSION PROTEIN WITH TB AND NORMAL SERA | | | | | | | | | |
|---|---|---|---|---|---|---|---|---|---|
| Serum ID | Status | TbF OD450 | Status | TbF-2 OD450 | Status | ELISA Reactivity | | | |
| | | | | | | 38 kD | TbRa3 | Tb38-1 | DPEP |
| B931-40 | TB | 0.57 | + | 0.321 | + | - | + | - | + |
| B931-41 | TB | 0.601 | + | 0.396 | + | + | + | + | - |
| B931-109 | TB | 0.494 | + | 0.404 | + | + | + | ++ | - |
| B931-132 | TB | 1.502 | + | 1.292 | + | + | + | + | ++ |
| 5004 | TB | 1.806 | + | 1.666 | + | ±± | ++ | + | - |
| 15004 | TB | 2.862 | + | 2.468 | + | + | + | + | - |
| 39004 | TB | 2.443 | + | 1.722 | + | + | + | + | - |
| 68004 | TB | 2.871 | + | 2.575 | + | + | + | + | - |
| 99004 | TB | 0.691 | + | 0.971 | + | - | ++ | + | - |
| 107004 | TB | 0.875 | + | 0.732 | + | - | ++ | + | - |
| 92004 | TB | 1.632 | + | 1.394 | + | + | ++ | ++ | - |
| 97004 | TB | 1.491 | + | 1.979 | + | + | ++ | - | + |
| 118004 | TB | 3.182 | + | 3.045 | + | + | ++ | - | - |
| 173004 | TB | 3.644 | + | 3.578 | + | + | + | + | - |
| 175004 | TB | 3.332 | + | 2.916 | + | + | + | - | - |
| 274004 | TB | 3.696 | + | 3.716 | + | - | + | - | + |
| 276004 | TB | 3.243 | + | 2.56 | + | - | - | + | - |
| 282004 | TB | 1.249 | + | 1.234 | + | + | - | - | - |
| 289004 | TB | 1.373 | + | 1.17 | + | - | + | - | - |
| 308004 | TB | 3.708 | + | 3.355 | + | - | - | + | - |
| 314004 | TB | 1.663 | + | 1.399 | + | - | - | + | - |
| 317004 | TB | 1.163 | + | 0.92 | + | + | - | - | - |
| 312004 | TB | 1.709 | + | 1.453 | + | - | + | - | - |
| 380004 | TB | 0.238 | - | 0.461 | + | - | ++ | - | + |
| 451004 | TB | 0.18 | - | 0.2 | - | - | - | - | ++ |
| 478004 | TB | 0.188 | - | 0.469 | + | - | - | - | ++ |
| 410004 | TB | 0.384 | + | 2.392 | + | ++ | - | - | + |
| 411004 | TB | 0.306 | + | 0.874 | + | - | + | - | + |
| 421004 | TB | 0.357 | + | 1.456 | + | - | + | - | + |
| 528004 | TB | 0.047 | - | 0.196 | | - | - | - | + |
| A6-87 | Normal | 0.094 | - | 0.063 | - | - | - | - | - |
| A6-88 | Normal | 0.214 | - | 0.19 | - | - | - | - | - |
| A6-89 | Normal | 0.248 | - | 0.125 | - | - | - | - | - |
| A6-90 | Normal | 0.179 | - | 0.206 | - | - | - | - | - |
| A6-91 | Normal | 0.135 | - | 0.151 | - | - | - | - | - |
| A6-92 | Normal | 0.064 | - | 0.097 | - | - | - | - | - |
| A6-93 | Normal | 0.072 | - | 0.098 | - | - | - | - | - |
| A6-94 | Normal | 0.072 | - | 0.064 | - | - | - | - | - |
| A6-95 | Normal | 0.125 | - | 0.159 | - | - | - | - | - |
| A6-96 | Normal | 0.121 | - | 0.12 | - | - | - | - | - |
| | | | | | | | | | |
| Cut-off | | 0.284 | | 0.266 | | | | | |

## Claims

1. A polypeptide comprising an amino acid sequence encoded by a polynucleotide that hybridizes under moderately stringent conditions to a second polynucleotide which is complementary to a nucleotide sequence that encodes the amino acid of SEQ ID NO: 16, said amino acid sequence induces an immune response to *M*. *tuberculosis.*

2. A polypeptide according to claim 1, encoded by a polynucleotide that hybridizes under moderately stringent conditions to a second polynucleotide which is complementary to a nucleotide sequence that encodes the amino acid of SEQ ID NO: 16, said amino acid sequence induces an immune response to *M. tuberculosis.*

3. A polypeptide comprising the amino acid sequence of SEQ ID NO: 16, said amino acid sequence may optionally contain one or more conservative amino acid substitutions.

4. The polypeptide of Claim 1 or 2 which is a soluble polypeptide.

5. The polypeptide of Claim 1 or 2 which induces a T cell response.

6. The polypeptide of Claim 1 or 2 which is fused with a second heterologous polypeptide.

7. A pharmaceutical composition comprising the polypeptide of Claim 1 or 2.

8. A pharmaceutical composition comprising a polynucleotide that encodes the polypeptide of Claim 1 or 2.

9. A polypeptide according to claim 1 for use as a medicament.

10. A polypeptide according to claim 1 or 2 for use in the prevention of tuberculosis.

11. A polynucleotide which comprises a nucleic acid sequence which hybridizes under moderately stringent conditions to a second polynucleotide which is complementary to a nucleotide sequence that encodes the amino acid of SEQ ID NO: 16, said polynucleotide encoding an amino acid sequence which induces an immune response to *M. tuberculosis.*

12. An expression vector comprising a polynucleotide according to claim 11.

13. An expression vector according to claim 12 which is a viral vector.

14. A vaccine composition comprising a polypeptide according to claim 1 or 2 and an adjuvant.

15. A vaccine composition comprising a polynucleotide according to claim 11.

16. A polynucleotide according to claim 11 for use as a medicament.

17. A polynucleotide according to claim 11 for use in the prevention of tuberculosis.

18. A method for the production of a polypeptide according to claim 1 or 2 comprising recombinant expression of a polynucleotide according to claim 11.
